# EUROPEAN PATENT APPLICATION

(11) **EP 4 454 663 A1**
(43) Date of publication of application: **30.10.2024**
(21) Application number: 22911314.7
(22) Date of filing: 21.12.2022
(51) Int. Cl.: A61K 39/395, A61P 9/04, A61P 9/10, A61K 38/48, G01N 33/53, A01K 67/027, A61K 31/7088

(54) **THERAPEUTIC OR PROPHYLACTIC AGENT FOR HEART ATTACK, HEART FIBROSIS, OR HEART FAILURE, WHERE HTRA3 IS THERAPEUTIC TARGET**

(30) Priority: 21.12.2021 US 202163292079 P
(71) Applicant: The University of Tokyo, Bunkyo-ku, Tokyo 113-8654 (JP); OSAKA UNIVERSITY, Suita-shi Osaka 565-0871 (JP)
(72) Inventor: NOMURA, Seitaro, Tokyo 113-8654 (JP); KO, Toshiyuki, Tokyo 113-8654 (JP); KATOH, Manami, Tokyo 113-8654 (JP); ABURATANI, Hiroyuki, Tokyo 113-8654 (JP); KOMURO, Issei, Tokyo 113-8654 (JP); NAKAGAMI, Hironori, Suita-shi, Osaka 565-0871 (JP)
(74) Representative: Regimbeau
(86) International application number: PCT/JP2022/047234
(87) International publication number: WO 2023/120612

(57) **Abstract**

An object of the present invention is to provide a therapeutic or preventive agent for myocardial infarction, cardiac fibrosis, or heart failure using Htra3 as a therapeutic target. According to the present invention, there is provided a therapeutic or preventive agent for myocardial infarction, cardiac fibrosis, or heart failure containing at least one of (a) serine protease HtrA3, (b) a nucleic acid encoding HtrA3, (c) an expression vector containing a nucleic acid encoding HtrA3, (d) a cell transformed with the nucleic acid or the expression vector, and (e) an antibody that binds to serine protease HtrA3.

## Description

### CROSS- REFERENCE TO RELATED APPLICATION

The present application enjoys the benefit of priority from the prior U.S. application 63/292,079 (filed on December 21, 2021), the entire disclosures of which are incorporated herein by reference.

### TECHNICAL FIELD

The present invention relates to a therapeutic or preventive agent for myocardial infarction, cardiac fibrosis, or heart failure using Htra3 as a therapeutic target. The present invention also relates to an agent for regulating cardiac fibroblast DNA repair mechanisms.

### BACKGROUND ART

Tissue fibrosis and organ dysfunction are characteristics of age-related diseases including heart failure (Documents 1 to 5). Pathological stresses on the heart induce fibroblast activation and collagen secretion, leading to cardiac fibrosis (Documents 6 to 8). In contrast, suppression of fibroblast activation can alleviate cardiac fibrosis and ameliorate cardiac function after injury (Document 9), suggesting pathogenicity of fibrosis in heart failure. Persistent pathological stresses on the heart also induce the accumulation of DNA damage and activation of p53 signaling in cardiomyocytes, resulting in the conversion of heart failure to cardiomyocyte phenotypes characterized by mitochondrial dysfunction (Document 15), autophagic dysfunction (Document 16), elongate structures (Document 12), and telomere shortening (Document 17) (Non-Patent Documents 10 to 14). Inhibition of p53 or senolysis can improve the phenotypes of the heart following injury or aging (Documents 12 and 18), suggesting that senescence-like failing cardiomyocytes cause heart failure (Non-Patent Documents 1 and 2). Thus, tissue fibrosis and cellular senescence, which are characteristics of many age-related diseases, cause the onset and progression of heart failure, but whether there is a common pathway to trigger both tissue fibrosis and heart failure events is unknown.

### Reference List

### Non-Patent Document

Non-Patent Document 1: Nomura, S. et al. Cardiomyocyte gene programs encoding morphological and functional signatures in cardiac hypertrophy and failure. Nat. Commun. 9, 4435 (2018).
Non-Patent Document 2: Baker, D. J. et al. Naturally occurring p16 Ink4a-positive cells shorten healthy lifespan. Nature 530, 184-189 (2016).

### SUMMARY OF THE INVENTION

An object of the present invention is to provide a therapeutic or preventive agent for myocardial infarction, cardiac fibrosis, or heart failure using Htra3 as a therapeutic target, and an agent for regulating a DNA repair mechanism of cardiac fibroblasts. It is also an object of the present invention to provide a non-human mammalian heart failure model animal, a method for screening for an agent or the like for regulating the DNA repair mechanism of cardiac fibroblasts, a method of diagnosing myocardial infarction, cardiac fibrosis or heart failure, and a method of determining the severity level of myocardial infarction or heart failure.

The inventors hypothesized that intercellular communication between cardiac fibroblasts and cardiomyocytes may be important for inducing cardiac fibrosis and heart failure (Documents 19 and 20). The inventors have also found that cardiac fibroblasts strongly interact with different types of cardiac cells, including cardiomyocytes, in particular there are many interactions between cardiac fibroblasts and cardiomyocytes, by intercellular communication maps of the heart using single cell analysis (single-cell RNA sequencing, scRNA-seq). The inventors also identified high-temperature requirement A serine peptidase 3 (Htra3) in cardiac fibroblasts as an important regulator of cardiomyocyte homeostasis and cardiac fibrosis by single-cell co-expression network analysis. The inventors also found that through single cell analysis, spatial transcriptomics, and genetic perturbation, serine peptidase 3 (Htra3) is an important regulator of cardiac fibrosis and heart failure by maintaining quiescent cardiac fibroblast identity through degradation of transforming growth factor β (TGF-β). The present inventors have also produced and analyzed a knockout mouse of Htra3, and found that when the function of Htra3 is deficient, severe heart failure occurs when various loads such as pressure load and myocardial infarction are applied to the heart. The present inventors have also found that, the mechanism behind the state observed in knockout mice is that, when the function of Htra3 is lost, the amount of TGF-β protein, which is normally degraded by Htra3, increases, causing fibroblasts to produce more fibers and harden the heart, and that Nox4 protein, which is induced in cardiomyocytes by TGF-β, increases oxidative stress and damages DNA, thereby indirectly affecting cardiomyocytes and changing their properties. The present inventors have also found that pressure overload downregulates Htra3 expression in cardiac fibroblasts and activates TGF-β signaling, thereby inducing heart failure not only in cardiac fibrosis but also through accumulation of DNA damage and induction of a secretory phenotype in dysfunctional cardiomyocytes. The present inventors have also found that overexpression of Htra3 in the heart inhibits TGF-β signaling and ameliorates cardiac dysfunction after pressure overload. The present inventors have also found that Htra3-controlled induction of spatio-temporal cardiac fibrosis and cardiomyocyte secretory phenotype are specifically observed in the infarct regions after myocardial infarction. The present inventors have also found through Integrative analyses of human single cardiomyocyte transcriptome and plasma proteome that IGFBP7, a cytokine downstream of TGF-β and secreted from failing myocytes, is the most predictable marker of advanced heart failure. The present inventors have also found that these findings underscore the role of cardiac fibroblasts in cardiomyocyte homeostasis and regulation of cardiac fibrosis via the Htra3-TGF-β-IGFBP7 pathway, which is a therapeutic target for heart failure, and that cardiac fibroblasts regulate the development of heart failure via the Htra3-TGF-β-IGFBP7 axis. The present inventors have also found that overexpression of the inflammatory cytokine Igfbp7 in endothelial cells of aged hearts leads to reduced insulin signaling in cardiomyocytes. The present inventors have also found that overexpression of Igfbp7 in the heart degraded cardiac function, whereas vaccines targeting Igfbp7 ameliorated cardiac dysfunction in the presence of pressure overload. The present inventors have also concluded that pressure overload induces endothelial cell senescence and produces the inflammatory cytokine Igfbp7. The present inventors have also found that Igfbp7 suppresses cardiomyocyte metabolism, resulting in development of heart failure, and that vaccine therapy targeting IGFBP7 is useful in treating heart failure. The present invention is based on these findings.

### Solution to Problem

According to the present invention, the following invention is provided.
[1] An agent for regulating a DNA repair mechanism of cardiac fibroblasts, the agent comprising at least one of the following (a), (b), (c), (d) and (e):
   (a) serine protease HtrA3,
   (b) a nucleic acid encoding serine protease HtrA3 or a fragment thereof,
   (c) an expression vector comprising a nucleic acid encoding serine protease HtrA3 or a fragment thereof,
   (d) a cell transformed with a nucleic acid encoding serine protease HtrA3, a fragment thereof, or an expression vector comprising the nucleic acid or fragment, and
   (e) an antibody that binds to serine protease HtrA3 or a fragment thereof.
[2] The agent according to above [1], in which the serine protease HtrA3 is derived from a human or mouse.
[3] A pharmaceutical composition for treating or preventing myocardial infarction, cardiac fibrosis, or heart failure, the composition comprising the agent according to above [1] or [2] or at least one of (a), (b), (c), (d), and (e) according to above [1] or [2] as an active ingredient.
[4] The pharmaceutical composition of above [3], in which the heart failure is heart failure with reduced ejection fraction (HFrEF).
[5] A non-human mammalian heart failure model animal in which expression of serine protease HtrA3 in cardiac fibroblasts is reduced or inhibited.
[6] A method for screening for an agent or a method for regulation of a DNA repair mechanism in a cardiac fibroblast, the method comprising
   (A) a step of treating a cell in which expression of serine protease HtrA3 is reduced or inhibited with a test factor, and
   (B) a step of comparing the cell to an untreated control cell or a wild-type cell.
[7] A method for screening for an agent or a method for regulation of a DNA repair mechanism in a cardiac fibroblast, the method comprising
   (C) a step of treating the non-human mammal according to above [5] with a test factor, and
   (D) a step of comparing the non-human mammal to an untreated control animal or a wild-type animal.
[8] A method of diagnosing myocardial infarction, cardiac fibrosis, or heart failure, the method comprising
   (E) a step of determining that a subject may suffer from myocardial infarction, cardiac fibrosis or heart failure in a case where an expression level of serine protease HtrA3 in a cardiac fibroblast of the subject is significantly reduced as compared with an expression level in a cardiac fibroblast of a healthy subject.
[9] A pharmaceutical composition for treating or preventing myocardial infarction or heart failure, the composition comprising at least one of the following (f), (g), (h) and (i):
   (f) a nucleic acid consisting of a base sequence complementary to a nucleic acid encoding insulin-like growth factor binding protein 7 (IGFBP7) or a fragment thereof,
   (g) an antibody that binds to insulin-like growth factor binding protein 7 (IGFBP7) or a fragment thereof,
   (h) a nucleic acid that encodes an antibody or a fragment thereof that binds to insulin-like growth factor binding protein 7 (IGFBP7) or a fragment thereof, and
   (i) an expression vector comprising a nucleic acid that encodes an antibody or a fragment thereof that binds to insulin-like growth factor binding protein 7 (IGFBP7) or a fragment thereof.
[10] The pharmaceutical composition according to above [9], in which the nucleic acid (f) composed of a complementary base sequence is selected from the group consisting of siRNA, antisense nucleic acid, shRNA, miRNA, gRNA, a ribozyme, and a nucleic acid aptamer.
[11] The pharmaceutical composition according to above [9], in which the insulin-like growth factor binding protein 7 (IGFBP7) is from a human or mouse.
[12] A non-human mammalian heart failure model animal with enhanced expression of insulin-like growth factor binding protein 7 (IGFBP7) in cardiac fibroblasts.
[13] A method for screening for an agent or a method for treating or preventing myocardial infarction or heart failure, the method comprising
   (F) a step of treating a cell with enhanced expression of insulin-like growth factor binding protein 7 (IGFBP7) with a test factor; and
   (G) a step of comparing the cell to an untreated control cell or a wild-type cell.
[14] A method for screening for an agent or a method for treating or preventing myocardial infarction or heart failure, the method including
   (H) a step of treating the non-human mammal according to above [12] with a test factor, and
   (I) a step of comparing the non-human mammal to an untreated control animal or a wild-type animal.
[15] A method of diagnosing myocardial infarction or heart failure, the method including
   (J) a step of determining that a subject may suffer from myocardial infarction or heart failure in a case where an expression level of insulin-like growth factor binding protein 7 (IGFBP7) in cardiac fibroblasts, cardiomyocytes, or blood of the subject is significantly increased as compared with an expression level in a cardiac fibroblast, a cardiomyocyte, or blood of a healthy subject.
[16] A method of determining a severity level of myocardial infarction, or heart failure, the method including
   (K) a step of determining a severity level of myocardial infarction or heart failure of a subject by comparing an expression level of insulin-like growth factor binding protein 7 (IGFBP7) in a cardiac fibroblast, a cardiomyocyte, or blood of the subject with an expression level in a cardiac fibroblast, a cardiomyocyte, or blood of a healthy subject or a past expression level of the subject.
[17] A method of regulating a DNA repair mechanism of cardiac fibroblasts, and a method for treating or preventing myocardial infarction, cardiac fibrosis or heart failure, the method including administering at least one of (a), (b), (c), (d), and (e) described in above [1] or [2] to a subject in need thereof.
[18] Use of the heart failure model animal according to above [5] for screening a drug or a method of regulating a DNA repair mechanism of a cardiac fibroblast or for screening a therapeutic or preventive agent for myocardial infarction, cardiac fibrosis or heart failure.
[19] A method for treating or preventing myocardial infarction or heart failure, the method including administering at least one of (f), (g), (h), and (i) described in the above [9] to a subject in need thereof.
[18] Use of the heart failure model animal of above [12] for screening for a therapeutic agent or preventive agent for myocardial infarction or heart failure.
[19] Use of serine protease HtrA3 or a gene thereof as a marker for diagnosing of myocardial infarction, cardiac fibrosis, or heart failure.
[20] Use of insulin-like growth factor binding protein 7 (IGFBP7) or a gene thereof as a marker for diagnosing myocardial infarction or heart failure or as a marker for determining the severity level of myocardial infarction or heart failure.

According to the present invention, it is advantageous in that an agent for regulating of the DNA repair mechanism of cardiac fibroblasts and a pharmaceutical composition for the treatment or prevention of myocardial infarction or heart failure can be provided.

### BRIEF DESCRIPTION OF THE DRAWINGS

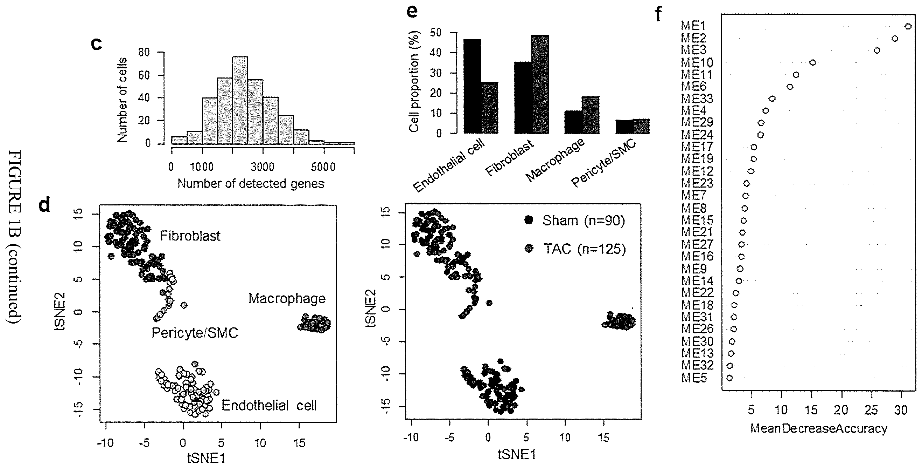

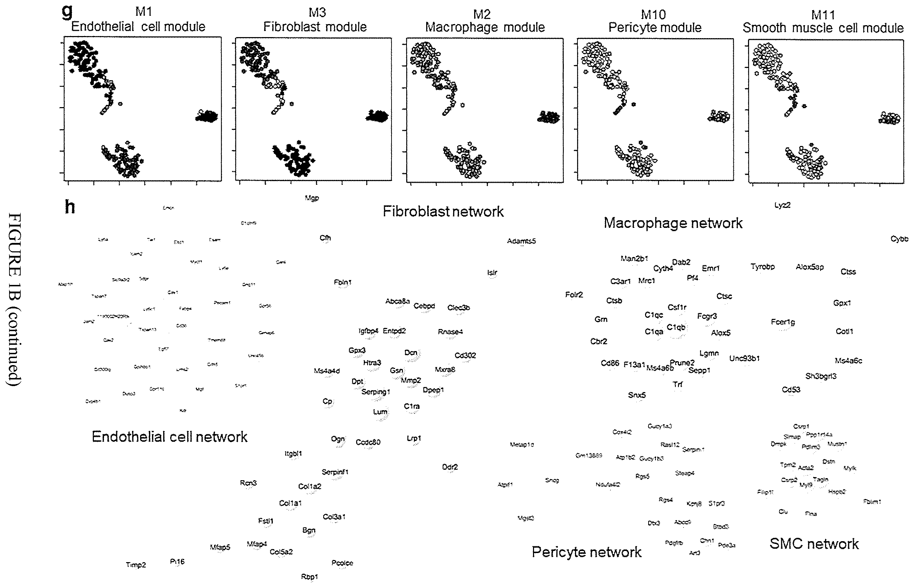

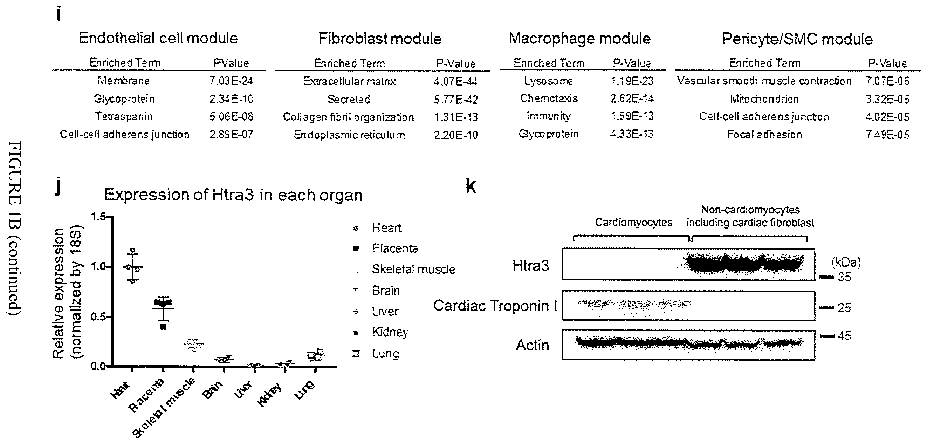

Fig. 1A illustrates that cardiac fibroblasts Htra3 are identified by single cell network analysis. a Isolation of non-cardiomyocytes and fibroblasts for single cell analysis using fluorescence-activated cell sorting (FACS). Sorted live non-cardiomyocytes (non-CM) were used for single cell analysis using the 10X Genomics platform. Sorted fibroblasts, including antibodies against PDGFR-α, were used for single cell analysis using full-length Smart-seq2. b, c Ligand-receptor interaction maps of the heart. The ligand and receptor genes were projected onto a two-dimensional map based on their correlation structures. The cell types were annotated by the cell-type specific expression profile of the genes assigned to each module. The edge illustrates a correlation (b) or an interaction (c). d Pathway analysis of genes illustrated in the heart interaction map. p-values were determined by Fisher's exact test. e Heatmap illustrating the number of interactions between cell types in the heart. VSMCs, vascular smooth muscle cells. f Gene ontology (GO) analysis of upregulated genes after pressure overload in cardiac fibroblasts. Representative genes were also illustrated. p-values were determined by Fisher's exact test. g Co-expression network of cardiac fibroblasts, h Bar chart illustrating the top 10 genes most correlated with cardiac fibroblast module. i Uniform manifold approximation and projection (UMAP) plots of the single-cell transcriptome of non-cardiomyocytes (n=1783) from mouse hearts (n=2). Cell type annotations (left) and Htra3 expression (right) are on UMAP plots. j RNA in situ hybridization of Htra3 and immunostaining of Pdgfr-α using paired mirror cardiac sections. Arrows indicate co-localization of Htra3 and Pdgfr-α within the same cell. k RNA in situ hybridization of HTRA3 in human hearts. Arrows indicate expression of HTRA3.

Fig. 1B illustrates single cell analysis of non-cardiomyocytes in the heart using Chromium (10X Genomics) and the Smart-seq2 platform associated with Fig. 1A. a Uniform Manifold Approximation and Projection (UMAP) plot of the single-cell transcriptome of non-cardiomyocytes (n=1,783) from murine hearts (n=2). Cell-type specific expression profiles are also illustrated. b Heatmap illustrating cell-type specific expression profile of genes including Htra3 (top). Violin plot illustrating fibroblast specific expression of Htra3 (bottom). c Histogram illustrating the distribution of the number of detected genes. Cells in which 2,000 or more genes were detected were used for subsequent analysis. d T-distributed Stochastic Neighbor Embedding (tSNE) plot of the single-cell transcriptome of non-cardiomyocytes (sham n=90, transverse aortic stenosis [TAC] n=125) from the heart (n=2 for sham, n=2 for TAC) of mice. Cell types were determined by graph-based clustering and random forest classifier (left). The origin of the cells was also annotated (right), e Bar chart illustrating cell type distribution in sham surgery (blue) and TAC surgery (red), f Average reduction of the accuracy of each module specific gene (module eigengene, ME) assigned by the random forest classifier (decreasing order of accuracy from top to bottom). g tSNE plot illustrating each ME expression. Module names were annotated by cell type-specific expression detected by each module network in (H). h Co-expression network of each module. The size of the dot represents the node centrality. i Gene ontology (GO) analysis of each module. The most characteristic GO terms in the top 4 ranked annotation clusters in the "functional annotation clustering" function with statistical significance (P<0.05) were extracted for each module. j mRNA expression levels of Htra3 in various organs of mice were assessed by real-time qPCR (n=4 in each organ, respectively). Data is presented as a mean and SD. k Western blot of Htra3 using cardiomyocytes and non-cardiomyocytes including cardiac fibroblasts.

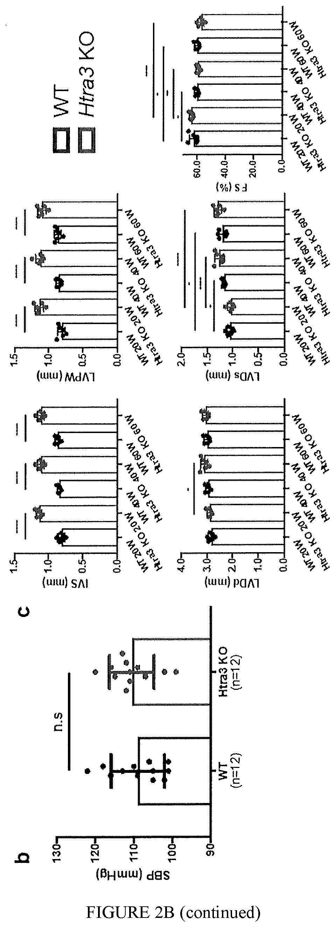

Fig. 2A illustrates that Htra3 knockout mice exhibit hypertrophy of the heart and cardiomyocytes and are vulnerable to mechanical stress. a Representative cardiac morphology of wild-type (WT) and Htra3 knockout (KO) mice at 2 weeks after TAC or sham surgery. b Echocardiographic assessment of hearts of wild type and Htra3 knockout mice after TAC or sham surgery. n=11 (wild-type sham), n=15 (wild-type TAC), n=10 (Htra3 knockout sham), n=15 (Htra3 knockout TAC). Data pieces were displayed as a mean and SD. *P<0.05, **P<0.01, ***P<0.005, ****P<0.001; Significance was determined by two-way analysis of variance (ANOVA) using Bonferroni's multiple comparison test. c Evaluation of cardiomyocyte cross-sectional area. WGA and DAPI were used to stain the plasma membrane and nuclei, respectively. The quantitative analysis results are also shown. Average data from approximately 40 to 100 cells per heart (n=3 each). Data pieces are displayed as a mean and SD. **P<0.01, ***P<0.005, ****P<0.001; Significance was determined by one-way analysis of variance using Tukey's or Dunnett's post-test. d Histochemical detection of collagen fibers by Sirius red/fast green dye staining in wild type and Htra3 knockout mice after TAC or sham surgery. The results of quantitative analysis of fibrotic areas are also shown (n=3 each). Data pieces are displayed as a mean and SD. *P<0.05, **P<0.01, ***P<0.005, ****P<0.001; Significance was determined by two-way ANOVA using Tukey's or Dunnett's post-test. e Violin plot showing Htra3 RNA expression in cardiac fibroblasts at 2 weeks after TAC (n=40) and sham surgery (n=109) in wild-type mice. f Violin plot illustrating HTRA3 expression levels (n=143 cells from control subjects (CTL), n=151 cells from heart failure (HF) patients). g mRNA expression levels of Htra3 and Tgfb1 in cultured cardiac fibroblasts after mechanical extension of 0 to 30% were assessed by real-time qPCR (n=5, 6, 6, 6 in each group). Data pieces are displayed as a mean and SD. *P<0.05, **P<0.01; Significance was determined by one-way analysis of variance (ANOVA) using Bonferroni's multiple comparison test.

Fig. 2B illustrates generation of Htra3 knockout mice and single cell analysis showing HTRA3 expression in the human heart, in relation to Fig. 2A. a Genomic structures of mouse Htra3 (top), targeting vector (middle), and target Htra3 gene (bottom). The 5' end of Htra3 exon 1 (1-424bp) was deleted. Genomic DNA from target ES cells was digested with Bam HI and subjected to southern blot analysis. The DIG-labeled probe (from intron 2 illustrated in gray lines) detected a 15kb fragment from the wild type Htra3 allele and a 11kb fragment from the target allele. Northern blots of total RNA prepared from wild-type (WT) and homozygous Htra3 knockout mice showed the absence of cardiac Htra3 transcripts. B, BamHI site; S, SalI sites; Xh, XhoI sites; No, NotI site. Open arrows indicate the PGK-neoPolyA cassette. Orange arrows indicate PMC-diphtheria toxin (DT-A)-PolyA cassette. b Comparison of systolic blood pressure of wild-type and Htra3 knockout mice. Significance was determined by an unpaired two-tailed Student's t-test. c Echocardiographic assessment of hearts from wild-type and Htra3 knockout mice at different stages of age. Data pieces are displayed as a mean and SD. The significance was determined by one-way ANOVA using Tukey's or Dunnett's post-test. *P<0.05, **P<0.01, ****P<0.001 d Morphological and histological comparison of multiple organs between wild-type and Htra3 knockout mice (scale bar, 100 µm). e UMAP plot of single cell analytical profile of human heart (N=2,459 cells). The expression levels of HTRA3 and LUM are also displayed. f Cell-type specific expression profiles on UMAP plots.

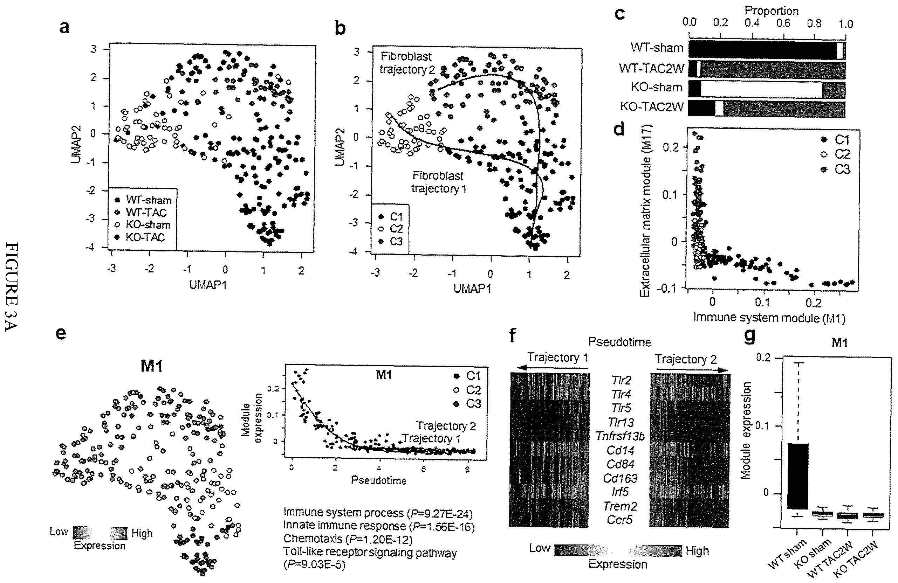

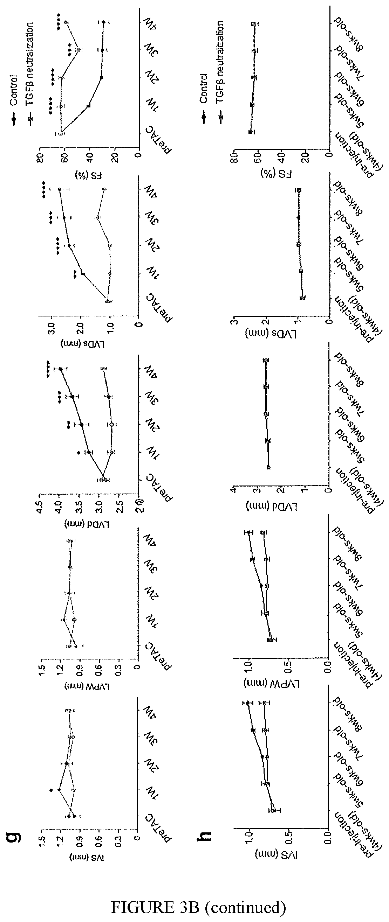

Fig. 3A illustrates that single-cell analysis of cardiac fibroblasts revealed that Htra3 maintains a quiescent state by TGF-β inhibition. a UMAP plot of single cell transcriptome of cardiac fibroblasts isolated from wild-type and Htra3 knockout mice at 2 weeks after TAC or sham surgery using antibodies against PDGFR-α (wild-type sham n=109 cells, wild-type TAC n=40 cells, knockout sham n=55 cells, knockout TAC n=53 cells). b Trajectory analysis of the UMAP plot of a. Clusters classified by graph-based clustering were displayed in color. The trajectory identified by the Slingshot algorithm is also displayed. c Bar chart illustrating the distribution of clusters in b. d Scatter plot illustrating module eigengene (ME) expression of M1 and M17 in each cell. e M1 expression on UMAP plot (left) and its dynamics along pseudotime (top right). The enriched GO terms and enhanced P-values are also displayed (bottom right). p-values were determined by Fisher's exact test. f Heatmap illustrating expression levels of selected M1 genes in the trajectory. g Box plot of M1 expression. Data represents box plots and individual data points. The box plots illustrate the median (centerline), the first and third quartiles (box edge) and the whisker goes from each quartile to the minimum or maximum. Wild-type-Sham, n=109; Knockout-Sham, n=55; Wild-type-TAC, n=40; and Knockout-TAC n=53. h M17 expression on UMAP plot (left) and its dynamics along pseudotime (top right). The enriched GO terms are also displayed (bottom right). p-values were determined by Fisher's exact test. i Heatmap illustrating expression levels of selected M17 genes in the trajectory. j Box plot of M17 expression. Data represents box plots and individual data points. The box plots illustrate the median (centerline), the first and third quartiles (box edge) and the whisker goes from each quartile to the minimum or maximum. Wild-type-Sham, n=109; Knockout-Sham, n=55; Wild-type-TAC, n=40; and Knockout-TAC n=53. k Western blot analysis using cardiac fibroblasts. TGF-β1 treatment was used as a positive control for pSmad2/3. l Immunoprecipitation followed by Western blot analysis showed binding of Htra3 to mature TGF-β1. m Immunostaining of pSmad2/3 in heart sections. Plasma membrane and nuclei were stained using pSmad2/3 (red), wheat germ agglutinin (WGA, Green), and 4',6-diamidino -2 phenylindole (DAPI, blue), respectively. The results of quantitative analysis were also shown (n=5, 5, 5, 3 biologically independent mice, left to right). Data pieces are displayed as a mean and SD. **P<0.01, ***P<0.005, ****P<0.001; Significance was determined by one-way analysis of variance using Tukey's or Dunnett's post-test.

Fig. 3B illustrates single cell analysis of cardiac fibroblasts and the effect of TGF-β neutralization in the TAC model, as associated with Fig. 3A. a Histogram illustrating the number of genes detected by single cell analysis of cardiac fibroblasts from wild-type and Htra3 knockout mice. Single cell transcriptomes with 3,000 or more genes detected were used for subsequent analysis. b Dendrogram illustrating clustered modules assigned using weighted gene correlation network analysis (WGCNA). c Mean decrease in accuracy (in order of decreasing accuracy from top to bottom) of each ME as assigned by the random forest classifier, d Module-specific expression profile on the UMAP plot. e Western blot analysis using cardiac fibroblasts. TGF-β1 treatment was used as a positive control for pSmad2/3. f Histochemical detection of collagen fibers by Sirius Red/Fast Green dye staining of wild-type and Htra3 knockout mice at 4 weeks after TAC with or without TGF-β neutralizing antibody treatment. g Echocardiographic assessment of heart in Htra3 knockout mice following TAC with or without TGF-β neutralizing antibody treatment (n=3 each). Data pieces are displayed as a mean and SD. *P<0.05, **P<0.01, ***P<0.005, ****P<0.001; Significance was determined by two-way ANOVA using Bonferroni's multiple comparison test. h Echocardiographic assessment of the heart of Htra3 knockout mice after injection of TGFβ antibody or IgG isotype control antibody (n=3 to 3). Data pieces are displayed as a mean and SD. Importance was determined by two-way ANOVA using Bonferroni's Multiple Comparisons Test. **P<0.01, ***P<0.005, and ****P<0.001

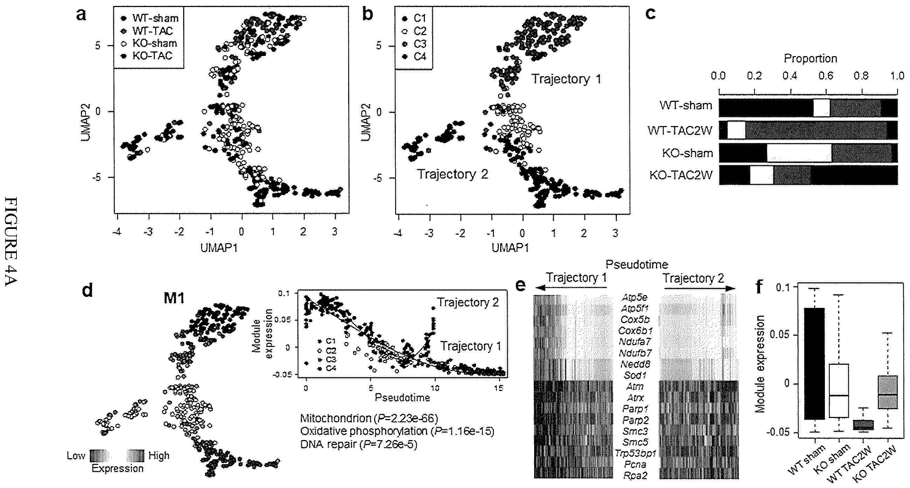

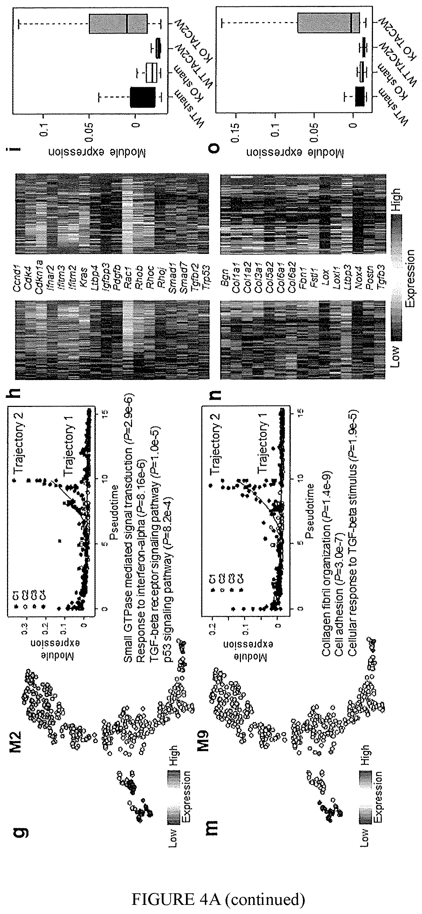

Fig. 4A illustrates that single-cell analysis of cardiomyocytes revealed that Htra3 prevents DNA damage and induction of failing cardiomyocytes with a secretory phenotype. a UMAP plot of single-cell transcriptome of cardiomyocytes isolated from wild-type and Htra3 knockout mice 2 weeks after TAC or sham surgery (wild-type sham n=175 cells, wild-type TAC n=87, knockout sham n=117, knockout TAC n=75). b Trajectory analysis of the UMAP plot of a. Clusters classified by graph-based clustering are displayed in color. The trajectory identified by the Slingshot algorithm is also displayed. c Bar chart illustrating the distribution of clusters in b. d M1 expression on UMAP plot (left) and its dynamics along pseudotime (top right). The enriched GO terms and enhanced P-values are also displayed (bottom right). p-values were determined by Fisher's exact test. e Heatmap illustrating expression levels of selected M1 genes in the trajectory. f Box plot of M1 expression. Data represents box plots and individual data points. The box plots illustrate the median (centerline), the first and third quartiles (box edge) and the whisker goes from each quartile to the minimum or maximum. Wild-type-Sham, n=175; Knockout-Sham, n=117; Wild-type-TAC, n=87; and Knockout-TAC n=75. g M2 expression on UMAP plot (left) and its dynamics along pseudotime (top right). The enriched GO terms are also displayed (bottom right). p-values were determined by Fisher's exact test. h Heatmap illustrating expression levels of selected M2 genes in the trajectory. i Box plot of M2 expression. Data represents box plots and individual data points. The box plots illustrate the median (centerline), the first and third quartiles (box edge) and the whisker goes from each quartile to the minimum or maximum. Wild-type-Sham, n=175; Knockout-Sham, n=117; Wild-type-TAC, n=87; and Knockout-TAC n=75. j Immunostaining of yH2A.X (yellow) and p21 (red) in heart sections of wild type and Htra3 knockout mice after TAC or sham surgery (4 weeks). WGA (green) and DAPI (blue) were used to stain the plasma membrane and nuclei, respectively. Arrows indicate gH2A.X/p21 positive nuclei. k Box plot illustrating expression of DNA damage-associated genes (Cdkn1a and Trp53) in single cardiomyocytes. Data represents box plots and individual data points. The box plots illustrate the median (centerline), the first and third quartiles (box edge) and the whisker goes from each quartile to the minimum or maximum. Wild-type-Sham, n=175; Knockout-Sham, n=117; Wild-type-TAC, n=87; and Knockout-TAC n=75. l Heatmap illustrating expression levels of selected M2 secretory factor genes in the trajectory. m M9 expression on UMAP plot (left) and its dynamics along pseudotime (top right). The enriched GO terms are also displayed (bottom right). p-values were determined by Fisher's exact test. n Heatmap illustrating expression levels of selected M9 genes in the trajectory. o Box plot of M9 expression. Data represents box plots and individual data points. The box plots illustrate the median (centerline), the first and third quartiles (box edge) and the whisker goes from each quartile to the minimum or maximum. Wild-type-Sham, n=175; Knockout-Sham, n=117; Wild-type-TAC, n=87; and Knockout-TAC n=75.

Fig. 4B illustrates single cell analysis of cardiomyocytes from wild-type and Htra3 knockout mice, in relation to Fig. 4A. a Histogram illustrating the number of genes detected by single cell analysis of cardiomyocytes of wild type and Htra3 knockout mice. Single cell transcriptomes with 4,000 or more genes detected was used for subsequent analysis. b Dendrogram illustrating clustered modules assigned using WGCNAs. c Mean decrease in accuracy (in order of decreasing accuracy from top to bottom) of each ME as assigned by the random forest classifier, d Module-specific expression profile on the UMAP plot. e Scatter plot illustrating the mean expression profile of single cell analysis of cardiomyocytes isolated from wild-type and Htra3 knockout mice after sham surgery. The enriched GO terms and associated P-values of the top 300 upregulated and downregulated genes are also displayed. Upregulation of genes associated with translation in Htra3 knockout cardiomyocytes suggests a transcriptional signature of enlarged cardiomyocytes in Htra3 knockout mice. f Immunostaining of yH2A.X (yellow) and p21 (red) in heart sections from Htra3 knockout mice 4 weeks after TAC surgery. WGA (green) and DAPI (blue) were used for staining the plasma membrane and nuclei, respectively. g Immunostaining of gH2A.X (Red) and PDGFRa (Green) in heart sections of wild-type and Htra3 knockout mice after TAC or sham surgery (4 weeks). Yellow arrows indicate yH2A.X/PDGFRa positive cardiac fibroblasts, and white arrows indicate gH2A.X positive cardiomyocytes. h Quantification of γH2A.X-positive nuclei in hearts of wild type and Htra3 knockout mice after TAC surgery (n=12 each). Data pieces were expressed as a mean +/-SEM. ****P<0.001; Significance was determined by unpaired two-tailed Student's t-test. i Hierarchical clustering of ME expression.

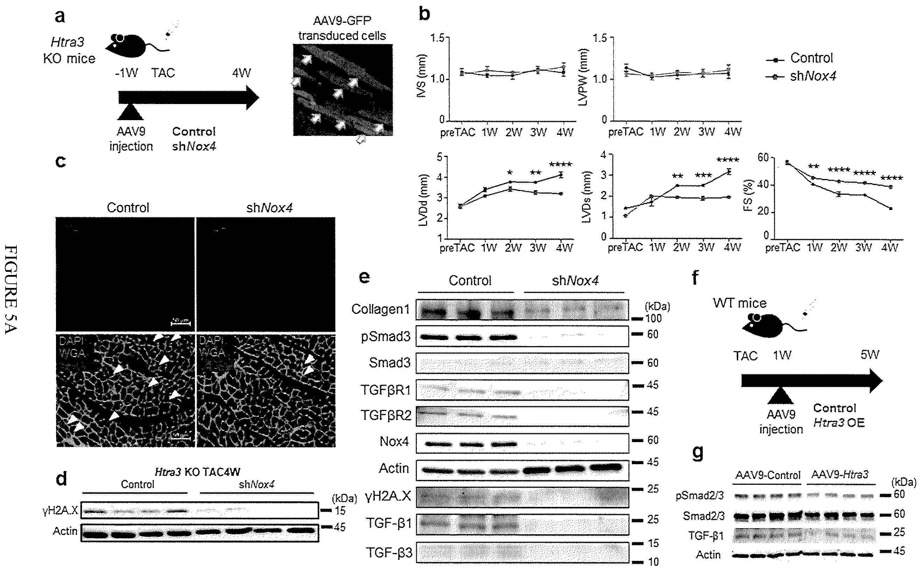

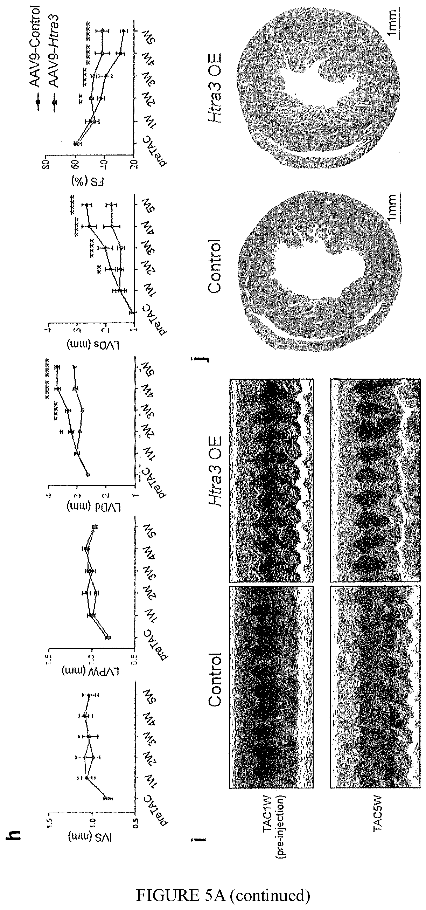

Fig. 5A illustrates that TGF-β induced Nox4 activation induces senescent cardiomyocytes and heart failure, a Experimental design to test the effects of shNox4 AAV9 vectors and validation of AAV9 transduction with AAV9-eGFP vectors. WGA (red) and DAPI (blue) were used to stain the plasma membrane and nuclei, respectively. Arrows indicate GFP introduced cells, b Echocardiographic assessment of hearts from TAC-induced Htra3 knockout mice after injection of control or shNox4 AAV9 vectors (n=4 each). Data pieces are displayed as a mean and SD. *P<0.05, **P<0.01, ****P<0.001; Significance was determined by two-way ANOVA using Bonferroni's multiple comparison test. The source data was provided as a source data file. c Immunostaining of γH2A.X on heart sections from TAC-induced Htra3 knockout mice after injection of control or shNox4 AAV9 vector. WGA (green) and DAPI (blue) were used to stain the plasma membrane and nuclei, respectively. Arrows indicate γH2A.X positive nuclei. d Western blot analysis of γH2A.X with isolated cardiomyocytes from TAC-induced Htra3 knockout mice after injection of control or shNox4 AAV9 vector. e Western blot analysis with isolated cardiomyocytes from TAC-induced Htra3 knockout mice after injection of control or shNox4 AAV9 vectors. f Experimental design to test the effect of AAV9-Htra3 vectors. g Western blot analysis using isolated cardiomyocytes from TAC-induced mice after injection of AAV9-control or AAV9-Htra3 vectors. h Echocardiographic assessment of hearts from TAC-induced mice after injection of AAV9-control or AAV9-Htra3 vectors (n=7 each). Data pieces are displayed as a mean and SD. *P<0.05, **P<0.01, ****P<0.001; Significance was determined by two-way ANOVA using Bonferroni's multiple comparison test. The source data was provided as a source data file. i Representative echocardiographic images of wild-type mice injected with either AAV9-control or AAV9-Htra3. j Histochemical detection of collagen fibers by Sirius red/fast green dye staining of hearts from TAC-induced mice after injection of AAV9-control or AAV9-Htra3 vectors.

Fig. 5B shows functional and transcriptional analysis of shNox4-induced and p53 knockout mice in relation to Fig. 5A. a Representative echocardiographic images of sh-control or shNox4-induced mice 4 weeks after TAC surgery. b Echocardiographic assessment of hearts from wild-type and Htra3 knockout mice following infection with AAV9-Nox4 overexpression (OE) or control vectors. n=3 (wild-type control), n=5 (wild-type Nox4 OE), n=3 (Htra3 knockout control), n=5 (Htra3 knockout Nox4 OE). Data pieces are displayed as a mean and SD. **P<0.01, ***P<0.005, and ****P<0.001 Nox4 OE vs. control (Wild type is blue, Htra3 knockout is red), †P<0.05, and ††P<0.01 Wild Type Nox4 OE vs. Htra3 Knockout Nox4 OE, ‡P<0.001 Htra3 Knockout (both Nox4 OE and control) vs. Wild Type (both Nox4 OE and control); Significance was determined by two-way analysis of variance (ANOVA) using Bonferroni's multiple comparison test. The source data is provided as a source data file. c Immunostaining of γH2A.X (red) in heart sections from wild-type and Htra3 knockout mice after TAC and sham surgery (4 weeks). Yellow arrows indicate γH2A.X positive cardiomyocytes. d Boxplots illustrating expression profiles of TGF-β signaling molecules, secretory factors, and mitochondrial genes in cardiomyocytes isolated from WT and cardiomyocyte-specific p53 knockout mice 2 weeks after TAC (WT n=48 cells, p53 KO n=48). Single cell analyzed data is from Document 12.

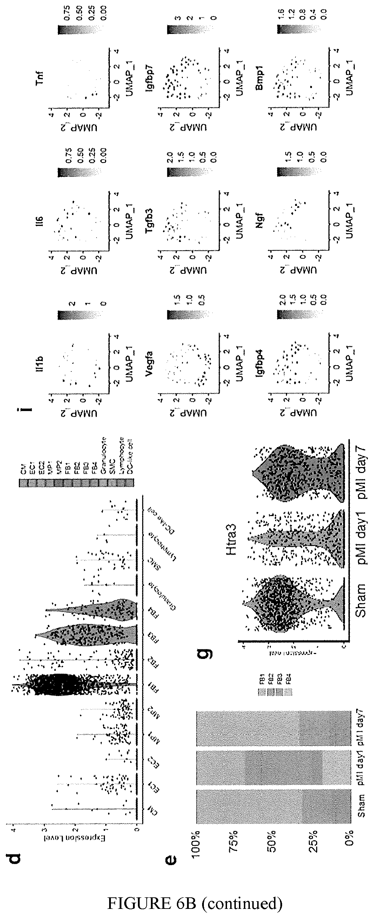

Fig. 6A illustrates that spatial transcriptomic analysis reveals Htra3 repression-mediated spatial induction of senescent failing cardiomyocytes with secretory phenotype. a Spatial expression profile of Htra3 across cardiac tissue sections of mice following sham or MI surgery. The dashed circle represents the infarction region. b Histochemical detection of collagen fibers by Sirius Red/Fast Green dye staining in wild-type and Htra3 knockout mice at 4 weeks after myocardial infarction (MI) surgery. c Echocardiographic assessment of hearts of wild type (wild type) and Htra3 knockout mice after MI surgery. n=12 (wild type), n=6 (knockout). Data pieces are displayed as a mean and SD. *P<0.05, **P<0.01, ***P<0.005; Significance was determined by two-way analysis of variance (ANOVA) using Bonferroni's multiple comparison test. d Hematoxylin and eosin stained tissue sections of the infarcted hearts and corresponding spatial distributions of clusters characterized by specific expression profiles (left). The dashed circle represents the infarction region. The color of the spatial points corresponded to a bar chart showing the distribution of clusters for each section (right). The number of analyzed spots is also displayed. e Differential gene expression analysis showing up regulated and down regulated genes in all five clusters. The regulated p-values <0.05 and log₂FC>0.25 are shown in red and the others in black. f Dot plot representing the distribution of cell types predicted based on single cell analysis of cells isolated from mice after sham or MI surgery. The dot size represents the average of the prediction scores of each spot in each cluster. g Single-cell trajectory analysis of cardiomyocytes isolated from the infarct zone. h Expression dynamics of genes characteristic of senescent failing cardiomyocytes along pseudotime. i Spatial expression pattern of genes characteristic of senescent cardiomyocytes. The dashed circle represents the infarction region.

Fig. 6B illustrates spatial induction via Htra3 inhibition of failing cardiomyocytes with secretory phenotype by spatial transcriptome analysis and single cell analysis of cardiomyocytes after MI, related to Fig. 6A. a Heatmap illustrating expression levels of representative genes of each cluster. b Enriched GO terms and enriched *P*-values associated with representative genes of Cluster 2. c UMAP plot of single-cell transcriptome of non-cardiomyocytes from sham and infarcted hearts (n=9,029 cells; Sham 2,673 cells, pMI Day 1 2,580 cells, pMI Day 7 3,776 cells).

CM, Cardiomyocytes. EC, Endothelial cells. MP; Macrophages; FB, Fibroblasts. SMC, Smooth muscle cells. DC, dendritic cells, d Violin plots showing Htra3 RNA expression in each cell cluster on sham, day 1 post-MI (pMI), and day 7 pMI. e Bar plots illustrating the distribution of FB clusters on sham, day 1 post-MI (pMI), and day 7 pMI. f Enriched GO terms for characteristic genes of FB2 and FB4 clusters. g Violin plots showing total expression levels of Htra3 on sham, post MI (pMI) day 1, and pMI day 7. h Heatmap illustrating expression patterns of genes characteristic of senescent failing cardiomyocytes and adaptive cardiomyocytes isolated from the infarct zone of mice 7 days after MI surgery. A UMAP plot of all of the cells is also displayed. i Expression pattern of characteristic genes on UMAP plot. j Spatial expression pattern of genes characteristic of cardiomyocyte dysfunction. The dashed circle represents the infarction region.

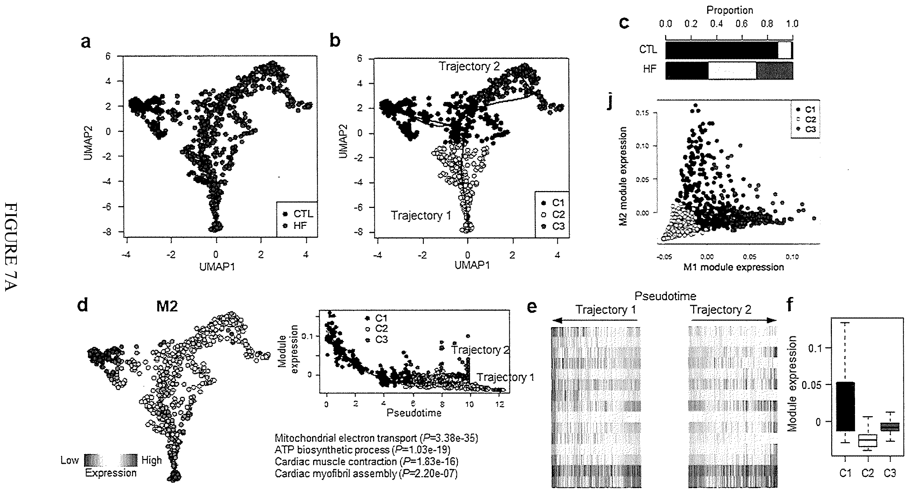

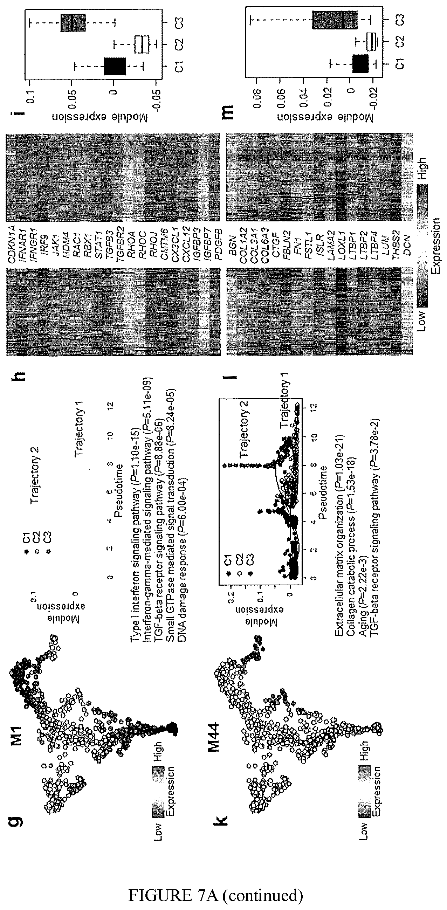

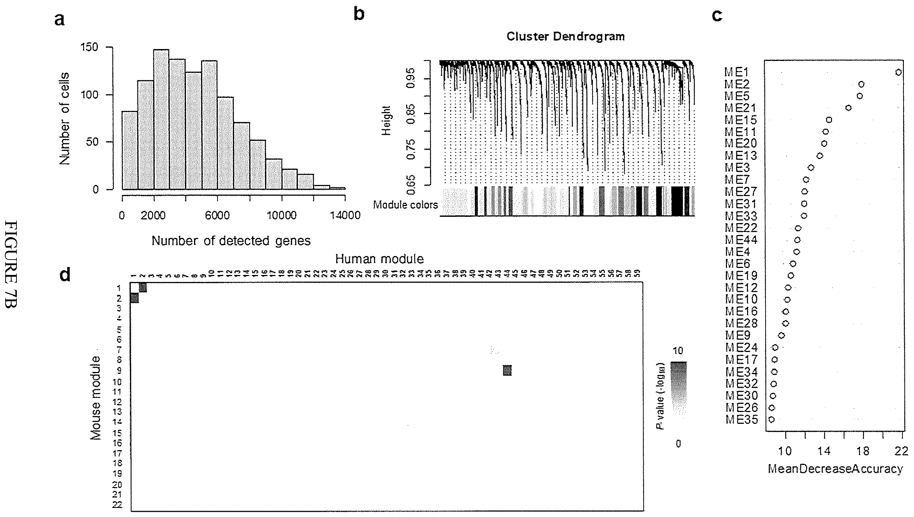

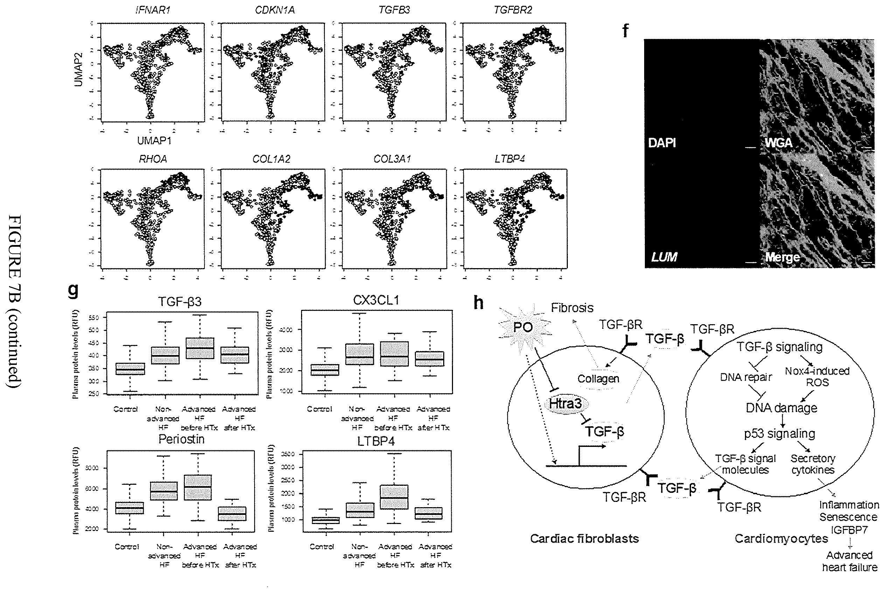

Fig. 7A illustrates single-cardiomyocyte RNA-seq and plasma proteome analysis of heart failure patients. a UMAP plot of single-cell transcriptome of cardiomyocytes isolated from control subjects (CTL) or heart failure (HF) patients (CTL n=85 cells, HF n=678). b Trajectory analysis of the UMAP plot of a. Clusters classified by graph-based clustering are displayed in color. The trajectory identified by the Slingshot algorithm is also displayed. c Bar chart illustrating the distribution of clusters in b. d M2 expression on UMAP plot (left) and its dynamics along pseudotime (top right). The enriched GO terms are also displayed (bottom right). e Heatmap illustrating expression levels of selected M2 genes in the trajectory. f Box plot of M2 expression. Data represents box plots and individual data points. The box plots illustrate the median (centerline), the first and third quartiles (box edge) and the whisker goes from each quartile to the minimum or maximum. C1, n=300; C2, n=267; and C3, n=196. g M1 expression on the UMAP plot (left) and its dynamics in the trajectory (top right). The enriched GO terms and enhanced P-values are also displayed (bottom right). h Heatmap illustrating expression levels of selected M1 genes in the trajectory. i Box plot of M1 expression. Data represents box plots and individual data points. The box plots illustrate the median (centerline), the first and third quartiles (box edge) and the whisker goes from each quartile to the minimum or maximum. C1, n=300; C2, n=267; and C3, n=196. j Scatter plot illustrating ME expression of M1 and M2 in each cell. k M44 expression on UMAP plot (left) and its dynamics along pseudotime (top right). The enriched GO terms are also displayed (bottom right). l Heatmap illustrating expression levels of selected M44 genes in the trajectory. m Box plot of M44 expression. Data represents box plots and individual data points. The box plots illustrate the median (centerline), the first and third quartiles (box edge) and the whisker goes from each quartile to the minimum or maximum. C1, n=300; C2, n=267; and C3, n=196. n Box plot of plasma protein levels of IGFBP7 (top) and NT-proBNP (bottom). RFU, Relative fluorescence unit. HF, Heart failure. HTx, Heart transplant. Data represents box plots and individual data points. The box plots illustrate the median (centerline), the first and third quartiles (box edge) and the whisker goes from each quartile to the minimum or maximum. n=768 is control, n=85 is less advanced HF, n=30 is advanced HF before HTx, and n=30 is advanced HF after HTx. o Average reduction in the accuracy of proteins involved in the classification between manifest and advanced heart failure assigned by the random forest classifier (order of decreasing accuracy from top to bottom). p Receiving-operating-characteristic (ROC) curves based on plasma protein levels of NT-proBNP and IGFBP7 to predict the incidence of heart failure. The area under the curve for each marker is also displayed. NT-proBNP was significantly superior in its ability to diagnose heart failure (P value =1.109e-07). The area under the ROC curve was calculated using logistic regression. q ROC curves based on plasma protein levels of NT-proBNP and IGFBP7 to identify patients with advanced heart failure. The area under the curve for each marker is also displayed. Although not statistically significant, IGFBP7 was highly capable of determining the severity level of heart failure (P-value =0.1063). The area under the ROC curve was calculated using logistic regression.

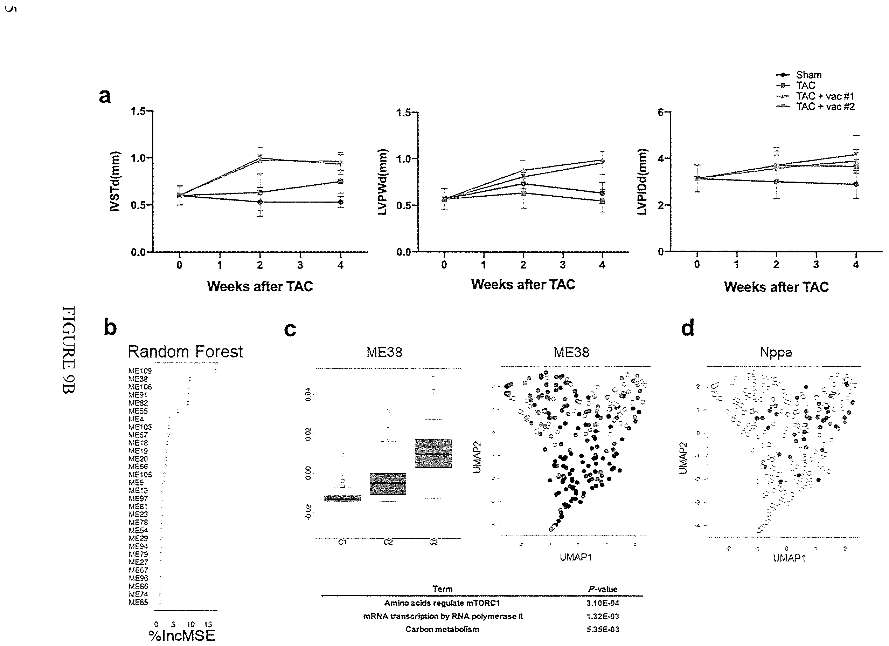

Fig. 7B illustrates single cell and plasma proteome analyses of heart failure patients in relation to Fig. 7A. a Histogram illustrating the number of genes detected by single cell analysis of cardiomyocytes from control subjects (n=2) and patients with heart failure (n=22). Single cell transcriptomes with more than 2,000 genes detected was used for subsequent analysis. b Dendrogram illustrating clustered modules assigned using WGCNAs. c Mean decrease in accuracy (in order of decreasing accuracy from top to bottom) of each ME as assigned by the random forest classifier. d Cross-tabulation table of cardiomyocyte modules obtained from mouse single cell analysis (Figs. 3A and 7B) and human single cell analysis (Figs. 4A and 9B). The table is colored with -logic (P-value) obtained using a two-tailed Fisher's exact test.

The yellow and red modules are largely conserved (the enrichment P values of all modules were >1×10⁻⁵). e Module-specific expression profile on the UMAP plot. f Single molecule RNA in situ hybridization of LUM in cardiac tissue of heart failure patients. Scale bar, 20 µm. g Box plot of plasma protein levels of secreted cytokines. RFU, Relative fluorescence unit. HF, Heart failure. HTx, Heart transplant. Data represents box plots and individual data points. The box plots illustrate the median (centerline), the first and third quartiles (box edge) and the whisker goes from each quartile to the minimum or maximum. n=control, 768; non-advanced HF, n=85; pre-HTx advanced HF, n=30; and post-HTx advanced HF, n=30. h Schematic representation of the mechanism by which Htra3 of cardiac fibroblasts prevents cardiomyocyte aging and heart failure induced by TGF-β signaling. PO, Pressure overload

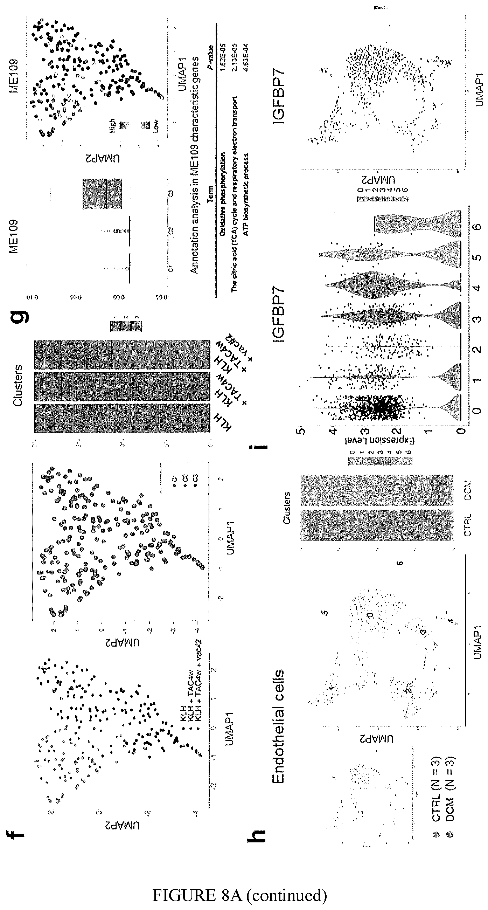

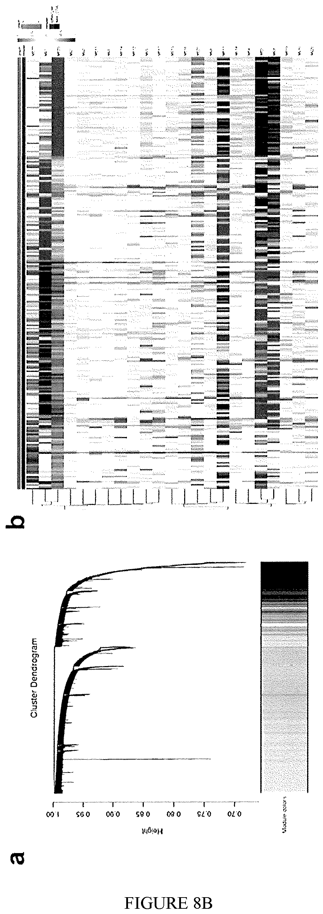

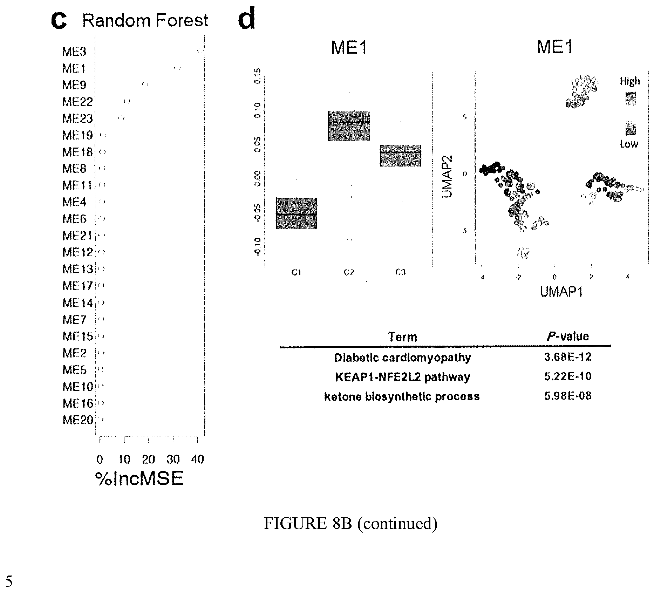

Fig. 8A illustrates that Igfbp7 from aged endothelial cells reduces cardiac function. a Echocardiographic analysis of pressure overload heart failure mice treated with AAV9-CMV-Igfbp7. Control (n=6), Igfbp7 (n=7), FS, fractional shortening. Data are shown as mean ± standard deviation. P-values were determined using a two-tailed Mann-Whitney U-test. b (Left, Center) Results of single cell analysis using WGCNAs and graph-based clustering of cardiomyocytes from mice injected with control vector and AAV9-Igfbp7. (Right) Expression level of Igfbp7 in UMAP. c (left) Results for cell populations classified by cluster. (Middle) Box plot and (right) UMAP plot colored by gene expression of ME3 module. (Bottom) Results of annotation analysis of ME3 gene. d Evaluation of murine antibody titers against Igfbp7 by ELISA after vaccination (n=5). e Echocardiographic analysis of pressure overload heart failure mice following Igfbp7 vaccination. Control (n=5), first Igfbp7 vaccine (N=5), second Igfbp7 vaccine (N=5), TAC, transverse aortic stenosis, FS, fractional shortening. f (Left, Center) Results of single cell analysis using WGCNAs of cardiomyocytes visualized with UMAP, N=243. (Right) Results of cell populations classified by clusters. g (Left) Box plot and (right) UMAP plot colored by gene expression of ME109 module. (Bottom) Annotation analysis of the ME109 generation. h Single nuclear RNA-seq from DCM patients without heart disease (N=3) and volunteers (N=3). Single-cell analysis of cardiomyocytes, resulted in dimensionality reduced by PCA and visualization by UMAP and colored by sample type (left) and UMAP colored by graph-based clustering (center). (Right) Results for cell populations classified by cluster. i IGFBP7 expression levels and UMAP on violin plots classified by cluster.

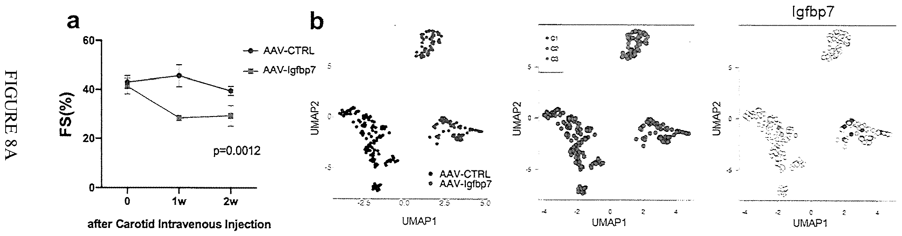

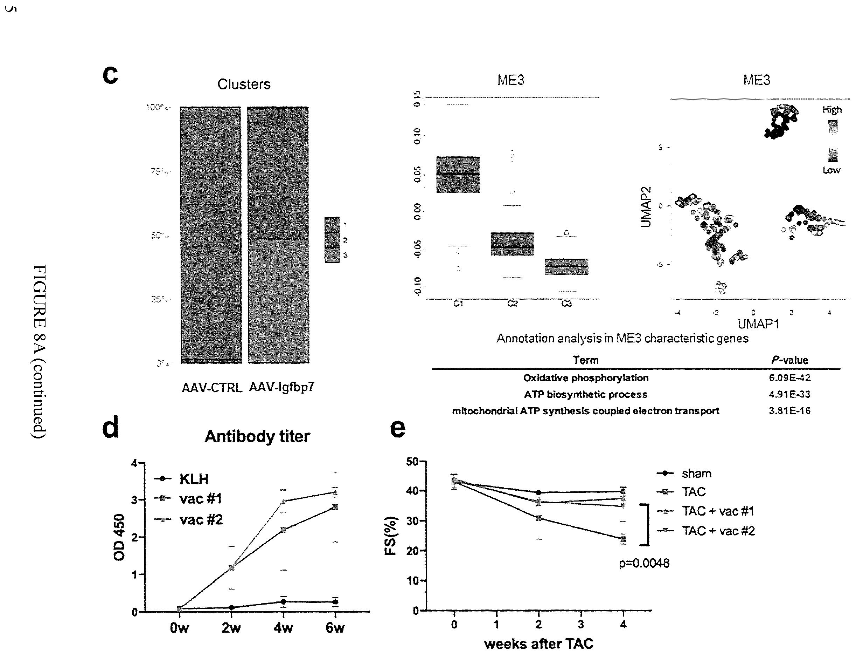

Fig. 8B illustrates single cell analysis of cardiomyocytes treated with AAV-Igfbp7 and a control vector, related to Figs. 8Ab and 8Ac. a Cluster dendritic diagram of cardiomyocytes from single cell analysis. b Heatmap classified into three clusters (clusters 1 to 3). c Random forest results. d (Left) Box plot and (right) UMAP plot colored by gene expression of ME1 module. (Bottom) Results of annotation analysis of ME1 gene.

Fig. 9A illustrates RNA-seq analysis of human iPSCM treated with IGFBP7. a (Top) Cultured human iPS cell-derived cardiomyocytes were treated with human recombinant IGFBP7 and incubated for 24 hours. Heatmap of bulk RNA-seq results for human iPSCMs treated with vehicle (n=3) or human recombinant IGFBP7 (n=4).

The DESeq2 package was used to select the differential expressed genes (DEG). (Bottom) Annotation analysis of Cluster 2 gene. b (Top) Heatmap of bulk RNA-seq results for human iPSCMs treated with human recombinant IGFBP7 (n=4) or IGFBP7 and serum from vaccinated mice (n=4). The DESeq2 package was used to select the differential expressed genes (DEG). (Bottom) Annotation analysis of Cluster 3 gene.

Fig. 9B illustrates functional analysis of vaccinated mice and single cell analysis of mouse cardiomyocytes pretreated with IGFBP7 vac#2. a Echocardiographic assessment of mouse hearts following TAC or sham surgery. n=3 (sham), n=6 (TAC), n=6 (TAC and vac#1), and n=6 (TAC, TAC and vac#2). b Results of random forest in single cell analysis of cardiomyocytes from vaccinated mice. c (Left) Box plot and (right) UMAP plot color-coded by gene expression of ME38 modules. (Bottom) Results of annotation analysis of ME38 gene. d Nppa expression level in UMAP.

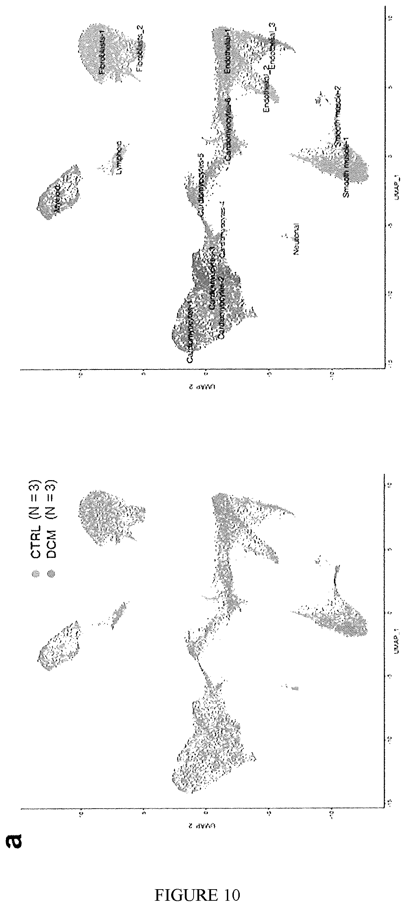

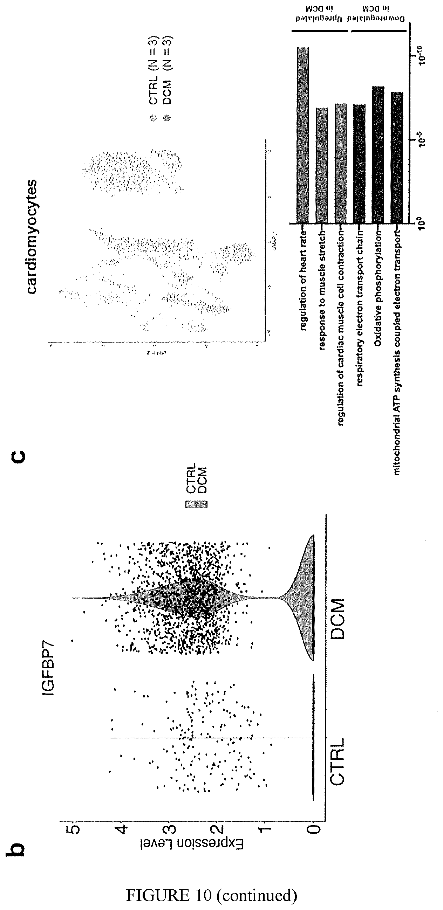

Fig. 10 illustrates single nuclear RNA-seq analysis in DCM patients and control patients. a As a result of single nuclear RNA-seq analysis in DCM patients (n=3) and control patients (n=3), dimensionality was reduced by PCA, visualized by UMAP, (left) colored by sample type, (right) annotated clusters. b Results of violin plots of endothelial cells divided by patient status. c (top) Results of UMAP of cardiomyocytes sorted by patient status. (Bottom) Annotation analysis of characteristic genes in DCM patient cardiomyocytes was compared to control patients.

### DETAILED DESCRIPTION OF THE INVENTION

According to the present invention, there is provided an agent for regulating a DNA repair mechanism of cardiac fibroblasts, the regulation agent comprising at least one of the above (a), (b), (c), (d), and (e) (in the present specification, it may be referred to as a regulation agent of the present invention).

"High-temperature requirement A serine peptidase 3" (Serine protease HtrA3), which is an active ingredient of the present invention, is a proteinase also called a high-temperature requirement A3 serine protease. In the present invention, the serine protease HtrA3 is preferably derived from human or mouse.

Both the base sequence encoding human serine protease HtrA3 and the base sequence encoding mouse serine protease HtrA3 are based on the base sequence published in the database (https://www.ncbi.nlm.nih.gov/gene) of U.S. National Center for Biotechnology Information (NCBI). Specifically, the human base sequence is based on the base sequence published in NCBI Gene ID: 94031, and the mouse base sequence is based on the base sequence published in NCBI Gene ID: 78558. The amino acid sequence of human serine protease HtrA3 and the amino acid sequence of mouse serine protease HtrA3 are both based on the amino acid sequences published in the database of U.S. National Center for Biotechnology Information (NCBI) (same as above). Specifically, the human amino acid sequence is based on the amino acid sequence published in NCBI Reference Sequence: NP_444272.1, and the mouse amino acid sequence is based on the amino acid sequence published in NCBI Reference Sequence: NP_084403.2. The transformed cells of (d) and the antibody or fragment thereof of (e) can be prepared by a method well known to those skilled in the art based on the sequence information. In addition, as the base constituting the nucleic acid in the present invention, in addition to a natural base, a modified base or an artificial base having in vivo stability can also be used.

In the present invention, the "DNA repair mechanism" is a mechanism for repairing DNA damage (damaged DNA). In the present invention, "DNA damage" means a single-stranded break and/or double-stranded break generated in nuclear DNA, and the DNA repair mechanism means a mechanism for repairing the single-stranded break and/or double-stranded break. In the present invention, "regulation of a DNA repair mechanism" means promoting a DNA repair mechanism, and includes inhibiting suppression of a DNA repair mechanism.

Cardiac fibroblasts produce TGF-β, which plays a physiologically important role, such as repairing wounds by promoting fibrosis. On the other hand, when the action of TGF-β is too strong in cardiac fibroblasts, accumulation of DNA damage in cardiac fibroblasts and cardiomyocytes, cardiac fibrosis, cardiac fibrosis, myocardial infarction, and heart failure are caused. Htra3 which is an active ingredient of the agent for regulating the present invention acts on TGF-β and decomposes TGF-β, thereby inhibiting accumulation of DNA damage caused by TGF-β, cardiac fibrosis, cardiac fibrosis, myocardial infarction, and heart failure.

Therefore, according to the present invention, there is provided a pharmaceutical composition (in the present specification, it may be referred to as a pharmaceutical composition 1 of the present invention) for the treatment or prevention of myocardial infarction, cardiac fibrosis or heart failure, containing the regulation agent of the present invention or at least one of the above (a), (b), (c), (d) and (e) as an active ingredient. According to the present invention, there is also provided a method of regulating a DNA repair mechanism of cardiac fibroblasts and a method for treating or preventing myocardial infarction, cardiac fibrosis or heart failure, including administering at least one of the above (a), (b), (c), (d), and (e) to a subject in need thereof. In the present invention, the heart failure is preferably heart failure with reduced ejection fraction (HFrEF). The heart failure with reduced ejection fraction results in poor left ventricular contraction and insufficient ejection, resulting in increased diastolic volume, increased diastolic pressure, and reduced ejection fraction.

In a case where the active ingredient of the present invention is administered to a subject, the administration route is not particularly limited as long as a desired therapeutic or preventive effect is obtained, but oral administration or parenteral administration (for example, intravenous administration, subcutaneous administration, or intraperitoneal administration) can be selected.

Examples of oral administration agent include granules, powders, tablets (including sugar coating tablets), pills, capsules, syrups, solutions, jellies, emulsions, and suspensions. As the parenteral administration agent, an appropriate dosage form can be selected depending on the specific administration route, and examples thereof include injections and suppositories. These formulations can be formulated using a pharmaceutically acceptable carrier by procedures commonly practiced in the art (for example, a known method described in General Rules for Preparations of the Japanese Pharmacopoeia, 18th edition, or the like). Examples of the pharmaceutically acceptable carrier include an excipient, a binder, a diluent, an additive, a fragrance, a buffer, a thickener, a colorant, a stabilizer, an emulsifier, a dispersant, a suspending agent, and a preservative.

The active ingredient of the present invention can be administered to a subject as a gene therapeutic agent. Gene therapy can be performed by methods known to those skilled in the art (Cannata A, Ali H, Sinagra G, Giacca M. Gene Therapy for the Heart Lessons Learned and Future Perspectives. Circ Res. 2020 May 8;126(10):1394-1414. doi:10.1161/CIRCRESAHA.120.315855. Epub 2020 May 7. PMID:32379579), for example, a nucleic acid or a fragment thereof can be administered using a delivery reagent, or an expression vector incorporating a nucleic acid or a fragment thereof can be administered.

In a case where the pharmaceutical composition 1 of the present invention is provided as a gene therapeutic agent, it can be a composition containing the nucleic acid of the active ingredient (b) or a fragment thereof (for example, naked DNA) together with a delivery reagent. Examples of delivery reagents include, but are not limited to, lipophilic reagents, lipofectin, lipofectamine, serfectin, cationic polymers (for example, polylysine), micelles, PEGylated liposomes or nanoparticles, oligonucleotide nanoparticles, and combinations thereof.

In a case where the pharmaceutical composition 1 of the present invention is provided as a gene therapeutic agent, it may also be a composition containing an expression vector of the active ingredient (c), optionally together with a delivery reagent. Examples of the expression vector include, but are not limited to, a viral vector system (for example, adeno-associated viral vectors (AAV vectors), cytomegalovirus vectors (CMV vectors), lentiviral vectors, retroviral vectors, adenoviral vectors, herpesviral vectors, or vaccinia viral vectors), and a non-viral vector system such as a plasmid.

In a case where the gene therapeutic agent of the present invention is administered to a subject, the gene therapeutic agent can be administered, for example, by intravenous administration, intraperitoneal administration, subcutaneous administration, local administration to the heart, or administration via the coronary artery. It is needless to say that the gene therapeutic agent can also be formulated using a pharmaceutically acceptable carrier by a method usually performed in the art as described above.

The dose of the active ingredient in the present invention can be determined depending on the type of the active ingredient, the sex, age, body weight, symptom, dosage form, administration route, and the like of the subject to be administered. In the present invention, in a case where the active ingredient is administered for the purpose of treating or preventing myocardial infarction, cardiac fibrosis or heart failure, the dose per adult can be determined, for example, in the range of 0.0001 mg to 1000 mg/kg of body weight, but is not limited thereto. In addition, the active ingredient in the above dosage can be administered once a day or in 2 to 4 divided doses. The active ingredient of the present invention can be administered not only to humans in need thereof but also to mammals other than humans (for example, mouse, rat, rabbit, dog, cat, cow, horse, pig, sheep, goat, monkey).

According to the present invention, there is provided a non-human mammalian heart failure model animal in which expression of serine protease HtrA3 in cardiac fibroblasts is reduced or inhibited (herein may be referred to as model animal 1 of the present invention). Animal species of the model animal 1 of the present invention include non-human mammals such as rodents (for example, mice and rats), cows, horses, pigs, monkeys, and dogs.

The reduction or inhibition of expression of the serine protease HtrA3 in the model animal 1 of the present invention can be carried out by a method known to those skilled in the art, and for example, an animal produced by deleting a sequence portion or a control region of the serine protease HtrA3 gene by genetic manipulation to eliminate the function (knockout) can be used, and examples of such a model animal include knockout mice.

In the present invention, "the HtrA3 gene is knocked out" refers to a state in which the expression product of the HtrA3 gene is not expressed at all, or even when expressed, the function of the normal HtrA3 gene product cannot be exhibited, and the function of the HtrA3 gene is deficient, due to modification of the base sequence of the HtrA3 gene or the like.

In the model animal 1 of the present invention, the specific mode of deleting the function of the HtrA3 gene is not particularly limited as long as the HtrA3 gene is knocked out, but includes, for example, a mode in which at least a part of the HtrA3 gene is modified or the promoter of the HtrA3 gene is inactivated.

In the present specification, "at least a part of the HtrA3 gene is modified" means that a modification causing deletion, substitution, or addition is added to at least a part of the base sequence of the HtrA3 gene. In order to knockout the HtrA3 gene, one or more mutations among deletion, substitution, and addition may be combined with respect to the HtrA3 gene.

In addition, in the present specification, "deletion" of at least a part of the base sequence of the HtrA3 gene means that the expression product of the HtrA3 gene is modified so as not to express the function as the HtrA3 protein by deleting a part or all of the HtrA3 gene. In the present invention, "substitution" of at least a part of the base sequence of the HtrA3 gene means that a part or all of the HtrA3 gene is substituted with a separate sequence not related to the HtrA3 gene, thereby modifying the expression product of the HtrA3 gene so that the function of the HtrA3 gene is not expressed. Furthermore, in the present invention, "adding" to at least a part of the base sequence of the HtrA3 gene means that a sequence other than the HtrA3 gene is added to the HtrA3 gene, thereby modifying the expression product of the HtrA3 gene so that the function as the HtrA3 protein is not expressed.

The model animal 1 of the present invention in which the HtrA3 gene is knocked out may be a heterozygote knock-out animal in which either one of the alleles in the HtrA3 gene is knocked out, or may be a homozygote knock-out animal in which both functions of the alleles are knocked out. The model animal 1 of the present invention may be either female or male.

The model animal 1 of the present invention in which the HtrA3 gene is knocked out can be produced by a method known to those skilled in the art. Examples of such a technique include, but are not limited to, a genome editing technique such as a CRISPR/Cas9 method or a transcription activation-like effector nuclease (TALEN) method, and a gene disruption technique by a homologous recombination method.

According to the present invention, there is provided a method for screening for an agent or method of the regulation of DNA repair mechanisms in cardiac fibroblasts, including the steps (A) and (B) (which may be referred to herein as Screening Method 1 of the present invention). According to the present invention, there is also provided a method for screening for an agent or method of the regulation of DNA repair mechanisms in cardiac fibroblasts, the method including the steps (C) and (D) (sometimes referred to herein as the screening method 2 of the present invention).

Examples of the test factor in Screening Methods 1 and 2 of the present invention include, but are not limited to, a low molecular weight compound, a middle molecular weight compound, a peptide, a protein, an antibody, and the like. In Screening Method 2, in order to treat a non-human mammal in which the expression of Htra3 is reduced or inhibited with the test factor, the test factor may be formulated into an optimal dosage form according to the administration route.

As the "cells in which the expression of HtrA3 is reduced or inhibited" used in the step (A) of Screening Method 1 of the present invention, cardiomyocytes or cardiac fibroblasts of human or non-human mammal in which the expression of serine protease HtrA3 is reduced or inhibited can be used, and the cells can be prepared according to the method of the knockout model animal described above. In addition, after the comparison procedure of the step (B) of Screening Method 1 of the present invention, in a case where the DNA damage response of cardiomyocytes or cardiac fibroblasts is reduced in the cells as compared to untreated control cells or wild-type cells, the test factor can be determined to be an agent candidate useful for regulating the DNA repair mechanism of cardiac fibroblasts.

"Treating with the test factor" in the step (C) of Screening Method 2 of the present invention means, for example, administering the test factor to a non-human mammal (model animal 1 of the present invention). The administration route can be appropriately selected according to the type of the test factor and the like. After the comparison procedure of the step (D) of Screening Method 2 of the present invention, the test factor can be determined to be an agent candidate useful for regulating the DNA repair mechanism of cardiac fibroblasts when cardiac dysfunction and/or fibrosis is improved.

According to the present invention, there is provided a method of diagnosing myocardial infarction, cardiac fibrosis or heart failure (which may be referred to as Diagnosis Method 1 of the present invention in the present specification) including the step (E). In the step (E), in a case where the expression level of serine protease HtrA3 in cardiac fibroblasts of a subject is significantly reduced as compared with the expression level of Htra3 in cardiac fibroblasts of a healthy subject, it can be determined that the subject may suffer from myocardial infarction, cardiac fibrosis, or heart failure, that is, there is a risk of suffering from myocardial infarction, cardiac fibrosis, or heart failure. The expression level of serine protease HtrA3 in cardiac fibroblasts of a subject and a healthy subject can be measured using a nucleic acid amplification method such as quantitative PCR for a biopsy sample of the subject. Diagnosis Method 1 of the present invention can be performed in vitro. In addition, in Diagnosis Method 1 of the present invention, a method including a determination step by a doctor can be excluded.

Since Htra3 is used as a marker for examination of myocardial infarction, cardiac fibrosis or heart failure in Diagnosis Method 1 of the present invention, according to another aspect of the present invention, use of serine protease HtrA3 or a nucleic acid encoding the same or a fragment thereof as a marker for diagnosing myocardial infarction, cardiac fibrosis or heart failure is provided.

According to the present invention, there is provided a pharmaceutical composition (in the present specification, it may be referred to as a pharmaceutical composition 2 of the present invention) for the treatment or prevention of myocardial infarction or heart failure, including at least one of the above (f), (g), (h), and (i). According to the present invention, there is also provided a method for treating or preventing myocardial infarction or heart failure containing administering at least one of the above (f), (g), (h), and (i) to a subject in need thereof.

"Insulin-like growth factor-binding protein 7" (IGFBP7), which is an active ingredient of the present invention, is a secreted protein also called insulin-like growth factor binding protein 7. In the present invention, the IGFBP7 is preferably derived from human or mouse.

Both the base sequence encoding human insulin-like growth factor binding protein 7 (IGFBP7) and the base sequence encoding mouse IGFBP7 are based on the base sequence published in the database of the U.S. National Center for Biotechnology Information (NCBI) (same as above). Specifically, the human base sequence is based on the base sequence published in NCBI Gene ID: 3490, and the mouse base sequence is based on the base sequence published in NCBI Gene ID: 29817. The amino acid sequence of human IGFBP7 and the amino acid sequence of mouse IGFBP7 are both based on the amino acid sequences published in the database of the U.S. National Center for Biotechnology Information (NCBI) (same as above). Specifically, the human amino acid sequence is based on the amino acid sequence published in NCBI Reference Sequence: NP_001544.1, and the mouse amino acid sequence is based on the amino acid sequence published in NCBI Reference Sequence: NP_001152990.2. An expression vector containing the antibody (g) or a fragment thereof, a nucleic acid or a fragment thereof encoding the antibody (h) or a fragment thereof, and a nucleic acid or a fragment thereof encoding the antibody (i) or a fragment thereof can be prepared by a method known to those skilled in the art based on this sequence information.

Examples of the nucleic acid (f) having a complementary base sequence include nucleic acids targeting IGFBP7 such as siRNA, antisense nucleic acid, shRNA, miRNA, gRNA, ribozyme, and nucleic acid aptamer.

The small interfering RNA (siRNA) is an artificially synthesized small double-stranded RNA used for gene silencing by RNA interference (degradation of mRNA), and is used in the sense of including an siRNA expression vector capable of supplying the double-stranded RNA in vivo. The siRNA introduced into the cell binds to an RNA-induced silencing complex (rise). This complex binds to and cleaves mRNA having a sequence complementary to the siRNA, whereby the expression of the gene can be suppressed in a sequence-specific manner. The siRNA can be prepared by synthesizing sense strand and antisense strand oligonucleotides with a DNA/RNA automatic synthesizer, respectively, for example, denaturing the sense strand and the antisense strand oligonucleotides at 90 to 95°C for about 1 minute in an appropriate annealing buffer, and then annealing the sense strand and the antisense strand oligonucleotides at 30 to 70°C for about 1 to 8 hours. The length of the siRNA is preferably 19 to 27 base pairs, and more preferably 21 to 25 base pairs or 21 to 23 base pairs.

The siRNA for IGFBP7 can be designed based on its base sequence so as to cause degradation (RNA interference) of mRNA transcribed from the IGFBP7 gene. Examples of the siRNA that inhibits the expression of IGFBP7 include siRNA having the above-described mRNA sequence of IGFBP7 as a target sequence.

The antisense nucleic acid is a nucleic acid complementary to the target sequence. The antisense nucleic acid can suppress the expression of the target gene by, for example, inhibition of transcription initiation by triplex formation, inhibition of transcription by hybridizing with a site where an open loop structure is formed locally by RNA polymerase, inhibition of transcription by hybridizing with RNA in the process of synthesis, inhibition of splicing by hybridizing at the junction of an intron and an exon, inhibition of splicing by hybridizing with a spliceosome formation site, inhibition of nucleus-to-cytoplasmic migration by hybridizing with mRNA, inhibition of splicing by hybridizing with a capping site or poly(A) addition site, inhibition of translation initiation by hybridizing with a translation initiation factor binding site, inhibition of translation by hybridizing with a ribosome binding site near the initiation codon, prevention of peptide chain elongation by hybridizing with the translation region or polysome binding site of mRNA, inhibition of gene expression by hybridizing with a site where a nucleic acid interacts with a protein, or the like.

The antisense nucleic acid for IGFBP7 refers to, for example, a single-stranded nucleic acid complementary to some base sequences selected from the above-described gene sequence of IGFBP7, the above-described base sequence encoding the amino acid sequence of IGFBP7, and the above-described mRNA sequence of IGFBP7. The nucleic acid may be a naturally-derived nucleic acid or an artificial nucleic acid, or may be based on either DNA or RNA. The length of the antisense nucleic acid is usually about 15 bases to the same length as the entire length of the mRNA, and is preferably about 15 to about 30 bases. The complementarity of the antisense nucleic acid does not necessarily need to be 100%, and may be such that the antisense nucleic acid can complementarily bind to DNA or RNA encoding IGFBP7 in vivo.

The shRNA for IGFBP7 can be designed based on its base sequence so as to cause degradation (RNA interference) of mRNA transcribed from the IGFBP7 gene. Examples of the shRNA that inhibits the expression of IGFBP7 include shRNA having the above-described mRNA sequence of IGFBP7 as a target sequence.

A miRNA(microRNA) is a functional nucleic acid that is encoded on a genome and finally becomes a microRNA of about 20 bases through a multistage generation process. miRNAs are classified as functional non-coding RNAs (ncRNAs: generic term for RNA that is not translated into protein) and play an important role in life phenomena, regulating the expression of other genes. In the present invention, the expression of the IGFBP7 gene can be suppressed by introducing an miRNA having a specific base sequence into a cell by a vector and administering the miRNA to a living body.

The guide RNA (gRNA) is an RNA molecule used for genome editing technology. In the genome editing technology, gRNA specifically recognizes a target sequence, leads binding of Cas9 protein to the target sequence, and enables gene knockout and knock-in. In the present invention, by administering gRNA targeting the IGFBP7 gene into a living body, the expression of the IGFBP7 gene can be suppressed in the living body. The gRNA is used in a sense including single guide RNA (sgRNA). Methods for designing gRNA in genome editing technology are widely known, and can be appropriately designed by referring to, for example, Benchmarking CRISPR on-target sgRNA design, Yan et al., Brief Bioinform, 15 Feb 2017.

Ribozymes are RNAs having catalytic activity. Although there are ribozymes having various activities, research on ribozymes as enzymes for cleaving RNA has made it possible to design ribozymes for site-specific cleavage of RNA. The ribozyme may have a size of 400 nucleotides or more such as M1RNA contained in group I intron type or RNaseP, or may have about 40 nucleotides called hammerhead type or hairpin type.

Aptamers include nucleic acid aptamers and peptide aptamers. The nucleic acid aptamer and the peptide aptamer used in the present invention can be obtained using an in vitro molecular evolution method in which a complex of a library molecule and a target molecule is formed in a test tube and then selected on the basis of affinity, represented by a Systematic Evolution of Ligands by Exponential enrichment (SELEX method), an mRNA display method, or the like.

The antisense nucleic acid, siRNA, shRNA, miRNA, ribozyme, and nucleic acid aptamer may contain various chemical modifications in order to improve stability and activity. For example, in order to prevent degradation by a hydrolase such as a nuclease, the phosphate residue may be substituted with a chemically modified phosphate residue such as phosphorothioate (PS), methyl phosphonate, or phosphorodithionate. In addition, at least a part thereof may be constituted by a nucleic acid analog such as a peptide nucleic acid (PNA).

The antibody against IGFBP7 refers to an antibody that specifically binds to IGFBP7 and inhibits the function of IGFBP7 by binding. In the present invention, any of a monoclonal antibody, a polyclonal antibody, a chimeric antibody, a humanized antibody, a human antibody, a mouse antibody, a rat antibody, a camel antibody, an antibody fragment (for example, Fab, Fv, Fab', F(ab')₂, scFv), and the like may be used as the antibody, and these can be prepared according to a known technique by those skilled in the art.

The antibody against IGFBP7 can be produced by a known method of producing an antibody or antiserum using the IGFBP7 protein or a part thereof as an antigen. The IGFBP7 protein or a part thereof can be prepared by known protein expression methods and purification methods. Examples of the IGFBP7 protein include, but are not limited to, human IGFBP7 defined by the sequence information of IGFBP7 described above. IGFBP7 proteins from various organisms may be used as immunogens. Antibodies to IGFBP7 that can be used in the present invention can also be produced via phage display (see, e.g., FEBS Letter, 441:20-24 (1998)). Apart (that is, a fragment) of the IGFBP7 protein includes a peptide having the amino acid sequence of SEQ ID NO: 13 and a peptide having the amino acid sequence of SEQ ID NO: 14.

In the present invention, IGFBP7 or an epitope having immunogenicity thereof may be administered to a subject to induce antibody production against IGFBP7 in a living body of the subject. The pharmaceutical composition 2 of the invention also comprises forms of such vaccine compositions. That is, the vaccine composition of the present invention contains IGFBP7 or an epitope having immunogenicity thereof. An epitope that induces a neutralizing antibody can be selected. Vaccines include mRNA vaccines, genetically modified protein vaccines and peptide vaccines. Examples of the epitope capable of inducing a neutralizing antibody against IGFBP7 include a peptide having the amino acid sequence of SEQ ID NO: 13 and a peptide having the amino acid sequence of SEQ ID NO: 14.

IGFBP7 is secreted from the failing cardiomyocytes, and excessive IGFBP7 in the cardiomyocytes causes cardiac dysfunction, myocardial infarction, and heart failure. It is considered that the active ingredients (f), (g), (h), and (i) of the pharmaceutical composition 2 of the present invention inhibit IGFBP7 gene expression or the action of IGFBP7 protein, and treat or prevent cardiac dysfunction, myocardial infarction and heart failure caused by IGFBP7.

The administration route, dosage form, formulation, and dose of the pharmaceutical composition 2 of the present invention can be carried out according to the description of the pharmaceutical composition 1 of the present invention. In a case where the pharmaceutical composition 2 of the present invention is provided as a gene therapeutic agent, it can be a composition containing the nucleic acid of the active ingredients (f) and (h) or a fragment thereof (for example, naked DNA) together with a delivery reagent. In a case where the pharmaceutical composition 2 of the present invention is provided as a gene therapeutic agent, it may also be a composition containing an expression vector of the active ingredient (i), optionally together with a delivery reagent. Formulation of delivery reagents and expression vectors and gene therapy agents can be performed according to the description for pharmaceutical composition 1 of the present invention.

According to the present invention, there is provided a non-human mammalian heart failure model animal in which the expression of insulin-like growth factor binding protein 7 (IGFBP7) in cardiac fibroblasts is enhanced (sometimes referred to herein as model animal 2 of the present invention). Animal species of the model animal 2 of the present invention include non-human mammals such as rodents (for example, mice and rats), cows, horses, pigs, monkeys, and dogs.

The enhancement of the expression of the insulin-like growth factor binding protein 7 (IGFBP7) in the model animal 2 of the present invention can be carried out by a method known to those skilled in the art, and for example, an animal produced by overexpressing the IGFBP7 gene by genetic engineering can be used, and examples of such a model animal include overexpressing mice. Examples of the method of obtaining the model animal 2 include, but are not limited to, a method of injecting an expression vector in which a target gene is connected to a promoter active in a cell type to be overexpressed into a fertilized egg, gene knock-in to a ROSA26 site, and knock-in of a target gene under a promoter of an endogenous gene.

According to the present invention, there is provided a method for screening for an agent or method of the treatment or prevention of myocardial infarction or heart failure including the steps (F) and (G) (which may be referred to as Screening Method 3 of the present invention herein). According to the present invention, there is also provided a method for screening for an agent or method of the treatment or prevention of myocardial infarction or heart failure including the steps (H) and (I) (which may be referred to as Screening Method 4 of the present invention herein).

Examples of the test factor in the Screening Methods 3 and 4 of the present invention include, but are not limited to, a low molecular weight compound, a middle molecular weight compound, a peptide, a protein, an antibody, and the like. In Screening Method 4, in order to treat a non-human mammal in which the expression of IGFBP7 is enhanced with the test factor, the test factor may be formulated into an optimal dosage form according to the administration route.

As the "cells in which the expression of IGFBP7 is enhanced" used in the step (F) of Screening Method 3 of the present invention, cardiomyocytes or cardiac fibroblasts of a human or a non-human mammal in which the expression of IGFBP7 is enhanced can be used, and the cells can be prepared according to the method of the overexpression model animal described above. In addition, after the comparison procedure of the step (G) of Screening Method 3 of the present invention, when insulin signaling is increased or expression of mitochondrial electron transport pathway and/or complex IV-related gene is increased in the cell as compared with an untreated control cell or a wild-type cell, the test factor can be determined to be an agent candidate useful for treatment or prevention of myocardial infarction or heart failure.

"Treating with the test factor" in the step (H) of Screening Method 4 of the present invention means, for example, administering the test factor to a non-human mammal (model animal 2 of the present invention). The administration route can be appropriately selected according to the type of the test factor and the like. After the comparison procedure of step (I) of Screening Method 4 of the present invention, in a case where the expression of Nppa, which is a marker gene for heart failure, is reduced, the test factor can be determined to be an agent candidate useful for the treatment or prevention of myocardial infarction or heart failure.

According to the present invention, there is provided a method of diagnosing myocardial infarction or heart failure (which may be referred to as Diagnosis Method 2 of the present invention in the present specification) including the step (J). In the step (J), in a case where the expression level of IGFBP7 in cardiac fibroblasts, cardiomyocytes, or blood of the subject is significantly increased as compared with the expression level of IGFBP7 in cardiac fibroblasts, cardiomyocytes, or blood of a healthy subject, it can be determined that the subject can suffer from myocardial infarction or heart failure, that is, there is a risk of suffering from myocardial infarction or heart failure. The expression level of IGFBP7 in cardiac fibroblasts, cardiomyocytes, or blood can be measured for a biopsy sample or blood sample of a subject using a nucleic acid amplification method such as quantitative PCR. Diagnosis Method 2 of the present invention can be performed in vitro. In addition, in Diagnosis Method 2 of the present invention, a method including a determination step by a doctor can be excluded.

Since IGFBP7 is used as a marker for diagnosing myocardial infarction or heart failure in Diagnosis Method 2 of the present invention, according to another aspect of the present invention, the use of IGFBP7 or a nucleic acid encoding the IGFBP7 or a fragment thereof is provided as a marker for diagnosing myocardial infarction or heart failure.

According to the present invention, there is also provided a method for determining the severity level of myocardial infarction or heart failure (which may be referred to as the method of determining of the present invention in the present specification), including the step (K). In the step (K), in a case where the expression level of IGFBP7 in cardiac fibroblasts, cardiomyocytes, or blood of the subject is significantly increased as compared with the expression level of IGFBP7 in cardiac fibroblasts, cardiomyocytes, or blood of a healthy subject, it can be determined that the severity level of myocardial infarction or heart failure in the subject is high, that is, there is a risk of exacerbation of myocardial infarction or heart failure. The method of determining of the present invention can be performed over time. For example, in a case where the expression level of IGFBP7 in cardiac fibroblasts, cardiomyocytes, or blood of a subject increases over time, it can be determined that the severity level of myocardial infarction or heart failure has deteriorated (or the risk of deterioration has increased). Conversely, when the expression level of IGFBP7 in cardiac fibroblasts, cardiomyocytes, or blood of a subject decreases over time, it can be determined that the severity level of myocardial infarction or heart failure has alleviated (or the risk of deterioration has decreased). The expression level of IGFBP7 in cardiac fibroblasts, cardiomyocytes, or blood can be measured in the same manner as in Screening Method 3 of the present invention. The method of determining of the present invention can be performed in vitro. In addition, in the method of determining of the present invention, a method including a determination step by a doctor can be excluded.

Since IGFBP7 is used as a marker for determining the severity level of myocardial infarction or heart failure in the method of determining of the present invention, according to another aspect of the present invention, use of IGFBP7 or a nucleic acid encoding the IGFBP7 or a fragment thereof as a marker for determining the severity level of myocardial infarction or heart failure is provided.

### EXAMPLES

The present invention will be described more specifically based on the following examples, but the present invention is not limited to these examples.

### Animal Model

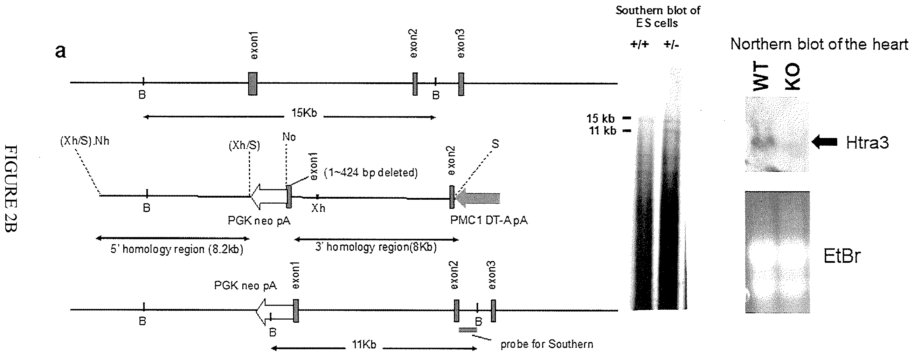

C57BL/6 N mice were purchased from CLEA Japan, Inc. Htra3 knockout mice were generated by a conventional homologous recombination method using RF8 ES cells, similar to the previous generation of Htra1 knockout mice (Document 55). DT-Ap/Neo cassettes were provided by RIKEN Biological Resource Research Center, Japan. The targeting vector was designed to delete 424 base pairs from the 5' end of exon 1 of the Htra3 gene. (Fig. 2Ba) To verify successful gene targeting in ES cells, 10 µg of genomic DNA extracted from ES cells was digested with BamHI and hybridized with an 800-bp DIG-labeled probe derived from a region near intron 2 of the Htra1 gene (Fig. 2Ba). The absence of Htra3 transcripts in homozygous Htra3 knockout mice was confirmed by northern blot analysis. Total RNA prepared from heart (20 µg) was hybridized with 1.4kbp DIG-labeled full-length cDNA of Htra3 (Fig. 2Ba). Htra3 knockout mice were maintained as a C57BL/6 N background after 10 or more generations of backcross. Only male mice were used in all experiments. Female and male mice were housed in separate cages with ad libitum access to food and water on a 12 hour light/dark cycle with up to 6 mice per cage in temperature-controlled bibarium without specific pathogens. The ambient room temperature was regulated to 73°±5°F and the humidity was controlled to 50°±10%. All experiments were approved by the University of Tokyo Ethics Committee for Animal Experiments and adhered to the University of Tokyo guidelines for animal experiments. Htra3 knockout mice are available in material transfer agreements.

### Systolic Blood Pressure Measurement

Systolic blood pressure was measured in conscious mice (wild-type n=12 vs. Htra3 knockout n=12) by a tail cuff system using MK-2000ST (Muromachi Kikai Co., Ltd., Tokyo, Japan) according to the manufacturer's protocol.

### TAC Model Surgery and Echocardiography

TAC surgery was performed on male mice randomly selected from male mice weighing 20 to 23 g at 9 to 11 weeks of age as previously described (Document 56). Mice were anesthetized with 2% isoflurane by inhalation, then approached the aorta with minimal sternotomy and a 7-0 ligature was placed around the vessel using a 26 gauge needle to ensure consistent occlusion. Sham-operated animals that underwent a similar surgical procedure without aortic constriction were used as controls. Surgeons were not informed about the genotypes of the mice. Mice that died within one week after surgery were excluded from analysis. Trans-thoracic echocardiography was performed in conscious mice using the Vevo 2100 imaging system (Visualsonics, Inc.). To minimize data variability, cardiac function was assessed only when the heart rate was 600 to 700 beats per minute. M-mode echocardiography images were acquired from a longitudinal view measuring the size and function of the left ventricle.

### Myocardial Infarction Model

Myocardial infarction (MI) was induced as previously described (Document 57). Mice were anesthetized by inhalation of 2% isoflurane. MI was induced by ligation of the left anterior descending artery. Mice that did not develop MI or died within one week after surgery were excluded from the analysis.

### Single Cell Analysis of Mouse Samples

For non-cardiomyocyte separation and collection, hearts were minced and enzymatically dissociated using 2 mg/mL type 2 collagenase (Worthington), 1 mg/mL dispase (Roche), and 20 U/mL DNase I (Roche) in a digestion cycle of 40 min total at 37°C. After cardiomyocytes were removed using a 40 µm cell strainer (Greiner), cells were stained with Zombie Green Fixable Viability Kit (BioLegend) and viable cells were collected by fluorescence-activated cell sorting (FACS) using a FACSJazz cell sorter (BD Biosciences). To separate cardiac fibroblasts, cells were stained with APC-conjugated anti-mouse CD140a (PDGFR-α) antibody (#135907, BioLegend, 1:200), FITC- conjugated anti-mouse CD31 antibody (#102506, BioLegend, 1:200), and Zombie Green, after which PDGFR-α positive live cells were collected by FACS.

Cardiomyocytes were isolated using Langendorff perfusion from the left ventricular free wall. Enzyme dissociation using Langendorff perfusion was performed using 35 mL of enzyme solution (Type 2 collagenase 1 mg/mL [Worthington], protease type XIV 0.05 mg/mL [Sigma-Aldrich], NaCl 130 mM, KCl 5.4 mM, MgCl₂ 0.5 mM, NaH₂PO₄ 0.33 mM, D-Glucose 22 mM, HEPES 25 mM; pH7.4) was preheated to 37°C and run at a rate of 3 mL/min. To neutralize enzyme activity, fetal bovine serum (FBS) was added to the solution to a final concentration of 0.2% (v/v). The cell suspension was filtered through a 100 µm nylon mesh cell strainer and centrifuged at 20 g for 3 minutes. The supernatant contained non-cardiomyocytes and could be recovered by centrifugation at 300 g×5 minutes. To prevent hypercontraction, the cardiomyocytes were resuspended in medium (NaCl 130 mM, KCl 5.4mM, MgCl₂ 0.5mM, NaH₂PO₄ 0.33mM, D-glucose 22mM, HEPES 25 mM, FBS 0.2%; pH 7.4) containing a low concentration of calcium (0.1 mM). Rod-shaped live cardiomyocytes (cardiomyocyte viability was ≥ 80% at all time points) were collected with a 0.2 to 2 µL pipette (sample volume, 0.5 µL) immediately after separation from 2 mice and incubated in lysis buffer.

Single cell cDNA libraries were generated using the Smart-seq2 protocol or Chromium3'v2 chemistry kit (10x Genomics) according to the manufacturer's instructions. For the cDNA library constructed by Smart-seq2, the distribution of cDNA fragment lengths was evaluated using LabChip GX (Perkin Elmer) and/or TapeStation 2200 (Agilent Technologies) using quantitative real-time polymerase chain reaction (qRT-PCR) CFX96 Real-Time PCR Detection System (Bio-Rad). The following primer set was used for qRT-PCR: Tnnt2 mRNA forward: TCCTGGCAGA GAGGAGGAAG (SEQ ID NO: 1); Tnnt2 mRNA reverse: TGCAGGTCGA ACTTCTCAGC (SEQ ID NO: 2); Actb mRNA forward: CAACTGGGACGACATGGAGA (SEQ ID NO: 3); and Actb mRNA reverse: GCATACAGGGACAGCACAGC (SEQ ID NO: 4). A Ct value of 25 was set as the threshold. Sequence libraries were subjected to paired-end 51 bp RNA sequences in HiSeq 2500 (Illumina) in rapid mode. The insert size was 345±40 bp (mean ± standard deviation). RefSeq transcripts (coded and non-coded) were downloaded from the UCSC Genome Browser (http://genome.ucsc.edu). Reads were mapped to the mouse genome (mm9) using the parameters "-g1-p8mm9-no-coverage-search" using TopHat and Cufflinks (Documents 58 and 59). FPKM was calculated using reads mapped to the nuclear genome (Document 60). Single cell analysis by full-length cDNA library synthesis by Smart-seq2 calculated the detected genes for each cell and generated histograms to set the cutoffs for the genes to be analyzed.

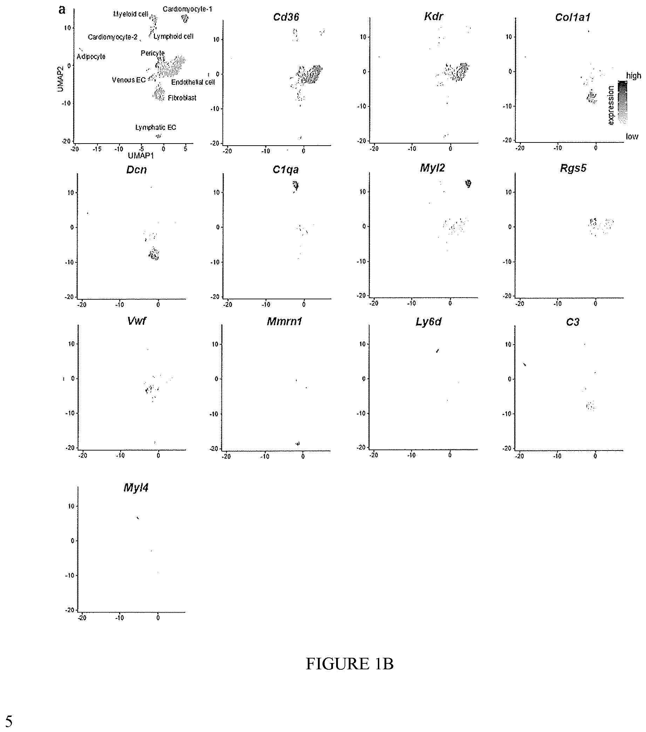

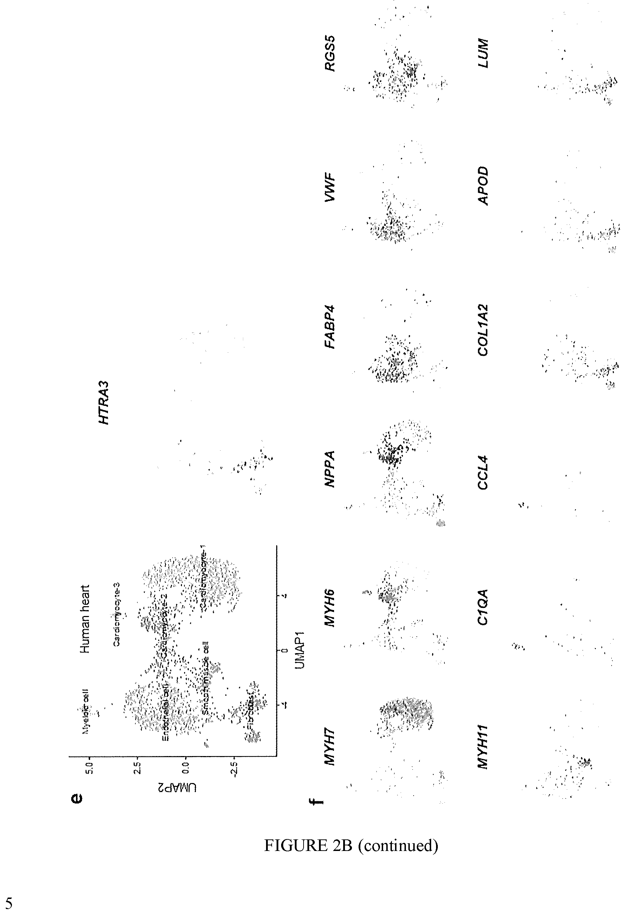

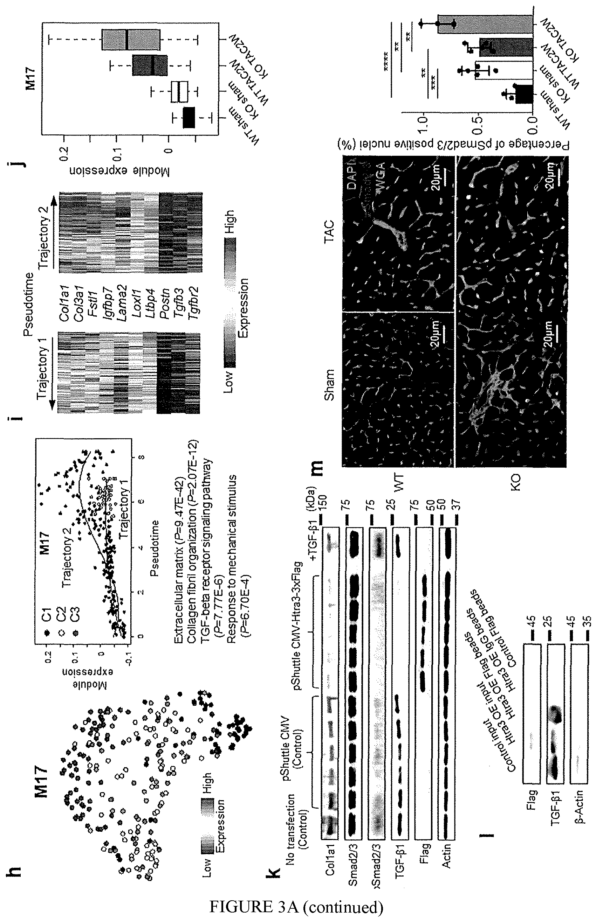

For weighted co-expression network analysis, a signed network was constructed using the WGCNA R package, which was also used for cell type annotation, using all genes expressed at FPKM values greater than or equal to 10 in at least one sample. The soft power threshold was analyzed with the "pickSoftThreshold" function and applied to construct a signed network and calculate the module-specific gene expression using the "blockwiseModules" function. Modules with less than 30 genes were merged with the closest large neighboring module. Cytoscape (version 3.7.2) with "edge-weighted force-directed" (Document 61) was used to visualize the weighted co-expression network. The correlation coefficients between each gene expression and fibroblast module expression were calculated in Fig. 3Ah and Supplemental Table 2. UMAP (Document 62) was used for dimension reduction. Graph-based clustering was performed using the "buildSNNGraph" function in scran (Document 63). The "randomForest" package in R was used to evaluate the accuracy of the classification. The cell type of each cluster was determined by characteristic marker genes such as Myl2, Myl4, Myh6, or Myh7 for cardiomyocytes, Kdr, Fabp4, or Vwf for endothelial cells, Col1a1, Dcn, or Lum for fibroblasts, and their expression profiles were illustrated (Figs. 1Ba and 2Bf), and in the LR communication analysis, LR interaction pairs were extracted using a published database (Document 22). Matrix data for cardiac LR communication was described in Supplemental Table 1. In the pseudotime analysis, trajectories were detected using Slingshot (Document 64). Pearson's correlation coefficient was calculated using the "cor" function in R. Single- cardiomyocyte RNA-seq data for p53 knockout mice was downloaded from the Gene Expression Omnibus with an accession code GSE95143. Hierarchical clustering was performed using Cluster 3.0 (Document 65) and JAVA Treeview (Document 66).

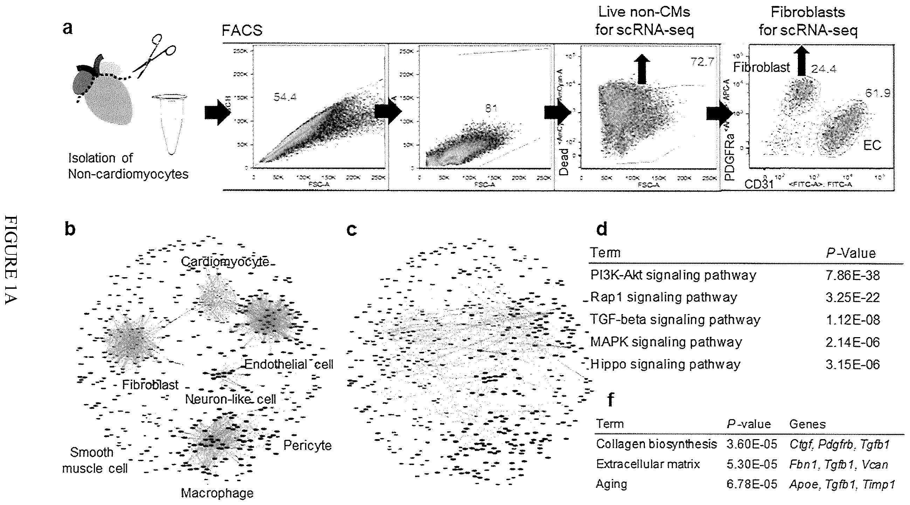

For the droplet based single cell analysis data (Fig. 1Aa) using the Chromium Controller (10x Genomics), the dataset was aligned and quantified using the CellRanger software package and default parameters. Cells with more than 300 expressed genes were retained, and 1783 cells were obtained from 2 mice for subsequent analysis using Seurat v3 (Document 67) (sham surgery). After SCTransform normalization was implemented individually for each dataset, the top 500 characteristic genes were selected and used for data integration. Data integrated with the function of "FindIntegrationAnchors" was used for dimension reduction and cluster detection. Marker genes specific for each cluster were used for cell type annotation.

### Gene Ontology (GO) Analysis

Database for annotation, visualization and integrated discovery (DAVID) (Document 68) was used for GO analysis and Kyoto Encyclopedia of Genes and Genomes (KEGG) path enrichment analysis. Enrichment P values for all extracted GO terms for each module were calculated with DAVID.

### Spatial Transcriptome Analysis of Mouse MI Model

Frozen samples were embedded in OCT (TissueTek Sakura) and cryosections were generated at -14°C (Leica CM1860). Sections were placed on chilled Visium Spatial Gene Expression Slides (2000233, 10X Genomics), and adhered by warming the back of the slide. Tissue sections were then fixed in chilled methanol and stained according to the Visium Spatial Gene Expression User Guide (CG000239 Rev D, 10X Genomics). For gene expression samples, tissues were permeabilized for a period of 15 minutes, which was selected as the optimal time based on tissue optimization time course experiments (CG000238 Rev D, 10X Genomics). Brightfield histology images were taken on a BZ-X700 microscope (Keyence Corporation, Itasca, Illinois) using a 10X objective. Raw images were stitched together using the BZ-X Analyzer software (Keyence Corporation) and exported as a TIFF file.

The libraries were prepared according to the Visium Spatial Gene Expression User Guide (CG000239 Rev D, 10X Genomics) and sequenced on a NovaSeq 6000 System (Illumina) using the NovaSeq S4 Reagent Kit (200 cycles, 20027466, Illumina) , at a sequencing depth of approximately 250-400 M read-pairs per sample. The sequence was performed using the following read protocol: Read 1, 28 cycles; i7 index read, 10 cycles; i5 index read, 10 cycles; Read 2, 91 cycles. Raw FASTQ files and histology images were processed on a Cell Ranger mm10 reference genome "refdata-gex-mm10-2020-A" sample by sample using Space Ranger software (ver1.1.0, 10X Genomics) and are available at https:// cf. 10xgenomics.com/supp/spatial-exp/refdata-gex-mm10-2020-A.tar.gz. Seurat v4 was used for downstream analysis. SCTransform normalization was implemented individually for each dataset and merged them. The merged data were used for dimension reduction and cluster detection. Differentially expressed genes were detected using FindMarkers in Seurat (log2fc.threshold>0.25, p_val_adj<0.05). To predict the proportion of cell type in each spot, predict, score was calculated using Seurat's "FindTransferAnchors" and "TransferData" functions. Single cell analysis of cells isolated from mice after sham or MI surgery was used as reference data.

### Single Cell Analysis of Human Samples

The procedure for single cell analysis of cardiomyocytes isolated from heart failure patients and control subjects was approved by the University of Tokyo Ethics Committee (approval number G-10032). All procedures were performed in accordance with the Helsinki Declaration and written informed consent was obtained from all participants. Heart tissue was obtained within 1 hour post-mortem from 2 patients with non-cardiac causes of normal cardiac function at necropsy or from 22 patients who developed severe heart failure during left ventricular assist device surgery or heart transplantation. The population including control subjects and patients with heart failure included 17 males and 7 females, and the mean age at separation of cardiomyocytes in the single cell analysis was 46.4±12.2 years.

Immediately after the collection of the heart tissue, the tissue was minced and incubated in lysis buffer containing 2 mg/mL type 2 collagenase (Worthington), 1 mg/mL dispase (Roche), and 20 U/mL DNase I (Roche). After 4 cycles of lytic digestion with gentle shaking for a total of 20 min at 37°C, rod-shaped live cardiomyocytes were isolated. Single cell cDNA libraries were generated using the Smart-seq2 protocol. The efficiency of reverse transcription was assessed by examining the Ct value of a control gene (TNNT2) from qRT-PCR using CFX96 Real-Time PCR Detection System (Bio-Rad). The distribution of cDNA fragment length was evaluated using LabChip GX (Perkin Elmer) and/or TapeStation 2200 (Agilent Technologies). The following primer set was used for qRT-PCR: TNNT2 mRNA forward, AAGTGGGAAG AGGCAGACTGA (SEQ ID NO: 5); TNNT2 mRNA reverse, GTCAATGGCC AGCACCTTC (SEQ ID NO: 6). A Ct value of 25 was set as the threshold. The remaining libraries were sequenced using the HiSeq 2500 System (Illumina). Reads were mapped to the human genome (hg19) with the parameter "-S - m 1 -1 36 -n 2 hg19" using Bowtie (Document 69) as previously described. RPKM values were calculated with reads mapped to the nuclear genome using DEGseq70. A single-cell transcriptome consisting of 2,000 or more detected genes was used for subsequent analysis.

Seurat v3 (Document 67) was used for single cell analysis of human hearts using a published dataset (GSE121893) (Document 4). Cells with more than 500 expressed genes were retained, and 2,459 cells were obtained from 8 patients (6 patients with heart failure, 2 healthy control subjects) for subsequent analysis. After SCTransform normalization was implemented individually for each dataset, the top 2,000 characteristic genes were selected and used for data integration. The integrated data was used for dimensionality reduction and cluster detection. Marker genes specific for each cluster were used for cell type annotation.

### Anti-TGF-β Treatment

In anti-TGF-β treatment experiments, Htra3 knockout mice were intraperitoneally injected daily with 1.5 mg/kg body weight of isotype IgG1 control antibody (#MAB002, R&D Systems) or anti-TGFβ1 antibody (#MAB240, R&D Systems) as previously described (Document 71). Anti-TGF-β treatment was initiated 24 hours prior to TAC surgery.

### Cell Culture and Transfection of Plasmids

Cardiac fibroblasts were isolated from C57BL/6 N (Takasugi Experimental Animal Supply Co., Ltd) hearts at day 1 after birth. Isolated cardiac fibroblasts were cultured in Dulbecco's Modified Eagle's Medium supplemented with 10% FBS at 37°C, 5% CO₂. Plasmid cDNA encoding Htra3 tagged with FLAG at the C-terminus (pCMV-Htra3-flag) was prepared by VectorBuilder Inc., and transfected into these fibroblasts using Lipofectamine 3000 (Thermo Fisher Scientific) according to the manufacturer's instructions. Transfected cells were cultured in media containing 10% FBS serum for 48 hours prior to analysis. Each experiment was repeated at least three times.

### Western Blotting

Cardiac tissue, purified cardiomyocytes, purified non-cardiomyocytes, or cultured cardiac fibroblasts were used as samples for western blotting in this study. Samples were homogenized and lysed with RIPA buffer containing 10 mM Tris-HCl, 150mM NaCl, 5mM EDTA, 1% Triton X-100, 1% sodium deoxycholate, and 0.1% sodium dodecyl sulfate with protease and phosphatase inhibitor cocktails for 30 min on ice. The lysate was centrifuged at 15,000×g for 30 min and the supernatant was used as a whole cell extract. Bicinchoninic acid assay (Pierce BCA Protein Assay Kit; Thermo Scientific) was used to measure total protein concentration in the supernatant. For western blot analysis, extracted protein samples were separated on a 5 to 20% Mini-PROTEAN TGX precast gradient gel (Bio-Rad) and transferred to nitrocellulose membranes (BioRad). The membrane was blocked with 5% FBS in which 0.05% Tween was added to Tris buffered saline, incubated with the primary antibody overnight at 4°C, and then horseradish peroxidase-conjugated secondary antibody (Cell Signaling Technology) and ECL Plus (GE Healthcare) were used. Immunoreactive signals were detected using a LAS 4000 analyzer (GE Healthcare).

The following antibodies were used for western blotting: a rabbit monoclonal anti-pSmad2/3 (Ser456/467) antibody (#8828, Cell Signaling Technology, 1:1000), a rabbit monoclonal anti-Smad3 (phosphorylated S423+S425) antibody (ab52903, Abcam, 1: 1000), a rabbit monoclonal anti-Smad2/3 antibody (#8685, Cell Signaling Technology, 1: 1000), a rabbit monoclonal anti-Smad3 antibody (ab40854, Abcam, 1:1000), a rabbit monoclonal anti-Collagen1 antibody (ab138492, Abcam, 1:2000), a rabbit monoclonal anti-TGF beta1 antibody (ab179695, Abcam, 1: 1000), a rabbit polyclonal anti-TGF beta1 antibody (ab92486, Abcam, 1:1000), a horseradish peroxidase (HRP)-linked rabbit polyclonal anti-DDDDK tag antibody (PM020-7, MBL, 1: 1000), a mouse monoclonal ANTI-FLAG M2 antibody (Sigma-Aldrich, #F1804, 1:1000), a HRP-linked horse anti-rabbit IgG antibody (#7074, Cell Signaling Technology, 1:3000), a HRP-linked horse anti-mouse IgG antibody (#7076, Cell Signaling Technology, 1:3000), an anti-TGFβ receptor I antibody (ab235178, abcam, 1: 1000), an anti-TGFβ receptor II antibody (SAB4502960, Sigma-Aldrich, 1:500), an anti-TGF beta3 antibody (ab53727, Abcam, 1:1000), an anti-phospho-histone H2A.X (Ser139) monoclonal antibody (#MA1-2022, Invitrogen, 1:1000), an anti-NADPH oxidase 4 antibody (ab109225, Abcam, 1:2000), an anti-HtrA3 polyclonal antibody (NB600-1151, Novus Biologicals, 1:1000), an anti-cardiac troponin I polyclonal antibody (ab47003, Abcam, 1:2000), and an anti-actin monoclonal antibody (#MA5-11869, Invitrogen, 1:5000).

### Protein Purification of Htra3-flag

After pCMV-Htra3-flag was transfected into primary cultures of mouse cardiac fibroblasts, the Htra3-flag protein was immunoprecipitated from the lysate of the cells at 4°C using anti-DDDDK-tagged magnetic beads (MBL M185-11). Mouse IgG2a magnetic beads (MBL M076-11) were used for isotype control. Immunoprecipitates from fibroblasts without transfection (using the same beads) were also used as negative controls. After immunoprecipitation, each sample was washed and eluted according to the manufacturer's instructions (MBL).

### RNA in situ Hybridization

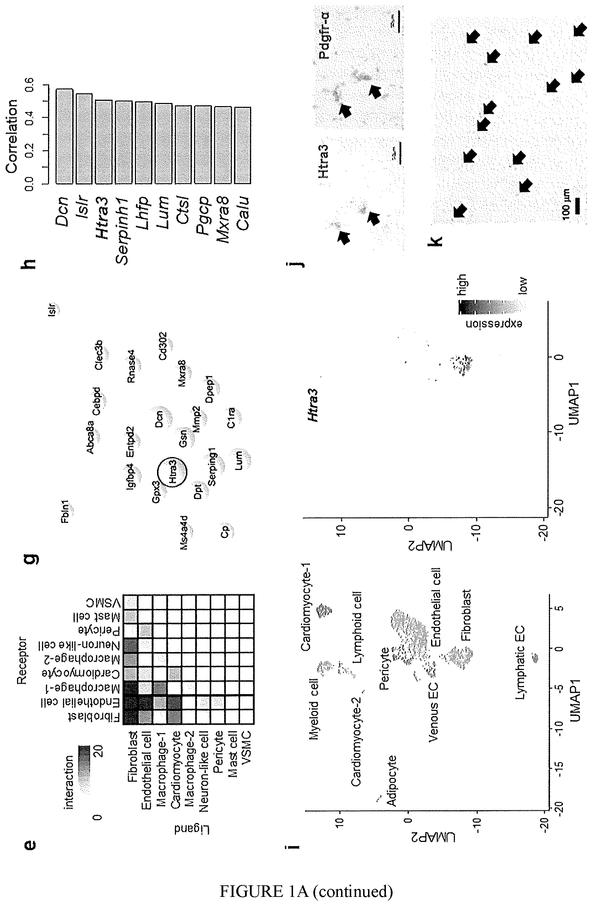

Both mouse and human tissues were fixed with G-Fix (Genostaff), embedded in paraffin on CT-Pro20 (Genostaff) and sectioned at 5 µm using G-Nox (Genostaff) as a less toxic organic solvent than xylene. RNA in situ hybridization was performed using the ISH Reagent Kit (Genostaff) according to the manufacturer's instructions. Tissue sections were deparaffinized with G-Nox and rehydrated in a set of ethanol and phosphate buffered saline (PBS). The sections were fixed with 10% neutral buffered formalin (10% formalin in PBS) for 30 min at 37°C, washed in distilled water, placed in 0.2 N HCl for 10 min at 37°C, washed with PBS, treated with 4 µg/mL proteinase K (Wako Pure Chemical Industries, Ltd.) in PBS for 10 min at 37°C, washed with PBS, and placed in Coplin jar containing 1xG-Wash (Genostaff), equal to 1x saline-sodium citrate. Hybridization was performed using probe (250 ng/mL) in G-Hybo-L (Genostaff) for 16 hours at 60°C. After hybridization, sections were washed in 1×G-Wash for 10 min at 60°C and in 50% formamide in 1×G-Wash for 10 min at 60°C. Sections were then washed twice with 1×G-Wash for 10 min at 60°C, twice with 0.1×G-Wash for 10 min at 60°C, twice with TBST (0.1% Tween20 in Tris buffered saline) at room temperature. After treatment with 1×G-Block (Genostaff) for 15 min at room temperature, sections were incubated with anti-DIG AP conjugate (Roche Diagnostics) diluted 1:2000 in G-Block (Genostaff; 1/50 dilation) in TBST for 1 h at room temperature. Sections were washed twice with TBST and incubated with 100 mM NaCl, 50 mM MgCl₂, 0.1% Tween20, and 100 mM Tris-HCl (pH9.5). The chromogenic reaction was performed overnight in NBT/BCIP solution (Sigma-Aldrich) and washed with PBS. Sections were counterstained with Kernechtrotstain (Muto Pure Chemicals) and mounted with G-Mount (Genostaff). Paired mirror heart sections were used to perform immunostaining of Pdgfra protein and in situ hybridization of Htra3 mRNAin Fig. 1Aj.

### Singl-molecule RNA in situ Hybridization

For single-molecule RNA fluorescence in situ hybridization (Fig. 7Bf), the RNAscope system72 (Advanced Cell Diagnostics) was used with probes against human LUM mRNA (NM_002345.3, #494761). Frozen sections (10 µm) were fixed in PBS containing 4% paraformaldehyde for 5 minutes at room temperature, dehydrated by continuous immersion in 50%, 70%, and 100% ethanol for 5 minutes at room temperature, and treated with protease for 30 minutes at room temperature. The probes were then hybridized for 2 h at 40°C, followed by RNAscope amplification and costaining with Alexa Fluor 488-conjugated wheat germ agglutinin (W11261; Thermo Fisher Scientific; 1:200) and DAPI to detect cell membranes and nuclei. Images were acquired as a Z-stack using an In Cell Analyzer 6000 (GE Healthcare).

### Histology

For histological analysis, mice were anesthetized by isoflurane inhalation and sacrificed by cervical dislocation. The chest was opened and a 25 gauge needle was inserted into the apex and the heart was flushed with cold PBS. The right atrium was cut to allow evacuation of blood from the heart and mice were briefly perfused through the apex of the heart with cold fixative (4% paraformaldehyde in PBS). Tissues and organs were excised, flushed with fixative, incubated for 12 h at 4°C with gentle rotation with fixative, and finally embedded in paraffin.

Paraffin-embedded cardiac tissue was cut into 4 µm slices using SM2010 R Sliding Microtome (Leica Biosystems). For fluorescent immunostaining, paraffin-embedded sections were treated with antigen retrieval solution (Dako) and blocked with 5% normal goat serum followed by overnight incubation with primary antibodies. After washing with PBS, samples were stained with the appropriate secondary antibody (Anti-rabbit IgG-Alexa594, 1:400; Anti-mouse IgG-Alexa647, 1:400) for 1 hour. The membranes and nuclei of the cells were counterstained with wheat germ agglutinin-Alexa488 (Thermo Fisher Scientific; 1:200) and DAPI (4',6 diamidino-2 phenylindole; Dojindo, 1:1000). Images were acquired using a LSM 880 META confocal microscope (Zeiss) or a BZ-X700 microscope (Keyence). The following primary antibodies were used for fluorescent immunostaining: rabbit monoclonal anti-pSmad2/3 (Ser456/467) antibody (#8828, Cell Signaling Technology, 1:200), rabbit monoclonal anti-p21 antibody (ab188224, Abcam, 1:200), mouse monoclonal anti-yH2A.X (Ser140) antibody (#MA1-2022, Thermo Fisher Scientific, 1:200), rabbit monoclonal anti-PDGFRa antibody (#3174, Cell Signaling Technology, 1:200).

To quantify the γH2A.X-positive nuclei of the heart, images of γH2A.X staining were acquired from both wild-type and Htra3 knockout mice after TAC surgery (n=3 each). Four images were taken from each heart. The raw imaging data was analyzed using the BZ-X analyzer software (Keyence Corp.) to quantify the fluorescence intensity of the γH2A.X signal merged with the DAPI of each nucleus. Thereafter, the threshold for detecting γH2A.X-positive nuclei was set, and the software automatically calculated the proportion of γH2A.X-positive nuclei. All raw image data pieces were analyzed using the same algorithm (Document 14).

To measure the amount of fibrosis, sections were stained with Picro-sirius red/Fast green dye. After captioning the entire image of the sections, the percentage of LV fibrotic area was assessed by areas of red and green stained area using the BZ Analyzer software (Keyence). All histological quantifications were performed in a blinded manner by two observers.

### AAV Infection

AAV vectors were prepared by VectorBuilder Inc. (https://en.vectorbuilder.com) following established procedures (Document 73). Briefly, AAV vectors of serotypes 2 and 9 were generated in HEK293T cells using triple plasmid co-transfection for packaging. Virus stocks were obtained by CsCl₂ gradient centrifugation. Titration of AAV viral particles was performed by real-time PCR quantification of the number of viral genomes, measured as CMV copy number. The viral preparation had a titer between 1×10¹² and 5×10¹² genomic copies (GC)/mL. The virus was administered in 100 µL of saline by tail vein injection. In experiments performed in HtrA3 knockout mice, 3×10¹¹ GC doses of rAAV9-GFP or 3×10¹¹ GC doses of rAAV9-shNox4 were administered to intact mice 1 week prior to TAC surgery. In experiments of Htra3 overexpression, wild type mice were administered 3×10¹¹ GC doses of rAAV9-GFP or rAAV9-Htra3 1 week after TAC surgery. In the Nox4 overexpression experiment, 3×10¹¹ GC doses of rAAV9-GFP or rAAV9-Nox4 were administered to wild-type mice at 8 weeks of age.

### qRT-PCR Analysis

In the mechanical stretch experiment, cardiac fibroblasts isolated from neonatal mice were plated on silicone rubber culture plates coated with 100 mm square gelatin (0.1%) and cultured in serum-free DMEM for 4 hours as previously described (Documents 74 and 75). After 4 hours, the medium was changed to 2% FBS/DMEM and cultured for another 24 hours. Mechanical stretch was introduced to the attached fibroblasts by 10-30% persistent elongation. One day after stretching, cells were lysed using Smart-seq2 lysis buffer and used for cDNA library construction using Smart-seq2. mRNA expression was evaluated by qRT-PCR using a CFX96 Real-time PCR detection system and relative expression levels of target genes were normalized to expression of internal control genes using the comparative Ct method. The following primer sets were used for qRT-PCR.

Rps18 mRNA forward, CTTAGAGGGACAAGTGGCG (SEQ ID NO: 7)
Rps18 mRNA reverse, ACGCTGAGCCAGTCAGTGTA (SEQ ID NO: 8)
Htra3 mRNA forward, TGACCAGTCCGCGGTACAAG (SEQ ID NO: 9)
Htra3 mRNA reverse, TTGGAGCTGGAGACCACGTG (SEQ ID NO: 10)
Tgfb1 mRNA forward, CTCCCGTGGCTTCTAGTGC (SEQ ID NO: 11)
Tgfb1 mRNA reverse, GCCTTAGTTTGGACAGGATCTG (SEQ ID NO: 12)

For qRT-PCR of Htra3, multiple organs such as heart, placenta, skeletal muscle, brain, liver, kidney, lung were collected from both Htra3 knockout mice and wild-type controls after whole body perfusion of cold PBS. Total RNA was isolated from these tissues using TRIzol reagent (#15596026, Thermo Fisher Scientific). After its purity was confirmed by 260/280nm absorbance (>1.8), single-stranded cDNA was synthesized from 1 µg of RNA according to the manufacturer's instructions using the High-Capacity cDNA Reverse Transcription Kit (#4374966, Thermo Fisher Scientific). mRNA expression was evaluated by qRT-PCR using a CFX96 real-time PCR detection system and relative expression levels of target genes were normalized to expression of internal control genes using the comparative Ct method.

### Plasma Proteome Analysis

Plasma proteomics data from various stages of heart failure (n=768 for control subjects, n=84 for non- advanced heart failure, n=30 for advanced heart failure prior to transplantation, and n=30 for advanced heart failure after transplantation) were obtained from Egerstedt et al. and used for proteomic analysis (Document 34). The "randomForest" package of R was used to evaluate the accuracy of the classification and identify factors to identify groups. Receiver operating characteristic analysis was performed to assess diagnostic and discriminative performance using the "pROC" package of R.

### Statistics and Reproducibility

All statistical tests and graphical depictions of the data (mean and error range) are defined within the figure legends of the respective data panel. For comparison between the two groups, unpaired or Student's two-tailed t-tests were performed as described in the figure legends. For comparison between three or more groups, one-way or two-way ANOVA was performed as described in the figure legends. P<0.05 was considered statistically significant unless otherwise stated. In box plots, horizontal lines indicate median values, boxes indicate 25 to 75 percentiles, and whiskers represent minimum and maximum values. Imaging experiments (Figs. 1Aj, k, 4Aj, and 5Ac and Figs. 3Bf, 4Bf, g, 5Bc, and 7Bf) were performed twice by independent researchers. Western blotting experiments (Figs. 3Ak, l, 5Ad, e, and g and Figs. 1Bk and 3Be) were performed once using different samples of protein. In vitro experiments such as imaging studies and western blotting were performed and checked by at least two independent researchers.

### Bulk RNA sequence

Total RNA was extracted from iPSCM using RNeasy Plus Mini Kit (Qiagen). Single-cell cDNA libraries were generated using the Smart-seq2 protocol and cDNA libraries were prepared using the Nextera XT Library Prep Kit (Illumina) according to the manufacturer's protocol. Sequencing libraries were then subjected to paired-end 150 bp RNA sequencing on the NovaSeq 6000 platform (Illumina). RefSeq transcripts (coded and non-coded) were downloaded from ENSEMBL (//asia.ensembl.org/). Reads were mapped to the mouse genome (mm10) using STAR (version 2.7.9a). The DEG was detected using the "FindMarkers" function (log2fc.threshold>0.25 and p_val_adj<0.05). Following GO enrichment analysis using DAVID, the DEG was identified based on log₂|fold-change|.

### Vaccine Design and Synthesis

First, based on the report of Evdokimova et al. (Evdokimova V, Tognon CE, Benatar T, Yang W, Krutikov K, Pollak M, Sorensen PH, Seth A. IGFBP7 binds to the IGF-1 receptor and blocks its activation by insulin-like growth factors. Sci Signal. 2012 Dec 18; 5 (255): ra92. doi: 10.1126/scisignal. 2003184. PMID: 23250396.), the functional region of IGFBP7 was examined, and it was found that the site of 1 to 98 amino acids on the N-terminal side interacts with IGFR1. From the sites, two candidate peptides were designed in consideration of protein structure, hydrophilicity and hydrophobicity: Epitope 1 (vac#1) corresponding to the region of N-terminal sequence (86 to 97 amino acids: CVKSRKRRKGKA (SEQ ID NO: 13)) of human IGFBP7; and Epitope 2 (vac#2) corresponding to the region of the N-terminal sequence of human IGFBP7 (47 to 55 amino acids: CPLGETRDA (SEQ ID NO: 14)). After synthesis of these peptides, the N-terminus of each peptide was conjugated to KLH, and the conjugated peptide was purified by reverse phase high performance liquid chromatography (HPLC) (>98% purity) (Peptide Institute). Before immunization, the peptide solution was mixed with an equal volume of alhydrogel adjuvant 2%

### (Invivogen).

### Evaluation of IGFBP7 Vaccine Design

Seven to eight week old male mice (C57BL/6 J) were purchased from CLEA Japan. Mice (n=5) were intradermally immunized with 100 mg KLH-binding peptides 1 and 2, followed by the same immunization for 1 week and 2 weeks. Control mice were injected with KLH in alhydrogel adjuvant according to the same protocol. To evaluate the persistence of the vaccine, blood samples of mice were taken from the tail vein 2, 4 and 6 weeks after the first immunization.

### Evaluation of Anti-IGFBP7 Antibody Titer in Immunized Mice

ELISA plates (MaxiSorp Nunc; Thermo Fisher Scientific) were coated with 5 mg/mL of the candidate IGFBP7 peptide in carbonate buffer overnight at 4°C. The peptides were conjugated to BSA carrier proteins (Peptide Institute). After blocking with PBS containing 5% skim milk, serum was diluted in a range of 100 to 312,500 fold with blocking buffer. After overnight incubation at 4°C and washing, the plates were incubated with a horseradish peroxidase (HRP)-conjugated antibody specific for mouse IgG (GE Healthcare) for 3 h at room temperature. After washing with PBS, the reaction was developed using the peroxidase chromogenic substrate 3,3'-5,5'-tetramethylbenzidine (Sigma-Aldrich) and quenched with 0.5 N sulfuric acid. Absorbance at 450 nm was monitored with a microplate reader (iMARK, Bio-Rad).

### AAV9-CMVIgfbp7 Infection

AAV vectors were prepared by VectorBuilder Inc. (https://en.vectorbuilder.com) following established procedures (Document 34). Mice were administered 2×10¹² GC doses of AAV9-CMV-Igfbp7 or 2×10¹¹ GC doses of EGFP control AAV9.

### Patient Recruiting and Gene Analysis

All experiments using cells and tissues obtained from patients were approved by the Institutional Review Board (IRB) at the University of Tokyo Hospital (Approval Nos. G-2249, G-10032, 11044, and 11801).

### Example 1: Identification of Htra3 as Central Molecule of Cardiac Fibroblasts by Single Cell Network Analysis

To investigate the molecular interactions leading to the induction of failing cardiomyocytes, cardiomyocytes and non-cardiomyocytes were separated from mice 2 weeks after transverse aortic constriction (TAC) or sham surgery, single cell analysis of the droplet-based encapsulation using the 10X Genomics platform and detailed transcriptional phenotypic analysis by Smart-seq2 profiling (Document 21) were performed (Fig. 1Aa and Figs. 1Ba to 1Bd). 1,019 expression profiles of ligand and receptor (LR) from all datasets were used to generate a co-expression network map to reveal the cell-type specific transcription properties of LR in the heart (Fig. 1Ab). The LR interaction database (Document 22) was then used to reconstruct the LR interaction network map of the heart to reveal the potential comprehensive interactions between the same or different cell types (Fig. 1Ac, Table S1, and Supplementary Data 1). Pathway analysis revealed that specific signaling pathways, such as PI3K-Akt, Rap1, and TGF-β signaling pathways, were strongly activated in the heart (Fig. 1Ad). Among the various cell types, cardiac fibroblasts interacted most strongly with multiple cell types, including cardiomyocytes (Fig. 1Ae). Pressure overload with TAC increased the cardiac fibroblast population in which extracellular matrix-associated genes such as Ctgf, Tgfb1, and Fbn1 were activated (Figs. 1Af and 1Be).

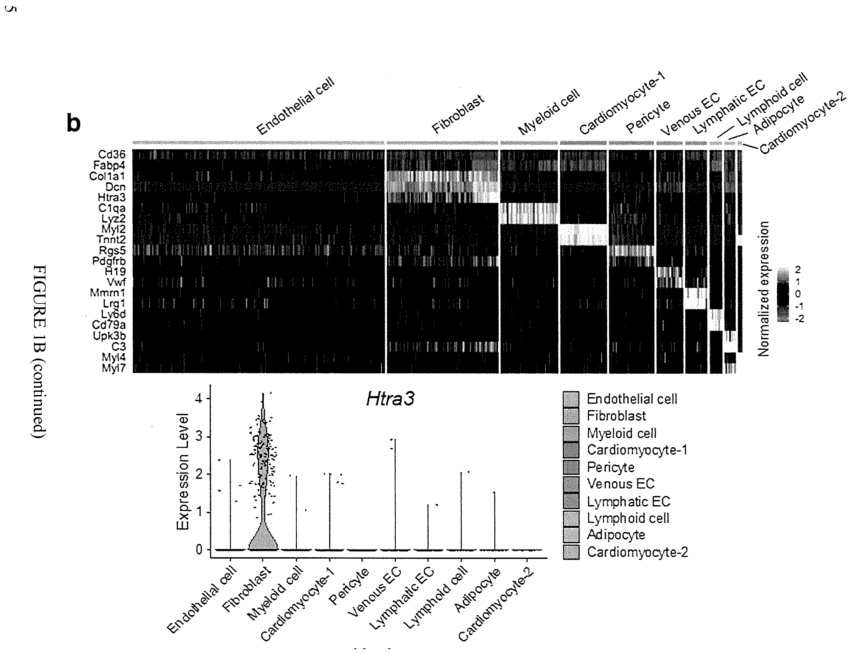

Single cell co-expression network analysis (Documents 12 and 23) was performed to identify key gene modules characteristic of each cell type (Figs. 1Bf to 1Bh). By generating a cardiac fibroblast network, *High-temperature requirement A serine peptidase* 3 (Htra3) is identified as a molecule located in the center of the network, together with TGF-β signaling related molecules such as Dcn (decorin) and Islr (Meflin) (Fig. 1Ag, Fig. 1Bh, and Supplementary Data 2)(Documents 24 and 25). Htra3 expression strongly correlated with expression of cardiac fibroblast modules (Fig. 1Ah), suggesting that Htra3 defines cardiac fibroblast identity. Although Htra3 has been reported to be specifically expressed in placenta and heart and involved in the development of placenta (Documents 26 and 27) (Fig. 1Bj), its role in the heart is not known. Cardiac fibroblast-specific Htra3 expression was confirmed by single-cell analysis (Figs. 1Ai and 1Bb) and by immunostaining and RNA-in-situ hybridization of Pdgfr-α, a well-known cardiac fibroblast marker protein (Fig. 1Aj). Western blot analysis also showed that Htra3 protein is specifically detected in non-cardiomyocytes, including cardiac fibroblasts (Fig. 1Bk). Furthermore, HTRA3 expression in the human cardiac interstitium was also confirmed (Fig. 1Ak).

### Example 2: Cardiac Fibroblast Htra3 governs Cardiac Homeostasis and is Downregulated by Pressure Overload

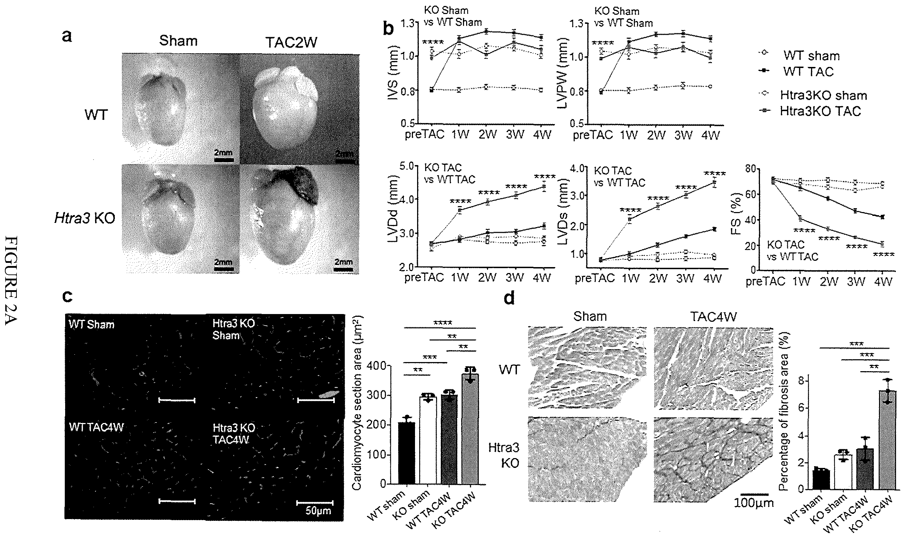

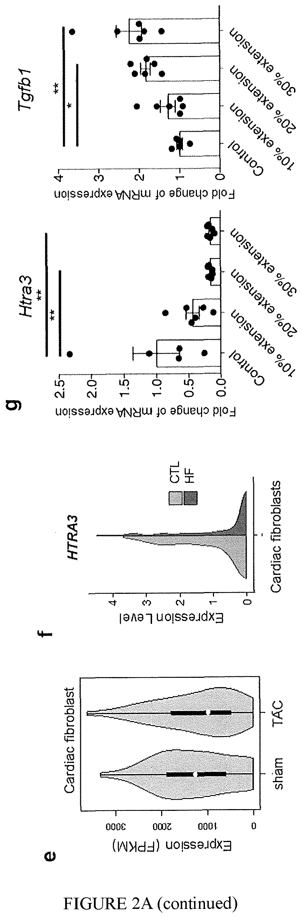

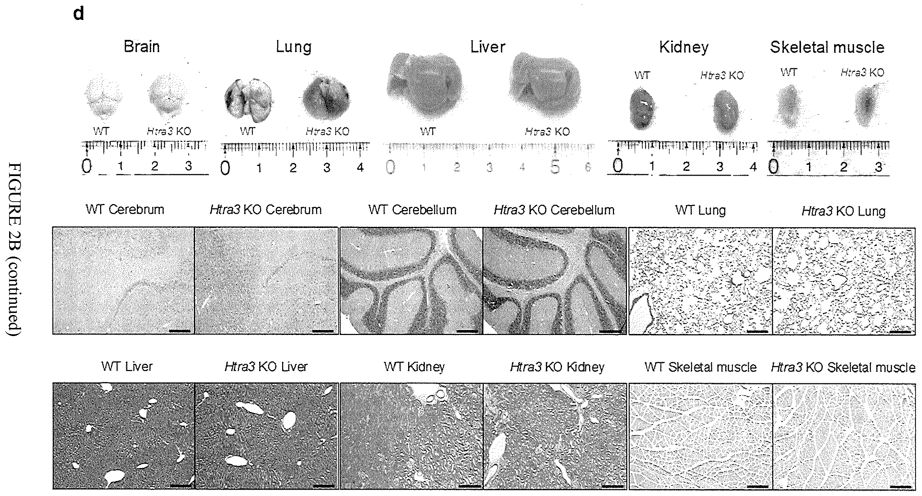

To investigate the functional role of Htra3 in the heart, Htra3 knockout mice were generated (Supplemental Fig. S2a). Compared with wild-type (WT) control mice, Htra3 knockout mice exhibited cardiac hypertrophy with expansion of cardiomyocyte size even in the absence of pressure overload without changes in blood pressure (Figs. 2Aa-c, 2Bb, and c). Additional mild pressure overload with TAC rapidly induced severe heart failure in Htra3 knockout mice but not in wild-type mice (Fig. 2Ab). In major organs other than the heart, there were no apparent morphological and histological differences between wild type and Htra3 knockout mice (Fig. 2Bd). Sirius Red/Fast Green collagen staining showed that TAC induced more severe cardiac fibrosis in Htra3 knockout mice than in wild-type mice (Fig. 2Ad). Pressure overload reduced the expression level of Htra3 in cardiac fibroblasts of wild-type mice (Fig. 2Ae). Single cell analysis of human hearts confirmed specific expression of HTRA3 in cardiac fibroblasts and reduced expression thereof in heart failure hearts (Document 4) (Fig. 2Af and Figs. 2Be and 2Bf). Mechanical stretch of isolated primary cardiac fibroblasts significantly suppressed Htra3 expression and increased Tgfb1 expression (Fig. 2Ag). These results suggested that Htra3 specifically expressed in cardiac fibroblasts is essential to maintain the size of the heart in a basal state and functions against hemodynamic overload and that mechanical stress on cardiac fibroblasts reduces Htra3 expression.

### Example 3: Htra3-induced degradation of TGF-β is essential for the prevention of heart failure and fibrosis

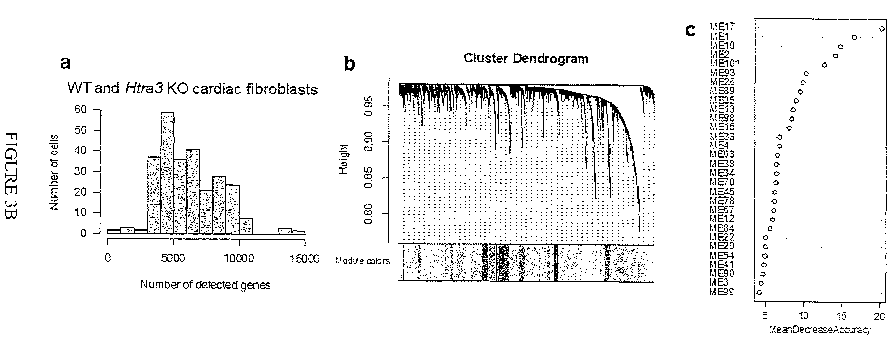

To elucidate the role of Htra3 in cardiac fibroblast phenotype, the wild-type and Htra3 knockout mice at 2 weeks after TAC or sham surgery were used and performed full-length single-cell analysis (full-length sc-RNA seq, Smart-seq2) of cardiac fibroblasts isolated using an antibody against Pdgfr-α (Fig. 1Aa and Fig. 3Ba).

Uniform Manifold Approximation and Projection (UMAP) plots and graph-based clustering separated cardiac fibroblasts into 3 clusters (C1-3), and pseudotime analysis revealed 2 trajectories (Figs. 3Aa and 3Ab). Fibroblast trajectory 1 (C1 to C2) was mainly induced by Htra3 deletion and fibroblast trajectory 2 (C1 to C3) was induced by pressure overload with or without Htra3 deletion (Figs. 3Aa to 3Ac). Co-expression network analysis and random forest analysis revealed that modules M1 and M17 (Figs. 3Bb and 3Bc), which are largely involved in cell classification, are mutually exclusive in modular activity (Fig. 3Ad).

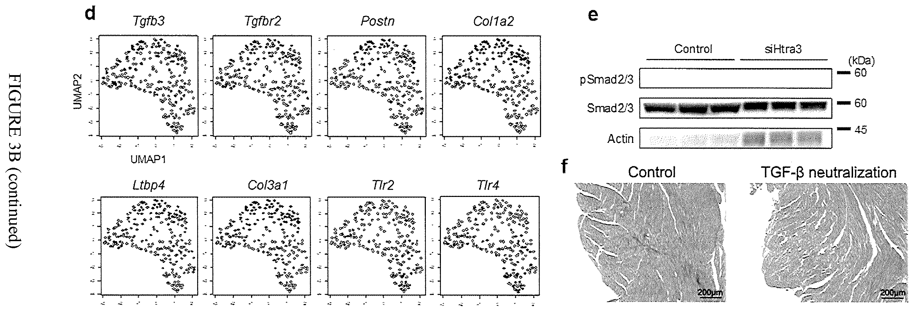

Module M1 contains genes involved in innate immunity and Toll-like receptor signaling pathways including Tlr2 and Tlr4 (Figs. 3Ae and 3Af), which were shown to be expressed in cardiac fibroblasts (Document 28). The activity of the module M1 was strongly suppressed between trajectories 1 and 2 (Figs. 3Ae to 3Ag and Fig. 3Bd), both of which were associated with Htra3 downregulation, suggesting that Htra3 is important for maintaining the quiescent state of cardiac fibroblasts (Documents 6 and 29).

Module M17 was enriched in genes involved in extracellular matrix formation and TGF-β signaling pathways, and its activity was upregulated to different degrees in both trajectories 1 and 2 (Fig. 3Ah-j and Fig. 3Bd). Either pressure overload or Htra3 deletion activated M17 and they synergistically enhanced its activity (Fig. 3Aj). Expression of collagen fiber organization genes such as Col1a1 and Col3a1 was activated in both trajectories, whereas expression of TGF-β signaling molecules such as Tgfb3 and Tgfbr2 and Postn (Document 6), a marker for activated cardiac fibroblasts, was activated only in trajectory 2 (Fig. 3Ai). These findings showed that Htra3 essentially inhibited TGF-β signaling and pressure overload and Htra3 deletion synergistically activated TGF-β signaling leading to fibroblast activation.

Next, Htra3 was examined how Htra3 inhibits TGF-β signaling in vitro and in vivo. Biochemical analysis using primary cardiac fibroblasts revealed that overexpression of Htra3 reduced the protein levels of both TGF-β1 and phosphorylated Smad2/3 (phospho-Smad2/3), a marker of activated TGF-β signaling (Fig. 3Ak and Fig. 3Be). Htra3 also binds directly to TGF-β (Fig. 3Al), suggesting that Htra3 inhibits TGF-β signaling by degrading TGF-β. Phospho-Smad2/3 immunostaining of cardiac tissue showed that either Htra3 deletion or TAC activated Smad2/3 and their combination activated Smad2/3 synergistically not only in cardiac fibroblasts but also in cardiomyocytes (Fig. 3Am). Furthermore, TGF-β-neutralizing antibodies significantly ameliorated systolic dysfunction and cardiac fibrosis in Htra3 knockout mice after TAC surgery (Fig. 3Bf, g). In addition, TGF-β neutralizing-antibody injection from 4 weeks of age also improved ventricular hypertrophy (Fig. 3Bh). These results suggest that Htra3-induced TGF-β degradation is essential for the prevention of heart failure and fibrosis.

### Example 4: Htra3 repression-induced activation of TGF-β signaling promotes the induction of senescent failing cardiomyocytes.

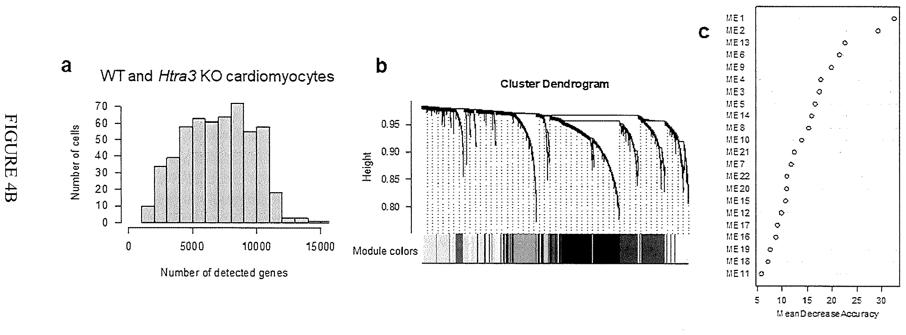

To understand how activation of TGF-β signaling affects cardiomyocytes and leads to heart failure, a single cell analysis of cardiomyocytes isolated from wild-type and Htra3 knockout mice at two weeks after TAC or sham surgery was performed. The UMAP plots classified cardiomyocytes into four clusters and pseudotime analysis revealed two trajectories (Figs. 4Aa, 4Ab, and 4Ba). Co-expression network analysis and random forest analysis identified modules M1 and M2 as being highly involved in cell classification (Figs. 4Bb and Fig. 4Bc). Htra3 deletion, TAC surgery, and their combination induced the transition of cardiomyocytes from C1 to C2, C3, and C4, respectively (Fig. 4Ac). Trajectories 1 and 2 of cardiomyocytes correspond to C1 to C3 transition via C2 and C1 to C4 transition via C2, respectively (Fig. 4Ab).

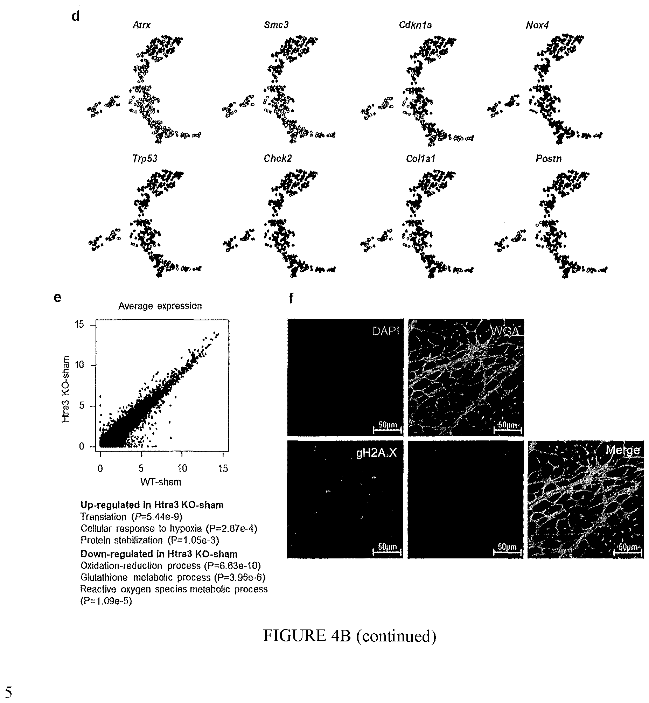

Module M1 enriched in genes involved in mitochondrial oxidative phosphorylation and DNA repair (Atm, Parp1/2, Rpa2, or the like) (Fig. 4Ad) was significantly repressed in both trajectories 1 and 2 (Figs. 4Ad to 4Af and Fig. 4Bd), suggesting that activation of TGF-β signaling represses expression of genes associated with mitochondrial and DNA repair (Figs. 4Ad and 4Ae). In cardiomyocytes from Htra3 knockout mice, genes involved in oxidative-reduction processes are down-regulated and genes involved in protein synthesis are up-regulated without pressure overload (Fig. 4Be), which may lead to cardiomyocyte hypertrophy in Htra3 knockout mice (Fig. 2Ac).

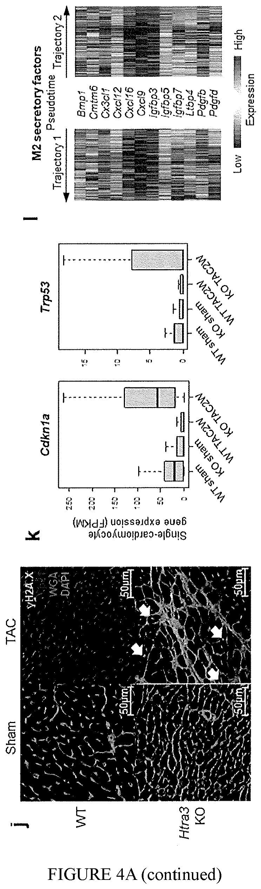

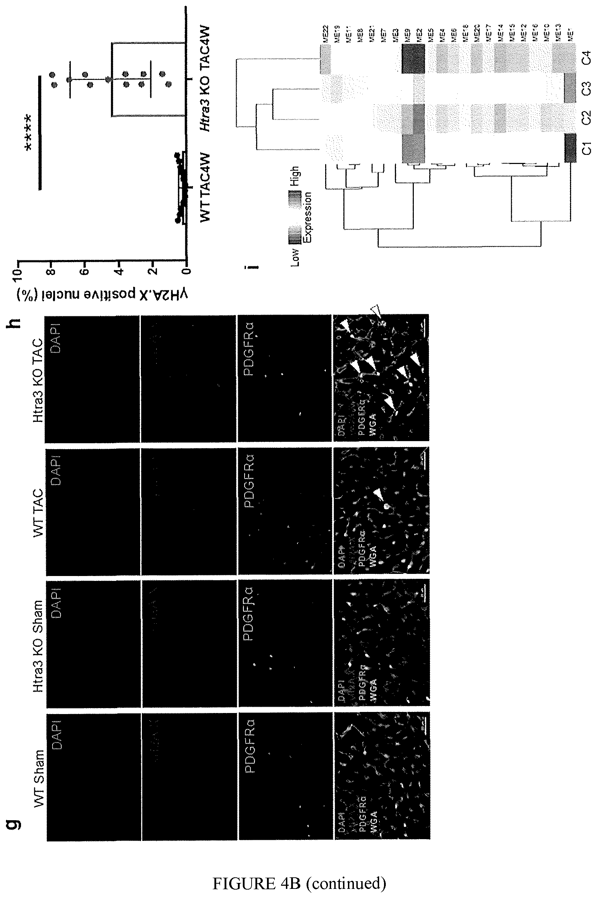

The module M2, containing genes associated with small GTPase signaling (such as Rhob and Rac1), TGF-β receptor signaling (such as Ltbp4 and Tgfbr2), and p53 signaling (such as Trp53 and Cdkn1a), was specifically activated at C4 via the trajectory 2 (Fig. 4Ag to 4Ai). The combination of Htra3 deletion and pressure overload significantly promoted the induction of failing cardiomyocytes stained with antibodies against γH2A.X and p21 (Document 12) (Fig. 4Aj and Fig. 4Bf). Immunostaining also showed increased DNA damage accumulation in cardiac fibroblasts of Htra3 knockout mice after TAC surgery (Figs. 4Bg and 4Bh), suggesting that activation of TGF-β signaling by Htra3 inhibition increased DNA damage not only in cardiomyocytes but also in cardiac fibroblasts. In addition, an increase in expression of DNA damage-related genes in cardiomyocytes of Htra3 knockout mice after TAC surgery was confirmed (Fig. 4Ak). M2 also includes various types of secretory factors, such as Bmp1, Cxcl12, or Igfbp7, suggesting that cardiomyocytes expressing M2 display a DNA damage-induced secretion phenotype similar to the senescence associated secretory phenotypes (SASP) (Fig. 4Al). Clustering analysis confirmed that module M9 rich in genes encoding proteins associated with collagen fiber organization (Col1a1, Col3a1, Fbn1, or the like) and TGF-β signaling (Tgfb3, Nox4, Postn, or the like) was also specifically activated at C4 like M2 (Figs. 4Am to 4Ao and Fig. 4Bi).

### Example 5: TGF-β Induced Nox4 Expression followed by p53 Activation is Essential for Induction of Failing Cardiomyocytes with Secretory Phenotype

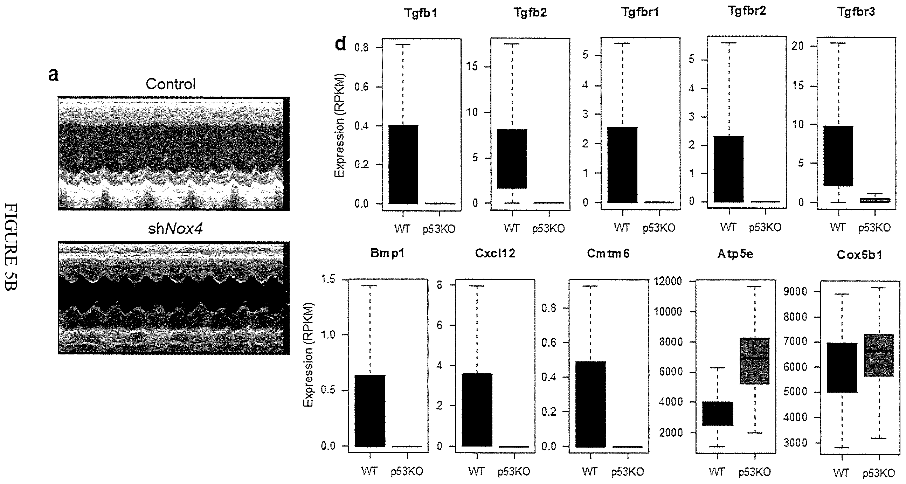

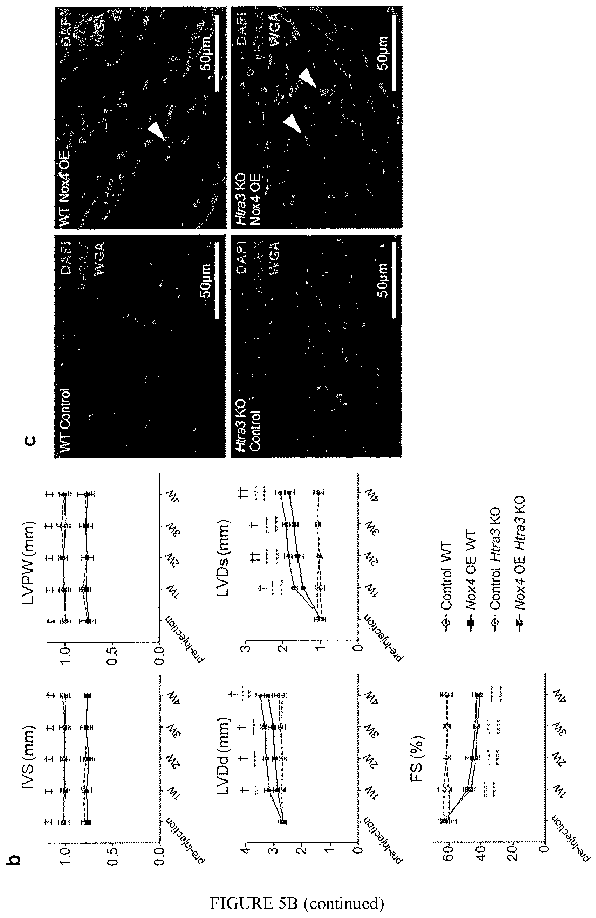

To elucidate whether TGF-β-induced activation of Nox4 (Document 33) in M9, a major ROS-generating NADPH oxidase (Documents 31 and 32) in cardiomyocytes (Document 30), is involved in increased DNA damage and development of heart failure, a Nox4-shRNA adeno-associated virus 9 (AAV9) vector that suppresses *Nox4* expression was injected (Fig. 5Aa). Echocardiography revealed that knockdown of Nox4 rescues cardiac dysfunction in Htra knockout mice after TAC surgery (Fig. 5Ab and Fig. 5Ba). Immunostaining and western blot analysis showed that Nox4 suppression significantly reduced DNA damage (Figs. 5Ac and 5Ad) and expression of TGF-β signaling-related molecules (Fig. 5Ae). Overexpression of Nox4 in the heart using the AAV9 vector increased the number of γH2A.X-positive cardiomyocytes and significantly deteriorated cardiac dilatation and function (Figs. 5Bb and 5Bc), consistent with previous reports (Document 33). Single cell analysis of cardiomyocytes isolated from cardiomyocyte-specific p53 knockout mice after TAC surgery showed that p53 deletion suppressed the TGF-β signaling-related molecule (for example, Tgfb3 and Tgfbr2) and secretory factor (for example, Cxcl12 and Igfbp7) in M2 (Fig. 5Bd). The AAV9-Htra3 vector was then injected to wild-type mice at one week after TAC surgery. Overexpression of Htra3 in the heart inhibited TGF-β signaling and ameliorated cardiac dysfunction and fibrosis (Figs. 5Af to 5Aj). These results suggest that TGF-β signaling suppresses DNA repair genes and promotes ROS production via Nox4, thereby inducing accumulation of DNA damage and subsequent activation of p53 signaling, resulting in induction of senescent failing cardiomyocytes characterized by expression of TGF-β signaling-related molecules and secretory factors. Htra3-induced TGF-β suppression (TGF-β suppression by Htra3) may be a novel therapeutic approach for heart failure.

### Example 6: Spatial Transcriptome demonstrates that Htra3-TGF-β Axis is Essential for Prevention of Cardiac Fibrosis and Induction of Cardiomyocyte Secretory Phenotype in Infarcted Area after Myocardial Infarction

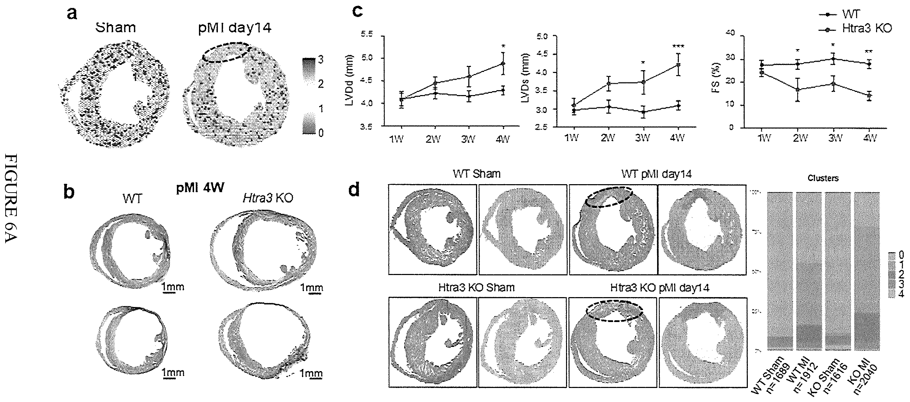

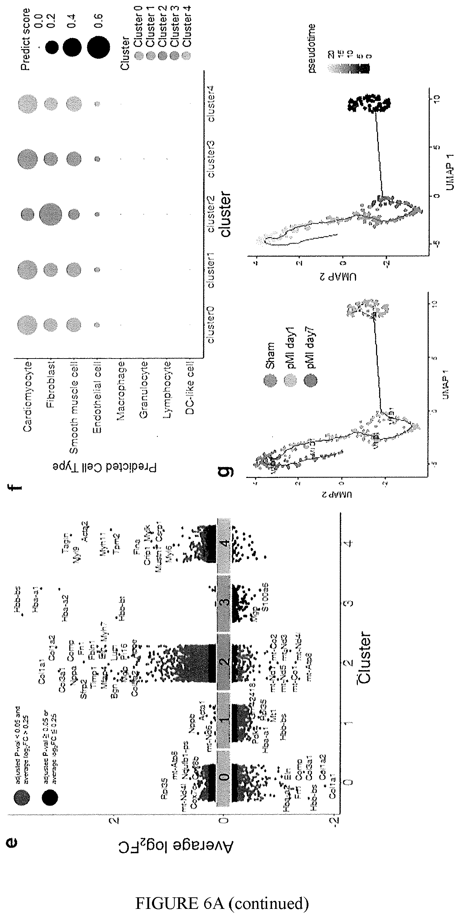

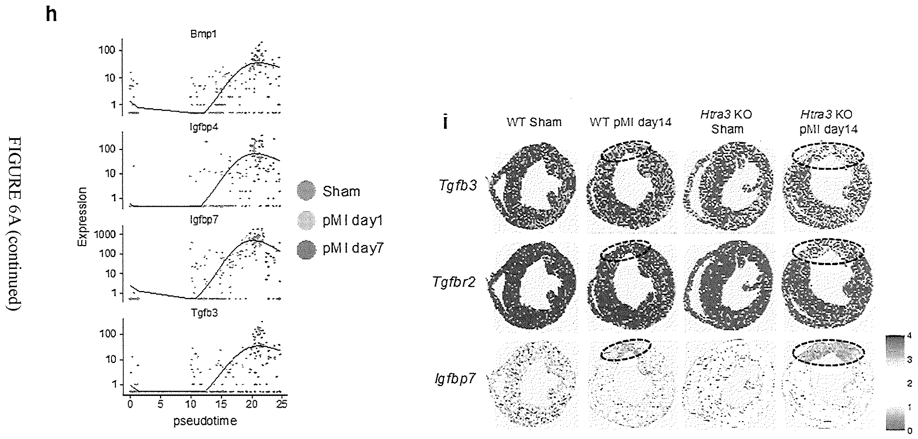

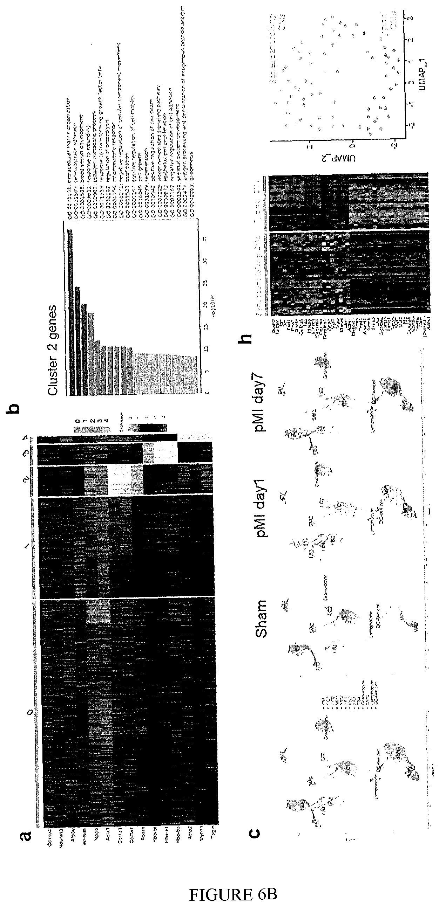

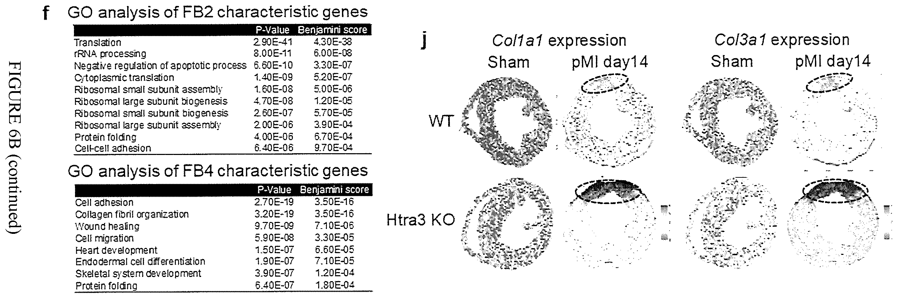

To elucidate how Htra3 spatially modulates cardiac remodeling, the role of Htra3 in the myocardial infarction (MI) model was investigated. Spatial transcriptome analysis using Visium (10X Genomics) showed high Htra3 expression in the infarct zone after MI, suggesting a possibility for spatial regulation in cardiac remodeling (Fig. 6Aa). Htra3 knockout mice showed severe cardiac remodeling after MI compared with wild-type mice (Fig. 6Ab). Echocardiographic analysis revealed that Htra3 knockout mice exhibited significant cardiac dilation and systolic dysfunction (Fig 6Ac). Spatial transcriptome analysis of cardiac tissue from wild-type and Htra3 knockout mice following sham or MI surgery identified specific gene clusters characterized by spatial localization (Fig. 6Ad) and specific expression profiles (Fig. 6Ae and Fig. 6Ba). MI induced expression of cluster 1 genes enriched in genes expressed in mitochondria (examples: mt-Nd6 and mt-Atp6) in the non-infarct zones of both wild-type and Htra3 knockout mice (Figs. 6Ad and 6Ae). The area assigned to cluster 2, corresponding to the infarct zone, was enlarged in Htra3 knockout mice after MI (Fig. 6Ad). Gene ontology analysis showed that Cluster 2 was characterized by genes involved in several biological phenomena, such as organization of the extracellular matrix, response to TGF-β, inflammatory responses, and integrin-mediated signaling pathway (Fig. 6Bb). Single cell analysis of cells isolated from the infarct zone following MI was then performed (Fig. 6Bc) and the spatially arranged spots were deconvoluted using a single cell analysis profile to indicate that fibroblasts were highly enriched in the regions assigned to Cluster 2 (Fig. 6Af). After MI, the fibroblast population was divided into four clusters (Fig. 6Bc). Fibroblast clusters 2 (FB2) and 4 (FB4), which were increased in the early stages after myocardial infarction, showed lower expression levels of *Htra3* (Figs. 6Bd to 6Bf), and the total expression levels of Htra3 were decreased in the early stages after MI (Fig. 6Bg), consistent with the finding that *Htra3* expression in cardiac fibroblasts was downregulated following cardiac pressure overload (Fig. 2Ae) or mechanical stretch (Fig. 2Ag). Trajectory analysis of cardiomyocytes following MI was also performed using a single cell analysis profile, demonstrating that MI elicited failing cardiomyocytes characterized by expression of TGF-β signaling-related molecules and secretory factors (for example, Bmp1, Tgfb3, and Igfbp7) (Figs. 6Ag and 6Ah and Figs. 6Bh and 6Bi). Spatial transcriptome analysis demonstrates that these genes, along with extracellular matrix genes, are specifically activated in the infarct zone of *Htra3* knockout mice after MI (Fig. 6Ai and Fig. 6Bj), suggesting that Htra3 downregulation promotes cardiac fibrosis and induces the secretory phenotype of cardiomyocytes in the infarct regions.

### Example 7: Transcriptome Signatures of Failing cardiomyocytes are conserved in Human Heart Failure

To evaluate conservation of transcriptome signatures in human failing cardiomyocytes, full-length single cell analysis of cardiomyocytes isolated from heart failure patients (n=22) and control subjects (n=2) was performed. UMAP plots and graph-based clustering classified cardiomyocytes into three clusters and pseudotime analyses revealed two trajectories (Figs. 7Aa, 7Ab, and 7Ba). Trajectories 1 and 2 corresponded to transitions from C1 to C2 and from C1 to C3, respectively (Fig. 7Ab). The cardiomyocytes of the control subjects were predominantly in C1 whereas the cardiomyocytes of heart failure patients were in C2 or C3 (Fig. 7Ac). Random forest and overlap analysis of the identified co-expressed gene modules identified modules M1, M2, and M44 as being highly involved in cell classification and conserved between humans and mice (Figs. 7Bb to 7Bd).

Corresponding to mouse cardiomyocyte M1 (Fig. 7Bd), the module M2 rich in genes involved in mitochondrial electron transport and ATP biosynthesis (Fig. 7Ad) was suppressed in both trajectories (Figs. 7Ad to 7Af). In contrast, corresponding to murine cardiomyocytes M2 (Fig. 7Bd), type I interferon signaling (IFNAR1, IRF9, JAK1, or the like), TGF-β receptor signaling (TGFB3 and TGFBR2), small GTPase signaling (such as RHOA or RAC1), and DNA damage response (CDKN1A, MDM4, RBX1, or the like) were specifically activated in the trajectory 2 (Figs. 7Ag to 7Ai and Fig. 7Be), and M1 and M2 showed mutually exclusive relationships in modular activity (Fig. 7Aj). M1 also included secretory factors specifically expressed in murine senescent failing cardiomyocytes (example: CXCL12 and IGFBP7) (Fig. 7Ah and Fig. 4Bg). By calculating the overlap between the gene modules detected from the single cardiomyocyte analysis of human and mouse, it was also found that M44 (Fig. 7Bd) corresponding to the mouse cardiomyocyte M9 is enriched and activated in genes involved in extracellular matrix organization, particularly in the trajectory 2 (Fig. 7Ak-m). This was confirmed by singlemolecule RNA in situ hybridization (Fig. 7Bf). These results suggested that human failing cardiomyocytes show the same conserved characteristics as murine failing cardiomyocytes, including impaired mitochondrial biogenesis, activated TGF-β receptor signaling, DNA damage response, and fibroblast-like secretory phenotype.

### Example 8 (1): IGFBP7 secreted from Failing Myocytes is Biomarker of Heart Failure Progression

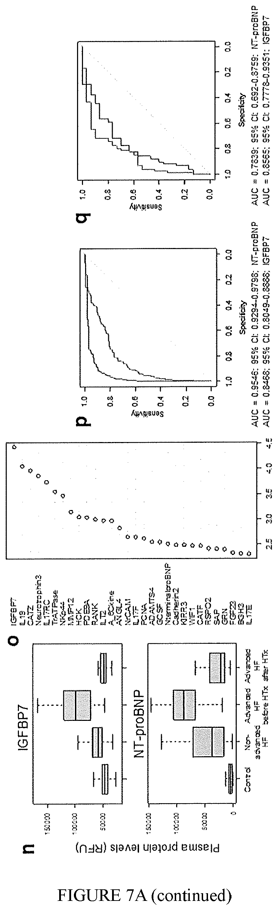

To determine whether cytokines secreted from failing cardiomyocytes may be related to the pathogenesis of heart failure, plasma proteome analysis of human heart failure at various stages was performed (control subjects n=768, non- advanced heart failure patients n=84, advanced heart failure patients before and after heart transplant n=30) (Document 34). Levels of cytokines, such as TGF-β3, LTBP4, or IGFBP7, which were expressed from senescent failing cardiomyocytes, were high in heart failure patients (depending on severity level) and low after transplantation, similar to NT-proBNP, an established biomarker for heart failure (Document 35) (Fig. 7An and Fig. 7Bg). Random forest analysis identified IGFBP7 as the protein most strongly involved in the classification of non-advanced and advanced heart failure (Fig. 7Ao). Receiver operating characteristic analysis showed that NT-proBNP was superior in its ability to diagnose heart failure, whereas IGFBP7 was superior in its ability to determine the severity level of heart failure (Figs. 7Ap and 7Aq). These results suggest that IGFBP7 secreted from failing cardiomyocytes is a useful biomarker for human advanced heart failure.

### Example 8 (2): Overexpression ofIgfbp7 in Cardiomyocytes Impairs Cardiac Function

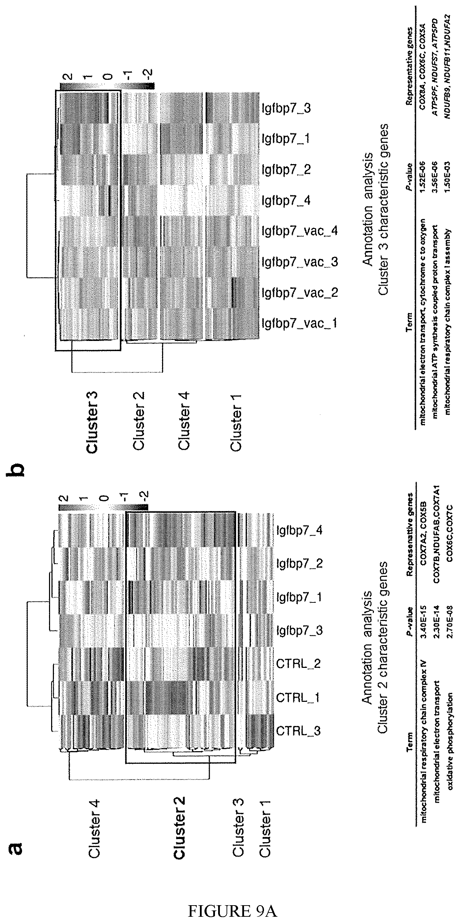

To determine the role of Igfbp7 in cardiac function, Igfbp7 was first overexpressed in the heart using the AAV9 vector (Document 0021) and subjected to pressure overload. Echocardiography revealed a significant reduction in cardiac function in Igfbp7 overexpressing mice compared to mice injected with control vector at TAC for 2 weeks (Fig. 8Aa). The weighted gene correlation network analysis (WGCNA) and annotation analysis of cardiomyocytes revealed that expression of the ME3 module including OXPHOS related gene groups was suppressed in Igfbp7 overexpressing mice (Fig. 8Ab, 8Ac, and 8Ba to 8Bc). In Igfbp7 overexpressing mice, the two clusters were cardiomyocytes infected with many AAVs and non-infected cardiomyocytes. Annotation analysis of the ME1 gene showed that cell populations expressing more Igfbp7, that is, those infected with AAV, were more likely to suggest reduced insulin signaling (Fig. 8Bd). This suggests that Igfbp7 has an autocrine effect on cardiomyocytes. In addition, human recombinant IGFBP7 was added to human iPS cell-derived cardiomyocytes (hiPSCM). Bulk RNA-seq analysis revealed that expression of mitochondrial electron transport pathway and complex IV-related genes was downregulated in IGFBP7-treated hiPSCM (Fig. 9Aa). These results suggest that overexpression of Igfbp7 causes cardiac dysfunction by inhibiting oxidative phosphorylation of mitochondria.

### Example 9: Igfbp7 Vaccine improves Cardiac Function

In recent years, vaccine-based therapies targeting endogenous proteins have attracted lots of attention for a variety of non-transmissible diseases such as cancer (Document 0022), hypertension (Document 0023), or hyperlipidemia (Document 0024). To develop a vaccine against Igfbp7, two B cell epitopes were selected from putative binding sites with other proteins (Document 0016) based on the amino acid composition and predicted structure of the N-terminal region. Vaccines were prepared by combining the synthesized epitope peptides with keyhole limpet hemocyanin (KLH) and administered to mice with aluminum hydroxide as adjuvant. After three injections of the vaccine in mice, endogenous antibody production was confirmed to be sufficient by ELISA (Fig. 8Ad). Administration of the vaccine to mice prior to TAC significantly suppressed a reduction in cardiac function compared to controls (Fig. 8Ae and Fig. 9Ba). Single cell analysis of cardiomyocytes from vaccinated mice was performed. WGCNA analysis of cardiomyocytes revealed that expression of the ME 109 gene associated with OXPHOS and TCA cycles was upregulated in TAC mice after administration of vaccine #2 (Figs. 8Af to 8Ag and Fig. 9Bd). The ME38 gene is elevated in cardiomyocytes of vaccinated mice and is associated with mTORC1 and RNA polymerase II (Fig. 9Bc). This result suggests that cardiomyocytes under pressure produce various proteins to improve cardiac function by improving glucose uptake. Nppa, a marker gene for heart failure, was downregulated in cardiomyocytes of the hearts of vaccinated mice compared to the hearts of TAC mice (Fig. 9Bd). This result suggests that cardiomyocytes in a state of heart failure due to pressure loading are released from the state of heart failure by the vaccine. In addition, hiPSCM was treated with serum collected from mice after vaccination. Bulk RNA-seq analysis revealed that expression of mitochondrial electron transport pathway-related genes was upregulated in the serum-treated group (Fig. 9Ab).

### Example 10: Vascular Endothelial Cells of Heart of DCM Patients Express IGFBP7

Single nuclei RNA-seq of heart biopsy samples from dilated cardiomyopathy (DCM) patients with severe heart failure (N=3) and control patients (N=3) were examined (Fig. 10a). The expression level of IGFBP7 was significantly upregulated in cardiovascular endothelial cells of patient samples compared to control samples (Fig. 10b). Single nuclear RNA-seq analysis of endothelial cells using Seurat revealed that IGFBP7 expression was upregulated in DCM patients (Figs. 8Ah to 8Ai). Annotation analysis of cardiomyocytes from DCM patients showed a decrease in oxidative phosphorylation pathways (Fig. 10c). These results suggest that IGFBP7, in pressure-overloaded mice, is involved in the pathogenesis of human heart failure due to a variety of causes.

### Discussion

This study showed that cardiac fibroblasts are critically involved in the development of heart failure by inducing not only cardiac fibrosis but also cardiomyocyte secretory phenotype via the Htra3-TGF-β-IGFBP7 axis. Mechanical stress on fibroblasts reduces Htra3 expression and causes upregulation of TGF-β in the heart. Sustained activation of TGF-β signaling represses the expression of many DNA repair genes in cardiomyocytes, consistent with previous findings observed in cancer and bone marrow failure that the accumulation of DNA damage induced by TGF-β signaling is based on the transcriptional repression of DNA repair genes (Documents 36 and 37). Sustained activation of TGF-β signaling also activates Nox4 expression, a major cause of oxidative stress in heart failure (Document 33), promoting ROS production in cardiomyocytes. These events collectively induce accumulation of DNA damage and activation of p53 signaling, resulting in induction of failing cardiomyocytes with expression of TGF-β signaling-related molecules and secretory factors, similar to senescence in proliferative cells (Documents 2, 38, and 39).

TGF-β signaling and DNA damage are involved in many age-related disorders, such as neurodegenerative diseases (Document 40) and cancers (Document 41). As DNA damage and subsequent cellular senescence activate the expression of TGF-β signaling-related molecules that play a major role in paracrine senescence (Documents 42, 43, and 44), the vicious cycle between TGF-β signaling and DNA damage as well as the senescence associated disorders may be the pathological basis of heart failure. Since Htra3 expression was downregulated in murine and human cardiac fibroblasts of heart failure and overexpression of Htra3 in the murine heart inhibited TGF-β signaling and ameliorated cardiac dysfunction and fibrosis (Figs. 5Ah to 5Aj), Htra3-mediated prevention of cardiac fibrosis and the secretory phenotype of cardiomyocytes would represent a novel therapeutic strategy for heart failure in addition to senolytic therapies (Documents 18 and 45). Htra3 expression has been shown to be downregulated as endometrial and ovarian cancer grades increase (Documents 46 and 47), suggesting that Htra3 may also be a target for cancer therapy.

Integrative analyses of human single cardiomyocyte transcriptome and plasma proteome identified a clinically significant association between failing cardiomyocytes and cytokines including IGFBP7. It has been reported that IGFBP7 expression is upregulated by TGF-β via smad248 and TGF-β-induced IGFBP7 expression in the stroma defines a poor prognostic subtype of colorectal cancer (Document 49). IGFBP7 has been reported to be secreted from senescent cells and to have the ability to induce senescence (Document 50), suggesting that IGFBP7 itself may be involved in the development of heart failure. Context-dependent SASPs have been reported to affect the tumor microenvironment and immune response (Documents 51 and 52) and may be therapeutic targets (Documents 53 and 54). Similarly, the failing cardiomyocyte-specific secretory phenotype and its secreted cytokine IGFBP7 could be potential therapeutic targets for heart failure.

Despite the importance of the findings, our research has several limitations. First, no fibroblast-specific Htra3 knockout mice were used. However, since Htra3 is mainly expressed in cardiac fibroblasts (Fig. 1Ai and Figs. 1Bj, 1Bk, and 6Bf), global Htra3 knockout mice are thought to be sufficient to assess the functional importance of Htra3 in the heart. Second, single cardiomyocyte analysis of human samples may bias the transcriptome due to differences in time from death (for control subjects) or tissue sampling (for patients with heart failure) to cell separation. However, the same cut-off was set for all single cell analysis data for subsequent analysis in quality control, and the detected gene modules corresponded to gene modules detected from murine single cardiomyocyte analysis. In addition, several secretory factors that are secreted from failing cardiomyocytes and associated with the severity level of heart failure were identified by integration with plasma proteome analysis. Our data set will proceed with further studies on the etiology of heart failure.

### Reference Documents

1. Henderson, N. C., Rieder, F. & Wynn, T. A. Fibrosis: From mechanisms to medicines. Nature 587, 555-566 (2020).
2. Gorgoulis, V et al. Cellular senescence: Defining a path forward. Cell 179, 813-827 (2019).
3. Lopez-Otin, C. & Kroemer, G. Hallmarks of health. Cell 184, 33-63 (2021).
4. Wang, L. et al. Single-cell reconstruction of the adult human heart during heart failure and recovery reveals the cellular landscape underlying cardiac function. Nat. Cell Biol. 22, 108-119 (2020).
5. Triposkiadis, F., Xanthopoulos, A. & Butler, J. Cardiovascular aging and heart failure: JACCC review topic of the week.J. Am. Coll. Cardiol. 74, 804-813 (2019).
6. Tallquist, M. D. & Molkentin, J. D. Redefining the identity of cardiac fibroblasts. Nat. Rev. Cardiol. 14, 484-491 (2017).
7. Yokota, T. et al. Type V collagen in scar tissue regulates the size of scar after heart injury. Cell 182, 545-562.e23 (2020).
8. Abe, H. et al. Macrophage hypoxia signaling regulates cardiac fibrosis via Oncostatin M. Nat. Commun. 10, 1-8 (2019).
9. Aghajanian, H. et al. Targeting cardiac fibrosis with engineered T cells. Nature 573, 430-433 (2019).
10. Sano, M. et al. p53-induced inhibition of Hif-1 causes cardiac dysfunction during pressure overload. Nature 446, 444-448 (2007).
11. Higo, T. et al. DNA single-strand break-induced DNA damage response causes heart failure. Nat. Commun. 8, 15104 (2017).
12. Nomura, S. et al. Cardiomyocyte gene programs encoding morphological and functional signatures in cardiac hypertrophy and failure. Nat. Commun. 9, 4435 (2018).
13. Nakada, Y et al. DNA damage response mediates pressure overload-induced cardiomyocyte hypertrophy. Circulation 139, 1237-1239 (2019).
14. Ko, T. et al. Quantification of DNA damage in heart tissue as a novel prediction tool for therapeutic prognosis of patients with dilated cardiomyopathy. JACC Basic Transl. Sci. 4, 670-680 (2019).
15. Sahin, E. et al. Telomere dysfunction induces metabolic and mitochondrial compromise. Nature 470, 359-365 (2011).
16. Oka, T. et al. Mitochondrial DNA that escapes from autophagy causes inflammation and heart failure. Nature 485, 251-255 (2012).
17. Sharifi-Sanjani, M. et al. Cardiomyocyte-specific telomere shortening is a distinct signature of heart failure in humans. J. Am. Heart Assoc. 6, e005086 (2017).
18. Baker, D. J. et al. Naturally occurring p16 Ink4a-positive cells shorten healthy lifespan. Nature 530, 184-189 (2016).
19. Martini, E. et al. Single-cell sequencing of mouse heart immune infiltrate in pressure overload-driven heart failure reveals extent of immune activation. Circulation 140, 2089-2107 (2019).
20. Wagner, J. U. G. & Dimmeler, S. Cellular cross-talks in the diseased and aging heart. J. Mol. Cell. Cardiol. 138, 136-146 (2020).
21. Picelli, S. et al. Full-length RNA-seq from single cells using Smart-seq2. Nat. Protoc. 9, 171-181 (2014).
22. Ramilowski, J. A. et al. A draft network of ligand-receptor-mediated multicellular signalling in human. Nat. Commun. 6, 7866 (2015).
23. Langfelder, P. & Horvath, S. WGCNA: An R package for weighted correlation network analysis. BMC Bioinforma. 9, 559 (2008).
24. Ferdous, Z., Wei, V M., Iozzo, R., Hook, M. & Grande-Allen, K. J. Decorintransforming growth factor-β interaction regulates matrix organization and mechanical characteristics of three-dimensional collagen matrices. J. Biol. Chem. 282, 35887-35898 (2007).
25. Hara, A. et al. Roles of the mesenchymal stromal/stem cell marker meflin in cardiac tissue repair and the development of diastolic dysfunction. Circ. Res. 125, 414-430 (2019).
26. Nie, G. et al. Serine peptidase HTRA3 is closely associated with human placental development and is elevated in pregnancy serum. Biol. Reprod. 74, 366-374 (2006).
27. Nie, G. Y, Hampton, A., Li, Y, Findlay, J. K. & Salamonsen, L. A. Identification and cloning of two isoforms of human high-temperature requirement factor A3 (HtrA3), characterization of its genomic structure and comparison of its tissue distribution with HtrA1 and HtrA2. Biochem. J. 371, 39-48 (2003).
28. Turner, N. A. Inflammatory and fibrotic responses of cardiac fibroblasts to myocardial damage associated molecular patterns (DAMPs). J. Mol. Cell. Cardiol. 94, 189-200 (2016).
29. Humeres, C. & Frangogiannis, N. G. Fibroblasts in the infarcted, remodeling, and failing heart. JACC Basic Transl. Sci. 4, 449-467 (2019).
30. Carmona-Cuenca, I. et al. Upregulation of the NADPH oxidase NOX4 by TGF-beta in hepatocytes is required for its pro-apoptotic activity. J. Hepatol. 49, 965-976 (2008).
31. Martyn, K. D., Frederick, L. M., Von Loehneysen, K., Dinauer, M. C. & Knaus, U. G. Functional analysis of Nox4 reveals unique characteristics compared to other NADPH oxidases. Cell. Signal. 18, 69-82 (2006).
32. Serrander, L. et al. NOX4 activity is determined by mRNA levels and reveals a unique pattern of ROS generation. Biochem. J. 406, 105-114 (2007).
33. Kuroda, J. et al. NADPH oxidase 4 (Nox4) is a major source of oxidative stress in the failing heart. Proc. Natl Acad. Sci. U.S.A. 107, 15565-15570 (2010).
34. Egerstedt, A. et al. Profiling of the plasma proteome across different stages of human heart failure. Nat. Commun. 10, 5830 (2019).
35. Masson, S. et al. Prognostic Value of Changes in N-Terminal Pro-Brain Natriuretic Peptide in Val-HeFT (Valsartan Heart Failure Trial). J. Am. Coll. Cardiol. 52, 997-1003 (2008).
36. Pal, D. et al. TGF-β reduces DNA ds-Break repair mechanisms to heighten genetic diversity and adaptability of CD44+/CD24- cancer cells. Elife 6, 1-28 (2017).
37. Zhang, H. et al. TGF-β inhibition rescues hematopoietic stem cell defects and bone marrow failure in Fanconi anemia. Cell Stem Cell 18, 668-681 (2016).
38. Coppe, J. P., Desprez, P. Y, Krtolica, A. & Campisi, J. The senescenceassociated secretory phenotype: The dark side of tumor suppression. Annu. Rev. Pathol. Mech. Dis. 5, 99-118 (2010).
39. Takahashi, A. et al. Downregulation of cytoplasmic DNases is implicated in cytoplasmic DNA accumulation and SASP in senescent cells. Nat. Commun. 9, 1249 (2018).
40. Tominaga, K. & Suzuki, H. I. TGF-β signaling in cellular senescence and aging-related pathology. Int. J. Mol. Sci. 20, 5002 (2019).
41. Lee, S. & Schmitt, C. A. The dynamic nature of senescence in cancer. Nat. Cell Biol. 21, 94-101 (2019).
42. Li, Y et al. DNA Damage Activates TGF-β Signaling via ATM-c-Cbl- Mediated Stabilization of the Type II Receptor TβRII. Cell Rep. 28, 735-745.e4 (2019).
43. Acosta, J. C. et al. A complex secretory program orchestrated by the inflammasome controls paracrine senescence. Nat. Cell Biol. 15, 978-990 (2013).
44. Hoare, M. et al. NOTCH1 mediates a switch between two distinct secretomes during senescence. Nat. Cell Biol. 18, 979-992 (2016).
45. Amor, C. et al. Senolytic CAR T cells reverse senescence-associated pathologies. Nature 583, 127-132 (2020).
46. Narkiewicz, J. et al. Changes in mRNA and protein levels of human HtrA1, HtrA2 and HtrA3 in ovarian cancer. Clin. Biochem. 41, 561-569 (2008).
47. Bowden, M. A., Di Nezza-Cossens, L. A., Jobling, T., Salamonsen, L. A. & Nie, G. Serine proteases HTRA1 and HTRA3 are down-regulated with increasing grades of human endometrial cancer. Gynecol. Oncol. 103, 253-260 (2006).
48. Pen, A., Moreno, M. J., Durocher, Y, Deb-Rinker, P. & Stanimirovic, D. B. Glioblastoma-secreted factors induce IGFBP7 and angiogenesis by modulating Smad-2-dependent TGF-β signaling. Oncogene 27, 6834-6844 (2008).
49. Calon, A. et al. Stromal gene expression defines poor-prognosis subtypes in colorectal cancer. Nat. Genet. 47, 320-329 (2015).
50. Wajapeyee, N., Serra, R. W., Zhu, X., Mahalingam, M. & Green, M. R. Oncogenic BRAF Induces Senescence and Apoptosis through Pathways Mediated by the Secreted Protein IGFBP7. Cell 132, 363-374 (2008).
51. Faget, D. V, Ren, Q. & Stewart, S. A. Unmasking senescence: contextdependent effects of SASP in cancer. Nat. Rev. Cancer 19, 439-453 (2019).
52. Lopes-Paciencia, S. et al. The senescence-associated secretory phenotype and its regulation. Cytokine 117, 15-22 (2019).
53. Farr, J. N. et al. Targeting cellular senescence prevents age-related bone loss in mice. Nat. Med. 23, 1072-1079 (2017).
54. Everett, B. M. et al. Anti-inflammatory therapy with canakinumab for the prevention of hospitalization for heart failure. Circulation 139, 1289-1299 (2019).
55. Tsuchiya, A. et al. Expression of mouse HtrA1 serine protease in normal bone and cartilage and its upregulation in joint cartilage damaged by experimental arthritis. Bone 37, 323-336 (2005).
56. Takimoto, E. et al. Chronic inhibition of cyclic GMP phosphodiesterase 5A prevents and reverses cardiac hypertrophy. Nat. Med. 11, 214-222 (2005).
57. Toko, H. et al. ATF6 is important under both pathological and physiological states in the heart. J. Mol. Cell. Cardiol. 49, 113-120 (2010).
58. Trapnell, C., Pachter, L. & Salzberg, S. L. TopHat: Discovering splice junctions with RNA-Seq. Bioinformatics 25, 1105-1111 (2009).
59. Trapnell, C. et al. Differential gene and transcript expression analysis of RNAseq experiments with TopHat and Cufflinks. Nat. Protoc. 7, 562-578 (2012).
60. Trapnell, C. et al. Transcript assembly and quantification by RNA-Seq reveals unannotated transcripts and isoform switching during cell differentiation. Nat. Biotechnol. 28, 511-515 (2010).
61. Shannon, P. et al. Cytoscape: A software environment for integrated models. Genome Res. 13, 2498-2504 (2003).
62. Becht, E. et al. Dimensionality reduction for visualizing single-cell data using UMAP. Nat. Biotechnol. 37, 38-47 (2019).
63. Lun, A. T. L., Mccarthy, D. J. & Marioni, J. C. A step-by-step workflow for low-level analysis of single-cell RNA-seq data with Bioconductor [version 2; referees: 3 approved, 2 approved with reservations]. F1000Research 5, 1-69 (2016).
64. Street, K. et al. Slingshot: cell lineage and pseudotime inference for single-cell transcriptomics. BMCGenomics 19, 477 (2018).
65. de Hoon, M. J. L., Imoto, S., Nolan, J. & Miyano, S. Open source clustering software. Bioinformatics 20, 1453-1454 (2004).
66. Saldanha, A. J. Java Treeview -Extensible visualization of microarray data. Bioinformatics 20, 3246-3248 (2004).
67. Stuart, T. et al. Comprehensive Integration of Single-Cell Data. Cell 177, 1888-1902.e21 (2019).
68. Huang, D. W., Sherman, B. T. & Lempicki, R. A. Systematic and integrative analysis of large gene lists using DAVID bioinformatics resources. Nat. Protoc. 4, 44-57 (2009).
69. Langmead, B., Trapnell, C., Pop, M. & Salzberg, S. L. Ultrafast and memoryefficient alignment of short DNA sequences to the human genome. Genome Biol. 10, R25 (2009).
70. Wang, L., Feng, Z., Wang, X., Wang, X. & Zhang, X. DEGseq: An R package for identifying differentially expressed genes from RNA-seq data. Bioinformatics 26, 136-138 (2009).
71. Yadav, H. et al. Protection from obesity and diabetes by blockade of TGF-β/ Smad3 signaling. Cell Metab. 14, 67-79 (2011).
72. Wang, F. et al. RNAscope: A novel in situ RNA analysis platform for formalinfixed, paraffin-embedded tissues. J. Mol. Diagnostics 14, 22-29 (2012).
73. Ruozi, G. et al. AAV-mediated in vivo functional selection of tissue-protective factors against ischaemia. Nat. Commun. 6, 7388 (2015).
74. Harshad, K. et al. An electromagnetic cell-stretching device for mechanotransduction studies of olfactory ensheathing cells. Biomed. Microdevices 18, 1-10 (2016).
75. Komuro, I. et al. Communication stretching cardiac myocytes stimulates protooncogene expression. J. Biol. Chem. 265, 3595-3598 (1990).
116. Evdokimova, V et al. IGFBP7 binds to the IGF-1 receptor and blocks its activation by insulin-like growth factors. Sci Signal 5, ra92, doi:10.1126/scisignal.2003184 (2012).
122. Saxena, M., van der Burg, S. H., Melief, C. J. M. & Bhardwaj, N. Therapeutic cancer vaccines. Nat Rev Cancer 21, 360-378, doi:10.1038/s41568-021-00346-0 (2021).
123. Koriyama, H. et al. Long-Term Reduction of High Blood Pressure by Angiotensin II DNA Vaccine in Spontaneously Hypertensive Rats. Hypertension 66, 167-174, doi:10.1161/HYPERTENSIONAHA.114.04534 (2015).
124. Fukami, H. et al. Vaccine targeting ANGPTL3 ameliorates dyslipidemia and associated diseases in mouse models of obese dyslipidemia and familial hypercholesterolemia. Cell Rep Med 2, 100446, doi:10.1016/j.xcrm.2021.100446 (2021).

## Claims

1. An agent for regulating a DNA repair mechanism of cardiac fibroblasts, the agent comprising at least one of the following (a), (b), (c), (d), and (e);
(a) serine protease HtrA3,
(b) a nucleic acid encoding serine protease HtrA3 or a fragment thereof,
(c) an expression vector comprising a nucleic acid encoding serine protease HtrA3 or a fragment thereof,
(d) a cell transformed with a nucleic acid encoding serine protease HtrA3, a fragment thereof, or an expression vector comprising the nucleic acid or fragment, and
(e) an antibody that binds to serine protease HtrA3 or a fragment thereof.

2. The agent according to claim 1, wherein the serine protease HtrA3 is derived from a human or mouse.

3. A pharmaceutical composition for treating or preventing myocardial infarction, cardiac fibrosis, or heart failure, the composition comprising: the agent according to claim 1 or 2 or at least one of (a), (b), (c), (d), and (e) according to claim 1 or 2 as an active ingredient.

4. The pharmaceutical composition of claim 3, wherein the heart failure is heart failure with reduced ejection fraction (HFrEF).

5. A non-human mammalian heart failure model animal in which expression of serine protease HtrA3 in cardiac fibroblasts is reduced or inhibited.

6. A method for screening for an agent or a method for regulation of a DNA repair mechanism in a cardiac fibroblast, the method comprising:
(A) a step of treating a cell in which expression of serine protease HtrA3 is reduced or inhibited with a test factor; and
(B) a step of comparing the cell to an untreated control cell or a wild-type cells.

7. A method for screening for an agent or a method for regulation of a DNA repair mechanism in a cardiac fibroblast, the method comprising:
(C) a step of treating the non-human mammal according to claim 5 with a test factor; and
(D) a step of comparing the non-human mammal to an untreated control animal or a wild-type animal.

8. A method of diagnosing myocardial infarction, cardiac fibrosis, or heart failure, the method comprising:
(E) a step of determining that a subject may suffer from myocardial infarction, cardiac fibrosis or heart failure in a case where an expression level of serine protease HtrA3 in a cardiac fibroblast of the subject is significantly reduced as compared with an expression level in a cardiac fibroblast of a healthy subject.

9. A pharmaceutical composition for treating or preventing myocardial infarction or heart failure, the composition comprising at least one of the following (f), (g), (h), and (i);
(f) a nucleic acid consisting of a base sequence complementary to a nucleic acid encoding insulin-like growth factor binding protein 7 (IGFBP7) or a fragment thereof,
(g) an antibody that binds to insulin-like growth factor binding protein 7 (IGFBP7) or a fragment thereof,
(h) a nucleic acid that encodes an antibody or a fragment thereof that binds to insulin-like growth factor binding protein 7 (IGFBP7) or a fragment thereof, and
(i) an expression vector comprising a nucleic acid that encodes an antibody or a fragment thereof that binds to insulin-like growth factor binding protein 7 (IGFBP7) or a fragment thereof.

10. The pharmaceutical composition according to claim 9, wherein the nucleic acid (f) composed of a complementary base sequence is selected from the group consisting of siRNA, antisense nucleic acid, shRNA, miRNA, gRNA, ribozyme, and a nucleic acid aptamer.

11. The pharmaceutical composition according to claim 9, wherein the insulin-like growth factor binding protein 7 (IGFBP7) is from a human or mouse.

12. A non-human mammalian heart failure model animal with enhanced expression of insulin-like growth factor binding protein 7 (IGFBP7) in cardiac fibroblasts.

13. A method for screening for an agent or a method for treating or preventing myocardial infarction or heart failure, the method comprising:
(F) a step of treating a cell with enhanced expression of insulin-like growth factor binding protein 7 (IGFBP7) with a test factor; and
(G) a step of comparing the cell to an untreated control cell or a wild-type cell.

14. A method for screening an agent or a method for treating or preventing myocardial infarction or heart failure, the method comprising:
(H) a step of treating the non-human mammal according claim 12 with a test factor; and
(I) a step of comparing the non-human mammal to an untreated control animal or a wild-type animal.

15. A method of diagnosing myocardial infarction or heart failure, the method comprising:
(J) a step of determining that a subject may suffer from myocardial infarction or heart failure in a case where an expression level of insulin-like growth factor binding protein 7 (IGFBP7) in cardiac fibroblasts, cardiomyocytes, or blood of the subject is significantly increased as compared with an expression level in a cardiac fibroblast, a cardiomyocyte, or blood of a healthy subject.

16. A method of determining a severity level of myocardial infarction, or heart failure, the method comprising:
(K) a step of determining a severity level of myocardial infarction or heart failure of a subject by comparing an expression level of insulin-like growth factor binding protein 7 (IGFBP7) in a cardiac fibroblast, a cardiomyocyte, or blood of the subject with an expression level in a cardiac fibroblast, a cardiomyocyte, or blood of a healthy subject or a past expression level of the subject.
